# NEW EUROPEAN PATENT SPECIFICATION

(11) **EP 1 507 798 B2**
(45) Date of publication and mention of the opposition decision: **16.11.2016**
(45) Mention of the grant of the patent: 20.11.2013
(21) Application number: 03733622.9
(22) Date of filing: 26.05.2003
(51) Int. Cl.: C07K 14/315

(54) **EXPORT AND MODIFICATION OF (POLY)PEPTIDES IN THE LANTIBIOTIC WAY**
EXPORT UND MODIFIZIERUNG VON (POLY)PEPTIDEN IM LANTIBIOTISCHEN WEG
EXPORTATION ET MODIFICATION DE (POLY)PEPTIDES DE TYPE LANTIBIOTIQUE

(30) Priority: 24.05.2002 EP 02077060; 07.02.2003 US 360101
(43) Date of publication of application: 23.02.2005
(73) Proprietor: MorphoSys AG, 82152 Planegg-Martinsried (DE)
(72) Inventor: MOLL, Gert Nikolaas, NL-9725 LJ Groningen (NL); LEENHOUTS, Cornelis Johannes, NL-9753 KX Haren (NL); KUIPERS, Oscar Paul, NL-9728 XG Groningen (NL); DRIESSEN, Arnold Jacob Mathieu, NL-9727 DA Groningen (NL)
(74) Representative: Hutter, Bernd
(86) International application number: PCT/NL2003/000389
(87) International publication number: WO 2003/099862

(56) References cited:
- US-A- 5 861 275
- US-A- 5 885 811
- US-A- 6 028 168
- US-A1- 2002 019 518
- NOVAK J ET AL: "CLONING, SEQUENCING AND EXPRESSION OF AN ABC TRANSPORTER INVOLVED IN THE PRODUCTION OF THE LANTIBIOTIC MUTACIN II IN STEPTOCOCCUS MUTANS" ABSTRACTS OF THE GENERAL MEETING OF THE AMERICAN SOCIETY FOR MICROBIOLOGY, AMERICAN SOCIETY FOR MICROBIOLOGY, WASHINGTON, US, vol. 96, 1996, page 217 XP000892159 ISSN: 0067-2777
- IZAGUIRRE GONZALO ET AL: "Use of alkaline phosphatase as a reporter polypeptide to study the role of the subtilin leader segment and the SpaT transporter in the posttranslational modifications and secretion of subtilin in Bacillus subtilis 168." APPLIED AND ENVIRONMENTAL MICROBIOLOGY, vol. 63, no. 10, 1997, pages 3965-3971, XP002217349 ISSN: 0099-2240
- PAUL LEENA K ET AL: "Studies of the subtilin leader peptide as a translocation signal in Escherichia coli K12." FEMS MICROBIOLOGY LETTERS, vol. 176, no. 1, 1 July 1999 (1999-07-01), pages 45-50, XP002217350 ISSN: 0378-1097
- FRANKE CHRISTIAN M ET AL: "Membrane topology of the lactococcal bacteriocin ATP-binding cassette transporter protein LcnC: Involvement of LcnC in lactococcin a maturation." JOURNAL OF BIOLOGICAL CHEMISTRY, vol. 274, no. 13, 26 March 1999 (1999-03-26), pages 8484-8490, XP002217351 ISSN: 0021-9258
- BIERBAUM G ET AL: "The biosynthesis of the lantibiotics epidermin, gallidermin, Pep5 and epilancin K7." ANTONIE VAN LEEUWENHOEK. NETHERLANDS FEB 1996, vol. 69, no. 2, February 1996 (1996-02), pages 119-127, XP009021430 ISSN: 0003-6072
- MOLL G N ET AL: "The lantibiotic nisin induces transmembrane movement of a fluorescent phospholipid." JOURNAL OF BACTERIOLOGY. UNITED STATES DEC 1998, vol. 180, no. 24, December 1998 (1998-12), pages 6565-6570, XP002262859 ISSN: 0021-9193
- QIAO MINGQIANG ET AL: "Evidence for a role of NisT in transport of the lantibiotic nisin produced by Lactococcus lactis N8." FEMS MICROBIOLOGY LETTERS, vol. 144, no. 1, 1996, pages 89-93, XP001117575 ISSN: 0378-1097
- BREUKINK E ET AL: "The lantibiotic nisin, a special case or not?" BIOCHIMICA ET BIOPHYSICA ACTA. BIOMEMBRANES, AMSTERDAM, NL, vol. 1462, no. 1-2, 15 December 1999 (1999-12-15), pages 223-234, XP004273118 ISSN: 0005-2736
- SIEZEN R J ET AL: "COMPARISON OF LANTIBIOTIC GENE CLUSTERS AND ENCODED PROTEINS" ANTONIE VAN LEEUWENHOEK, DORDRECHT, NL, vol. 2, no. 69, February 1996 (1996-02), pages 171-184, XP001095134
- MCAULIFFE OLIVIA ET AL: "Lantibiotics: Structure, biosynthesis and mode of action" FEMS MICROBIOLOGY REVIEWS, ELSEVIER, AMSTERDAM, NL, vol. 25, no. 3, May 2001 (2001-05), pages 285-308, XP002209342 ISSN: 0168-6445
- FATH M J ET AL: "ABC TRANSPORTERS: BACTERIAL EXPORTERS" MICROBIOLOGICAL REVIEWS, AMERICAN SOCIETY FOR MICROBIOLOGY, WASHINGTON, DC, US, vol. 57, no. 4, 1 December 1993 (1993-12-01), pages 995-1017, XP002050868 ISSN: 0146-0749
- P. FEKKES ET AL.: 'Protein Targeting to the Bacterial Cytoplasmic Membrane' MICROBIOLOGY AND MOLECULAR BIOLOGY REVIEWS vol. 63, no. 1, March 1999, pages 161 - 173
- S. NEIS ET AL.: 'Effect of leader peptide mutations on biosynthesis of the lantibiotic Pep5' FEMS MICROBIOLOGY LETTERS vol. 149, 1997, pages 249 - 255
- P. CHEN ET AL.: 'Effect of amino acid substitutions in conserved residues in the leader peptide on biosynthesis of the lantibiotic mutacin II' FEMS MICROBIOLOGY LETTERS vol. 195, 2001, pages 139 - 144
- J R VAN DER MEER ET AL.: 'Influence of Amino Acid Substitutions in the Nisin Leader Peptide on Biosynthesis and Secretion of Nisin by Lactococcus lactis*' THE JOURNAL OF BIOLOGICAL CHEMISTRY no. 5, 04 February 1994, pages 3555 - 3562

## Description

The invention is related to the field of lantibiotics and to the field of post-translational modifications of (poly)peptides.

Lantibiotics form a group of unique ribosomally synthesised and post-translationally modified antibiotic peptides that are produced by, and primarily act on, Gram-positive bacteria (for review see McAuliffe et al., FEMS Microbiol. Rev. 25,:285-308 (2001). Because by definition they contain intramolecular thioether bridges or rings formed by the thioether amino acids *lan*thionine (Lan) and 3-methyl*lan*thionine (MeLan) and they all are peptide *antibiotics* with moderate to strong bactericidal activity, they take their name from these most eye-catching properties.

Thioether rings protect peptides against proteolytic degradation. For instance the lantibiotic nisin remains active after trypsin treatment. Thioether rings are essential for some lantibiotic activities. For instance opening of ring A or C in nisin causes deletion of the membrane permeabilization capacity. Ring A of nisin is necessary for its capacity to autoinduce its own synthesis and for nisin's capacity to block the peptidoglycan synthesis by interacting with lipid II. It is essential to have thioether rings and not disulfide rings since replacement of thioether rings by disulfide bridges leads to loss of antimicrobial activity.

They do not spoil the environment and are not toxic for animals or man and find, or may find, applications as biopreservatives in the preparation of food and beverages, but also as bactericidal agent in cosmetics and veterinary and medical products. Because the growing number of multidrug resistant pathogenic microorganisms has created the threat of another "pre-antibiotic era" for many bacterial diseases, it is expected that lantibiotics also may serve as new lead compound to remedy this alarming problem. Mainly for these reasons, in the past decade, lantibiotics have experienced a marked increase in basic and applied research activities, leading to an extraordinary increase in our knowledge of their structural and functional properties, their mechanisms of action and of the genes and protein components involved in their biosynthesis and secretion. For example, lantibiotics have now become subject to "protein engineering" projects, with the aim of altering, via site-directed mutagenesis, their activity, stability and spectrum of susceptible target cells. In this description we focus upon the linear (type A) lantibiotics since at present very little specific information is available for the circular (type B) lantibiotics.

The lantibiotis subtilin and nisin belong to and are representative for the peptide antibiotics or lantibiotics of type A. They contain the rare amino acids dehydroalanine, (Dha) dehydrobutyrine (Dhb), *meso*-lanthionine, and 3-methyllanthionine and the characterising thioether bridges. Nisin is the most prominent lantibiotic and is used as a food preservative due to its high potency against certain Gram-positive bacteria. It is produced by *Lactococcus lactis* strains belonging to serological group N. The potent bactericidal activities of nisin and many other lantibiotics are based on their capacity to permeabilize the cytoplasmic membrane of target bacteria. Breakdown of the membrane potential is initiated by the formation of pores through which molecules of low molecular weight are released. In addition, nisin inhibits cel wall synthesis by binding to lipid II, a precursor of peptidoglycan synthesis, modulates the activity of autolytic enzymes and inhibits the outgrowth of spores (see also Breukink and de Kruijf, Biochem. Biophys. Acta 1462:223-234, 1999).

In several countries nisin is used to prevent the growth of clostridia in cheese and canned food. The nisin peptide structure was first described by Gross & Morell (J. Am. Chem. Soc 93:4634-4635, 1971), and its structural gene was isolated in 1988 (Buchmann et al., J. Biol. Chem. 263:16260-16266, 1988). Nisin has two natural variants, nisin A and nisin Z, which differ in a single amino acid residue at position 27 (histidin in nisin A is replaced by asparagin in nisin Z).

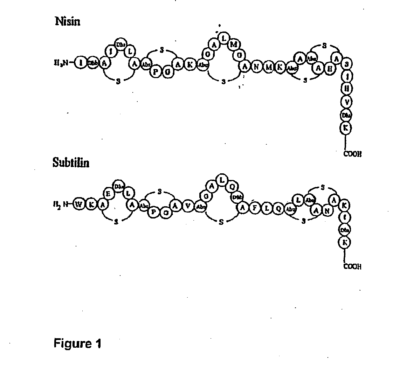

Subtilin is produced by *Bacillus subtilis* ATCC 6633. Its chemical structure was first unravelled by Gross & Kiltz (Biochem. Biophys. Res. Commun. 50: 559-565, 1973) and its structural gene was isolated in 1988 (Banerjee & Hansen, J. Biol. Chem. 263:9508-9514, 1988). Subtilin shares strong similarities to nisin with an identical organization of the lanthionine ring structures (Fig. 1), and both lantibiotics possess similar antibiotic activities.

Due to its easy genetic analysis *B. subtilis* became a very suitable model organism for the identification and characterization of genes and proteins involved in lantibiotic biosynthesis. The pathway by which nisin is produced is very similar to that of subtilin, and the proteins involved share significant homologies over the entire proteins (for review see also De Vos et al., Mol. Microbiol. 17:427-437, 1995).

Another well known and studied lantibiotic, produced by Staphylococcus epidermis 5, is Pep 5, which contains three ring structures (one MeLan and two Lan), an N-terminal oxobutyryl residue, and two Dhb residues (Kellner et al., Angew. Chemie Int. Ed. Engl. 28:616-619, 1989)

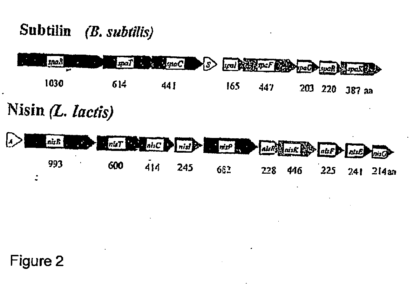

The respective posttranslationally acting genes have been identified adjacent to the structural genes, and together they are organized in operon-like structures (Fig. 2). These genes are thought to be responsible for post-translation modification, transport of the modified prepeptide, proteolytic cleavage, and immunity which prevents toxic effects on the producing bacterium. In addition to this, biosynthesis of subtilin and nisin is strongly regulated by a two-component regulatory system which consists of a histidin kinase and a response regulator protein.

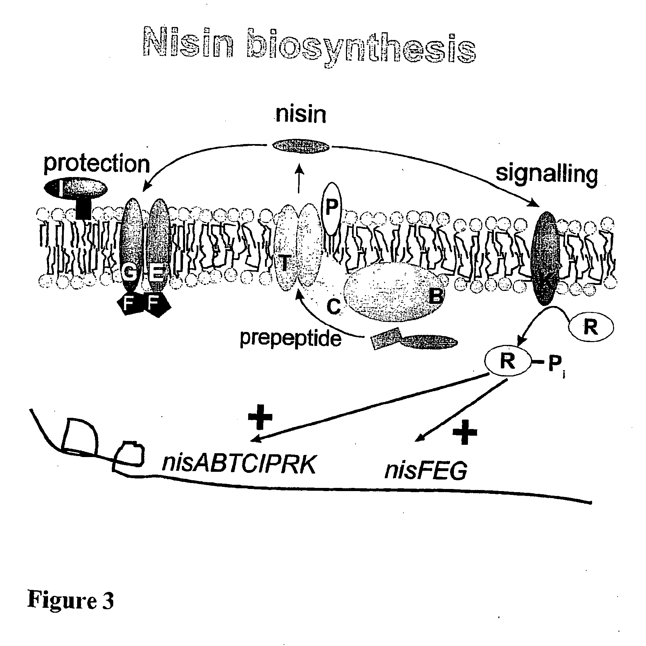

According to a present model (Fig. 3) it is assumed that an extracellular growth phase-dependent signal may activate the membrane localized histidine kinase. The nature of this signal may be different for subtilin and nisin biosynthesis. Whereas in nisin biosynthesis, nisin itself has an inducing function, it was shown for subtilin biosynthesis that its biosynthesis is sporulation dependent.

According to the model, after its auto-phosphorylation the SpaK and NisK histidine kinase transfer the phosphate residue to the response regulator which in turn activates the genes necessary for subtilin and nisin biosynthesis. Thereafter, the prepeptide is modified at a membrane localized modification complex (lantionine synthetase) consisting of the intracellular SpaB/SpaC and the NisB/NisC proteins, respectively. According to the model, these proteins are also associated with the SpaT and the NisT transporter, respectively.

As in any lantibiotic, the presubtilin or prenisin molecule consists of a leader segment and a mature segment, and the leader segment is thought to play several roles in the biosynthetic pathway. It is thought not to be just a translocation signal sequence, but thought to provide recognition signals for the modification enzymes and to suppress antimicrobial activity until the mature peptide is released from the cell. As also postulated by Qiao and Saris, (Fems Microbiol. Let. 144:89-93(1996), the modified prepeptide is in the case of some lantibiotics proteolytically cleaved after its transport through the cellular membrane, but in the case of other lantibiotics cleavage of the leader from the modified peptide occurs inside the cell before secretion. In the case of nisin, cleavage is performed by NisP, whereas in the case of subtilin no specific protease has been found within the operon-like structure. However, *B. subtilis* is rich in extracellular proteases and possibly subtilisin, which also recognizes proline at position-2, could cleave the modified pre-subtilin.

The gene clusters flanking the structural genes for various linear (type A) lantibiotics have recently been characterized (for review see Siezen et al., Antonie van Leeuwenhoek 69:171-184, 1996). The best studied representatives are those of nisin (*nis*), subtilin (*spa*), epidermin (*epi*), Pep5 (*pep*), cytolysin (*cyl*), lactocin S (*las*) and lacticin 481 (*lct*). Comparison of the lantibiotic gene clusters shows that they contain conserved genes that probably encode similar functions.

The *nis, spa, epi* and *pep* clusters contain *lanB* and *lanC* genes that are presumed to code for two types of enzymes that have been implicated in the modification reactions characteristic of all lantibiotics, i.e. dehydration and thio-ether ring formation. The *cyl, las* and *lct* gene clusters have no homologue of the *lanB* gene, but they do contain a much larger *lanM* gene that is the *lanC* gene homologue. Most lantibiotic gene clusters contain a *lanP* gene encoding a serine protease that is presumably involved in the proteolytic processing of the prelantibiotics. All clusters contain a *lanT* gene encoding an ATP-binding cassette (ABC)-like transporter spanning the plasma membrane of a cell and likely to be involved in the export of (precursors of) the lantibiotics from the cell. The *lanE, lanF* and *lanG* genes in the *nis, spa* and epi clusters encode another transport system that is possibly involved in self-protection. In the nisin and subtilin gene clusters two tandem genes, *lanR* and *lanK*, have been located that code for a two-component regulatory system.

Finally, non-homologous genes are found in some lantibiotic gene clusters. The *nisL* and *spaI* genes encode lipoproteins that are involved in immunity, the *pepL* gene encodes a membrane-located immunity protein, and *epiD* encodes an ensyme involved in a post-translational modification found only in the C-terminus of epidermin. Several genes of unknown function are also found in the *lan* gene cluster. Commonly, a host organism or cell carrying one or more of said genes (here for example *lanT, lanI, lanA, lanP, lanB* and *lanC)* in said cluster are identified with a shorthand notation such as *lanTIAPBC.* The above identified genes are clearly different from genes encoding the secretion apparatus for the non-lantibiotic lactococcins that is composed of two membrane proteins LcnC and LcnD, as for example discussed in Franke et al., J. Biol. Chem 274:8484-8490, (1999).

A database has been assembled for all putative gene products of type A lantibiotic gene clusters. Database searches, multiple sequence alignment and secondary structure prediction have been used to identify conserved sequence segments in the LanB, LanC, LanE, LanF, LanG, LanK, LanP, LanM, LanR and LanT gene products that may be essential for structure and function (Siezen et al., *ibid*). This database allows for a rapid screening of newly determined sequences in lantibiotic gene clusters.

However, despite all above cited recent knowledge obtained in the field, attempts to engineer novel lantibiotic-like peptides comprising newly synthesised non-naturally occurring thioether bridges have been scarce, if not rather unsuccessful. In US 5,861,275, nisin-subtilin chimeras have been produced in the Gram-positive *Bacilus subtilis,* that however, do not comprise thioether bridges other than naturally occurring in either nisin or subtilin. In a different application (US 2002/0019518), *Bacillus subtilis* was used to produce a chimeric polypeptide comprising a lantibiotic peptide and a subtilin leader segment, a lantibody, that remains associated within the cell wall.

A novel thioether bridge in lantibiotic Pep5 has been engineered by Bierbaum et al., Appl. Env. Microbiol. 62:385-392, 1996 by modifying the Gram-positive bacterium *Staphylococcus epidermis* 5 by depleting the host organism of the gene cluster *pepTIAPBC* and replacing it with a gene cluster *pepIAPBC,* wherein *pepA* was or was not replaced with mutated structural genes encoding for a Pep 5 peptide wherein amino acids were substituted; genes coding for peptides with substitutions C27A (Cysteine to Alanine at position 27), C33A, A19C, Dhb16A, Dhb20A, K18Dha were generated. Only the A19C substitution resulted in novel thioether ring formation. The clone corresponding to the A19C substitution produced a rather small amount of a peptide that showed only little activity. It was thought that prolonged exposure of the peptide to intracellular protease of the producing transformed cell was causal to this disappointing result.

The K18Dha substitution in Pep 5 resulted in a clone that produced incompletely dehydrated serine at position 18. Kuipers et al., (J. Biol. Chem. 267:24340-24346, 1992) engineered a new Dhb residue into nisin Z by substituting M17Q/G18T in said lantibiotic, but also obtained only incomplete dehydration of the resulting threonine and no additional ring formation. The incomplete dehydration is generally thought to be a result of questionable substrate specificity of the dehydrating enzyme LanB in the transformed cell.

In short, no large measure of success has yet been achieved in providing novel thioether bridges to lantibiotics in Gram-positive organisms, let alone that engineered thioether bridge formation has been provided to polypeptides of non-lantibiotic descent or by organisms other than Gram-positive bacteria.

Paul, Leena K et al (FEMS Microbiol. Lett. 176:45-50, 1999) recently studied the subtilin leader peptide as a translocation signal in the Gram-negative *E. coli*, by default devoid of a specific lantibiotic transporter system, and provided a fusion-protein comprising the subtilin leader peptide and part of the mature subtilin attached to E. coli alkaline phosphatase (AP) to study said possible translocation. Although the fusion protein was translocated to the periplasmic side of the cytoplasmic membrane, it remained associated with that membrane. In earlier work, (Izaguirre & Hansen, Appl. Environ. Microbiol 63:3965-3971, 1997) the same fusion protein was expressed in the Gram-positive *Bacillus subtilis,* where it was cleaved off from said membrane after successful translocation, but where no dehydration of serines or threonines of the AP polypeptide, let alone thioether bridge formation was observed. Novak J et al., ASM general meeting 96:217 (1999), recently provided an E. coli host cell with an ORF (ORF1) encoding an ABC transporter of 341 amino acids, which is thought to be involved in the translocation of the lantibiotic mutacin II in *Streptococcus mutans*. However, an intact gene product of said ORF1 was not produced in E. coli, whereas a truncated protein of unknown identity or functionality was observed.

For the purpose of protein engineering of lantibiotics (for an extensive review see Kuipers et al., Antonie van Leeuwenhoek 69:161-170, 1996) or for the purpose of engineering newly designed (poly)peptides with lantibiotic-type posttranslational modifications, for example for pharmaceutical use, much attention has recently (see for example Entian & de Vos, Antoni van Leeuwenhoek 69:109-117, 1996; Siegers et al., J. Biol. Chem. 271:1294-12301, 1996 ;Kiesau et al., J. Bacter. 179:1475-1481, 1997) been given to understanding the role of the LanB, LanC (or LanM) and LanT complex, the enzymes thought to be involved (in that order) in dehydration, thioether ring formation and transportation of the lantibiotic out of the cell.

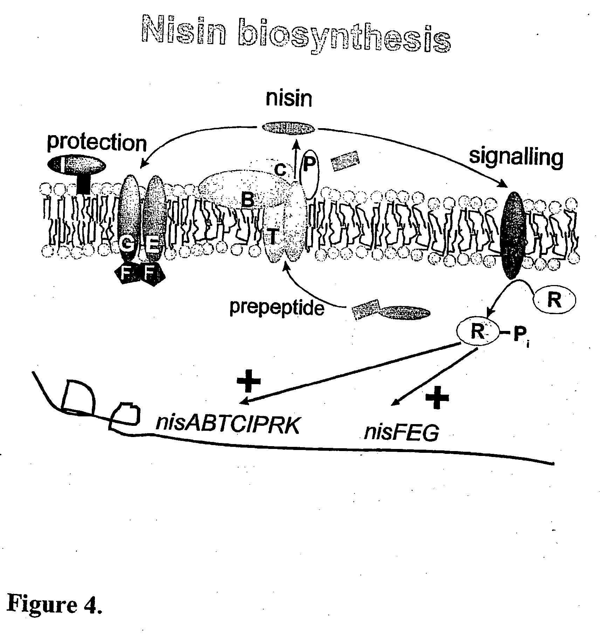

The present invention shows that the unmodified peptide, being coupled to its leader peptide, can be transported out of the cell without prior modification, and LanB and LanC (or LanM), when acting at all, may act not in, but also outside of the cell (see also fig. 4).

Where it was earlier commonly thought that LanB and LanC act in concert to modify the peptide only before it is translocated, it is herein furthermore provided that after transportation, the as yet unmodified peptide extracellularly may undergo its specific posttranslational modification leading to dehydration and thioether bridge formation, bringing the role of the transporter protein to central stage to modify a (poly)peptide in the lantibiotic way, it being a prerequisite to present the unmodified peptide to the modification machinery.

Thus, the invention provides the insight that dehydration of a serine or threonine of a (poly)peptide and subsequent thioether bridge formation can satisfactorily occur when a pre(poly)peptide has been transported out of the host cell wherein it was produced by translation, preferably by a transporter protein such as an ABC transporter, preferably at least functionally corresponding to a transporter commonly identifiable as LanT. Dehydration (and optionally thioether bridge formation) is then only enzymatically catalysed by an enzyme or enzymes that are at least functionally corresponding to LanB and/or LanC. Said transporter transports the to-be-modified (poly)peptide through the membrane of the host cell where it is positioned in working proximity to extra-cellular located LanB for dehydration.

The invention provides a method for producing a thioether bridge containing polypeptide of a non-Gram positive bacterium origin in a host cell, said method comprising:
a) selecting a host cell having a nucleic acid molecule comprising:
   - a first nucleic acid fragment encoding a leader peptide;
   - a second nucleic acid fragment encoding a desired polypeptide comprising a sequence selected from the group of sequences consisting of Ser-Xaaₙ-Cys and Thr-Xaaₙ-Cys, wherein Xaa is any amino acid and wherein n is 1-13, and wherein the polypeptide is of non-Gram positive bacterium origin;
   - wherein the first and second nucleic acid fragments are within the same open reading frame as the nucleic acid molecule; and
   - wherein the leader peptide is functionally equivalent to an N-terminal leader peptide found within a prepeptide of a lantibiotic;
b) selecting the host cell for the presence of the transporter protein LanT or a functional equivalent thereof having LanT activity;
c) translating the nucleic acid molecule, thus producing the leader peptide and the polypeptide comprising the sequence selected from the group of sequences consisting of Ser-Xaaₙ-Cys and Thr-Xaaₙ-Cys, wherein Xaa is any amino acid and wherein n is 1-13; and
d) harvesting the polypeptide containing the thioether bridge from a medium of the host cell.

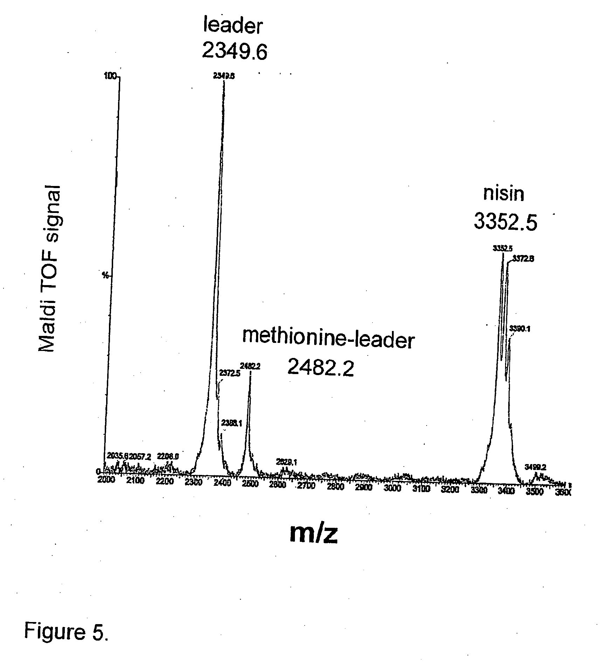

In a further preferred embodiment, the invention provides a method according to the invention further comprising harvesting said desired (poly)peptide after detecting the presence of said leader peptide in the culture medium or supernatant of said cell (see also fig. 5). For detecting said presence, it is preferred that said medium contains only few nutrients, i.e is a so-called minimal medium. Also, it is preferred that said presence is detected by harvesting the supernatant by aspirating and dispensing the supernatant into and out of a pipet tip (or other harvesting device) that contains a microvolume bed of affinity chromatography media fixed at the end of the tip that is preferably without dead volume. This procedure is herein also called "ziptipping" and allows, after subsequent elution, for relatively pure presentation of desired (poly)peptide for further analyses.

Preferably by using the combination of growth in minimal medium and ziptipping the supernatant of this culture, samples of sufficient purity can be obtained that are well suited for detection or analyses by high resolution MALDI-TOFMS. This allowes most significant measurement of leader peptide and thus prediction of desired (poly)peptide content. Detection of said leader peptide therefore can be used to ascertain the export of (poly)peptide coupled to this lantibiotic leader, especially in those cases where the leader peptidase acts extracellularly.

In a further preferred embodiment, the invention provides for a method wherein the host cell producing the desired (poly)peptide is essentially devoid of leader peptidase (LanP) activity, thereby allowing the production and extracellular harvest -by using anti-leader antibodies- of desired (poly)peptide that is essentially still coupled to its leader peptide. Also, in this way, potential intracellular toxic effects of desired (poly)peptide provided with thioether bridges are reduced. It of course also suffices to design a leader peptide that cannot be cleaved by the leader peptidase of the host cell used. In both cases, the desired (poly)peptide can later be obtained free from the leader peptide, for example by specific proteolytic cleavage, using added LanP, or by another suitable protease capable of cleaving the leader peptide from the desired (poly)peptide. Furthermore, it is herein provided that in a desired (poly)peptide, a serine or serines, that are N-terminally located from cysteines are dehydrated and coupled to more C-terminally located cysteines. As further exemplified herein in the detailed description, a (poly)peptide sequence with a serine and a cysteine "S - C" contains (preferably after thioether bridge formation) alanines in said positions of the serine and cysteine: "A - A". These alanines are coupled by-aside from the peptide backbone- a thioether bridge.

Generally a method as provided herein allows a high detection level for measuring levels of (lantibiotic) (poly)peptides directly from the culture supernatant, considering that the ratio leader peptide versus desired (poly)peptide is essentially 1:1. Such guidance allows for efficient culture methods to produce the desired polypeptide, and allows for determining appropriate or optimal time-points at which said culture may be harvested.

In a preferred embodiment, the invention provides a method according to the invention, and a (poly)peptide according to the invention wherein said desired (poly)peptide is selected from the group of peptide hormones or fragments of these hormones or analogues from these hormones originating from hypophysis and/or peptide hormones with similar actions such as vasopressin, terlipressin, desmopressin, cispressin, oxytocin, adrenocorticotropic hormone and human growth hormone.

In another preferred embodiment, the invention provides a method according to the invention, and a (poly)peptide according to the invention wherein said desired (poly)peptide is selected from the group of peptide hormones or fragments of these hormones or analogues from these hormones originating from hypothalamus, and/or peptide hormones with similar actions such as gonadoliberinII, luteinizing hormone releasing hormone, leuprolide, and other synthetic analogues of LHRH such as gonadoreline, gosereline, busereline, leuproreline, nafareline, triptoreline, and cetrorelix, somatostatin, analogues of somatostatin such as octreotide, somatostatin, corticotropin inhibiting peptide, corticotropin-release factor, urocortin, urotensin II and growth hormone release factor.

In another preferred embodiment, the invention provides a method according to the invention, and a (poly)peptide according to the invention wherein said desired (poly)peptide is selected from the group of peptide hormones or fragments of these hormones or analogues from these hormones originating from adrenocortex, adrenal medulla, kidney and heart and/or peptide hormones with similar actions such as adrenomedullin, angiotensin I, atrial natriuretic factor, bradykinin, brain natriuretic peptide, C-type natriuretic peptide and vasonatrin peptide.

In a another preferred embodiment, the invention provides a method according to the invention, and a (poly)peptide according to the invention wherein said desired (poly)peptide is selected from the group of peptide hormones or fragments of these hormones or analogues from these hormones originating from other endocrine/exocrine organs such as the pancreas, thyroid and parathyroid and/or peptide hormones with similar actions such as calcitonin, osteocalcin, glucagon, insulin, insulin-like growth factor-I or II, parathormone, and cholecystokinin.

In another preferred embodiment, the invention provides a method according to the invention, and a (poly)peptide according to the invention wherein said desired (poly)peptide is selected from the group of peptide hormones or fragments of these hormones or (synthetic) analogues from these hormones with antibiotic (-like) activity and/or peptide hormones with similar actions such as dermaseptin, defensin I, bombinin-like peptide, histatin-5, indolicidin, magainin-1 and ceratotoxin A.

In another preferred embodiment, the invention provides a method according to the invention, and a (poly)peptide according to the invention wherein said desired (poly)peptide is selected from the group of biological active peptides or fragments of these peptides and/or hormones or analogues from these peptides and/or peptides with similar actions such as exendin-3, secretin, human pancreatic polypeptide, peptide YY, gastric inhibitory polypeptide, big gastrin-I, pentagastrin, gastrin releasing peptide, motilin, neuropeptide Y, galanin, alpha-neurokinin, deltorphin, alpha-endorphin, beta-endorphin, leu-enkephalin, met-enkephalin, allatostatin I, anthopleurin-A, anti-inflammatory peptide 1, delta sleep inducing peptide, alpha-dendrotoxin, eledoisin, echistatin, small cardioactive peptide A or B, cerebellin, charybdotoxin, conopressin G, conotoxin EI, corazonin, experimental allergic encephalitogenic peptide, experimental autoimmune encephalomyelitis complementary peptide, tocinoic acid / pressinoic acid, brain derived acidic fibroblast growth factor (1-11), brain derived acidic fibroblast growth factor (102-111), brain derived basic fibroblast growth factor(1-24), fibrinogen binding inhibitor peptide, fibroblast growth factor inhibitory peptide and transforming growth factor alpha.

In another preferred embodiment, the invention provides a method according to the invention, and a (poly)peptide according to the invention wherein said desired (poly)peptide is selected from the group of biological active peptides or fragments of these peptides and/or hormones or analogues from these peptides and/or peptides with similar actions such as guanylin, helospectin I, hepatitis B surface antigen fragment, intercellular adhesion molecule, tachyplesin I, HIV (gp 120) antigenic peptide fragment, HIV (gp 41) antigenic peptide I fragment, HIV (gp41) antigenic peptide 5, HIV protease inhibitors, IGF II 69-84, interleukin-8 fragment, interleukin-2 fragment(60-70), leucokinin I, leukopyrokinin, mastoparan, melanin concentrating hormone, melittin, and ras oncogene related peptides.

Considering that lanthionine formation between for example dehydrobutyrine and cysteine is energetically possible at room temperature and can also occur spontaneously the transported (poly)peptide can form thioether bridges spontaneously or where it is positioned in working proximity to extra-cellular located LanC for subsequent enzymatically induced thioether bridge formation. Alternatively, said transporter transports the to-be-modified polypeptide through the membrane of the host cell where it is positioned in working proximity to extra-cellular located LanM for dehydration and subsequent thioether bridge formation.

With this insight, the invention provides a method from which several fields can benefit. In short, the invention provides use of lantibiotic exporters (LanT) for export of peptides or proteins which optionally may have been converted by lantibiotic enzyme(s), in particular enabling extracellular formation of lanthionines and other rings. Amino acids are able to form short sequences (peptides) and longer sequences (proteins). Peptides and proteins [herein also referred to as (poly)peptides] are both important classes of biomolecules, both e.g., for nutrition, for pest control and for fighting disease. Their importance is illustrated by the number and range of therapies based on them recently created by the biochemical and pharmaceutical industries. There is also a large number of protein and peptide based pharmaceuticals and it should also be understood that the use of therapeutic pharmaceuticals is not limited to humans but also extends to animal, plant and other biosystems. However, the manufacture of many present and potential protein or peptide pharmaceuticals has limitations: in particular many are prepared in living cells (*in vivo*) but these cells must be ruptured or lysed (killed), and the contents extracted, separated, and purified, in order to provide a given quantity of the peptide or protein. This is a complex process, and also the amount of any desired peptide or protein in any cell at any time is limited.

The present invention bypasses this problem by introducing into the living cells a factor which allows the cells to continuously transport proteins or peptides and export them through the cell wall, so that the product produced intercellularly may be collected extracellularly and the cells may remain vital and continuing to produce materials. It is evident that this permits both substantially easier and higher rate production of the desired products. Now that is known that said Lan T transporter or functional equivalent thereof acts on unmodified (poly)peptide to which the leader is still attached, one such field relates to the expression and production of recombinant (poly)peptides of other than bacterial descent and relates to expression and/or production of a (poly)peptide of eukaryotic (be it of plant, animal or fungal origin) or viral descent as well. Such peptides are these days widely produced by recombinant means for use in the production of pharmaceuticals, for example as active compound such as a (poly)peptide hormone, or cytokine, or antibody fragment, or biopesticide agent, or as antigen for a vaccine or immunogenic composition. Surprisingly, it is now possible to use a lantibiotic-type transporter system to export peptides of eukaryotic or viral, and not only of bacterial (prokaryotic) descent. In a first embodiment, the invention provides a method allowing for extra-cellular harvest of a desired (poly)peptide-which can be out of the realm of bacterial lantibiotics of even be of eukaryotic or viral descent-produced by a recombinant host cell, said method comprising the steps of: a) selecting a recombinant host cell comprising or provided with a first recombinant nucleic acid having a first nucleic acid fragment encoding a leader peptide and a second nucleic acid fragment encoding said desired (poly)peptide (useful examples of which are given in table 1), whereby said first and second fragment are within the same open reading frame of said first nucleic acid and said leader peptide is at least functionally equivalent to a N-terminal leader peptide found with the prepeptide of a lantibiotic (useful examples of such a leader peptide are given in table 2), and selecting said host cell for the presence of a lantibiotic transporter protein commonly known as LanT, (such a host cell can be a Gram-positive or Gram-negative prokaryote or an eukaryote provided with such a transporter) and allowing for the translation of said first nucleic acid. As said, it is preferred that said cell is essentially devoid of leader peptidase activity, or comprises leader peptidase that cannot cleave the specific leader peptide used. Such a host cell is for example obtained by, at least functionally, deleting the *lan* P gene.

In a preferred embodiment, the invention provides a method allowing for extra-cellular harvest of a desired (poly)peptide which has not undergone intra-cellular post-translational modification comprising dehydration of a serine or a threonine and/or thioether bridge formation. In the detailed description herein, it is for example demonstrated how to obtain nisin prepeptide (i.e. nisin leader and unmodified nisin) extracellularly. The nisin prepeptide was obtained using a host cell selected for the presence of two plasmids, one encoding the nisin prepeptide, and one encoding NisT, whereby said host cell was further characterized by at least the functional absence of at least one of the other gene products derived from the *Nis*-gene cluster, such as NisB, NisC, or NisP.

The invention thus provides a (poly)peptide harvestable after the (poly)peptide has been transported from the producing host cell, obviating the need to lyse or disrupt the host cells to proceed to harvest. However, if one wishes to do so, the desired polypeptide can of course be harvested from within the cell as well. Cultures of cells provided with said transporter protein can now be kept alive and in use, whereby the desired (poly)peptide can be harvested from for example the supernatant of spun-down host cells. These host cells need not be of Gram-positive descent per se, now that Gram-negative prokaryotes or even eukaryotes can be provided with such a properly placed transporter that greatly enhances the gamut of expression systems that can be used to express and produce a desired (poly)peptide.

Furthermore, the invention provides a method allowing for extra-cellular modification of a desired (poly)peptide produced by a recombinant host cell said method comprising the steps of: a) selecting a recombinant host cell comprising a first nucleic acid comprising a first nucleic acid fragment encoding a leader peptide and a second nucleic acid fragment encoding said desired (poly)peptide, whereby said first and second fragment are within the same open reading frame of said first nucleic acid and said leader peptide is at least functionally equivalent to a N-terminal leader peptide found with the prepeptide of a wild-type lantibiotic, and b) selecting said host cell for the presence of a transporter protein commonly known as LanT or a functional equivalent thereof and c) selecting said host cell for the presence of an essentially extra-cellular protein (such as LanB, LanC or LanM) capable of providing post-translational modification, and d) allowing for the translation of said first nucleic acid. In a preferred embodiment, the invention provides a method allowing for extra-cellular modification of a desired (poly)peptide which has not undergone intra-cellular post-translational modification comprising dehydration of a serine or a threonine and/or thioether bridge formation. It is preferred that said essentially extra-cellular enzyme is capable of dehydrating a serine or a threonine, or is capable of providing for thioether bridge formation. Herewith the invention provides a method for lantibiotic-type modification of non-lantibiotic polypeptides, surprisingly even when said (poly)peptide is of essentially eukaryotic or viral descent. This is very useful for altering various characteristics of such products, especially for example related to stability or pharmacological profiles of useful polypeptides, such as selectable from Table 1. It is of course useful to use a leader peptide as selected from Table 2 or functional equivalents thereof.

Furthermore, the invention provides a method allowing for extra-cellular modification of a desired (poly)peptide produced by a recombinant host cell, wherein said modification comprises thioether bridge formation. Preferably, the location of serines, threonines or cysteines in the desired (poly)peptide is selected such that thioether ring formation by the enzyme system selected follows naturally. For example, serine and threonine dehydration followed by thioether ring formation by coupling to cysteines preferably is performed as follows. In the case of lantibiotic enzymes belonging to the so-called type B lantibiotics, ring formation occurs from dehydrated serines/threonines to more C-terminally or to more N-terminally located cysteines. In the case of lantibiotic enzymes belonging to so-called type A lantibiotics ring formation occurs only from dehydrated serines/threonines to more C-terminally located cysteines. Conversion by enzymes belonging to type A lantibiotics occurs in time from N to C-terminal direction from dehydrated serines/threonines to the nearest more C-terminally located available cysteine. In the case of enzymes belonging to type A lantibiotics at a preferential distance of one to four amino acids to available cysteines, lanthionines are formed. It is more preferred that 2 to 3 amino acids are between a dehydrated serine/threonine on the one hand and a cysteine on the other hand. The optimal distance is two amino acids. From Table 1 peptides with above preferred distances for optimal thioetherbridge formation may be selected. At distances between four and thirteen amino acids lanthionine formation can occur but becomes less efficient. At these distances also dehydration of serines and threonines without subsequent lanthionine formation next to absence of dehydration of serine/threonine occurs. It is preferred to have flanking regions of serines and threonines that allow activity of the dehydrating enzyme. To help achieve this, it is preferred that at least the six to eight amino acids (three to four on each side) surrounding dehydrated serines/threonines are mostly hydrophobic. At each of these positions in 40-80% of the cases the amino acid is preferably hydrophobic, in 20-40% hydrophilic, of which in 5-15% are positively charged. It is preferred that negatively charged amino acids hardly occur. The composition of the flanking regions on the desired (poly)peptide preferably differs from the one of serine and threonine in lantibiotic-type leader peptides. In leader peptides serines and threonines occur but are never dehydrated, whereas cysteines do not occur. The six to eight positions most closely to leader serines/threonines contain less hydrophobic amino acids and more negatively charged amino acid than in positions around propeptide serine/threomine; per position in only 20-40% of the cases the amino acid is hydrophobic and in around 20% of the cases a negatively charged amino acid is preferred.

With respect to the peptidase cleavage site at least two types of leader peptides exist from which guidance can be obtained to design better cleavable peptides or proteins. One class needs the subtilisin-like serine protease LanP for cleavage, which occurs after Pro-Gln, Pro-Arg, Ala-Asp, Ala-Glu. In the case of nisin a positively charged residue at position -1 and a hydrophobic residue at position -4 seem necessary for interaction with NisP. This subtilisin-like serine protease LanP acts on the prepeptides of for instance Pep5, Epilancin K7, Nisin A, Nisin-Z, Epidermin, Gallidermin.

In the other class the leader peptides are cleaved after Gly-Gly, Gly-Ala or Gly-Ser sequences. The latter holds for many other non lantibiotic bacteriocin leader peptides. The subtilisin like proteases are not known to cleave these sequences, hence a different type of protease is cleaving these leader peptides. It has been shown that in some bacteriocins -both lantibiotic and non lantibiotic-this second protease is a domain of the transport system LanT. This type of leader peptidase acts for instance on prepeptides of Lacticin-481, Variacin, Mutacin-II, Streptococcin-A-FF22, Salivaricin-A and Sublancin.

In addition a two component lantibiotic, Cytolysin-LL / Cytolysin LS, exists of which each component is cleaved twice, once by the "double glycine type" and thereafter by the subtilisin-like peptidase.

The invention furthermore provides a method for the modification of a desired polypeptide according to the invention wherein said host cell is a Gram-negative prokaryote or an eukaryote. Furthermore, the invention provides a (poly)peptide modified with a method according to the invention.

Also, the invention provides a host cell, such as a Gram-negative prokaryote or an eukaryote, provided with a recombinant nucleic acid comprising a first nucleic acid fragment encoding a leader peptide and a second nucleic acid fragment encoding a desired (poly)peptide, whereby said first and second fragment are within the same open reading frame of said first nucleic acid and said leader peptide is at least functionally equivalent to a N-terminal leader peptide found with the prepeptide of a lantibiotic. In a preferred embodiment, a host cell according to the invention is provided wherein said desired (poly)peptide is of essentially eukaryotic or viral descent, for example selected from Table 1 and/or wherein said leader peptide is selected from Table 2.

Furthermore, the invention provides a host cell according to the invention said host cell provided with or selected for the presence of at least a LanT protein or functional equivalent thereof wherein said host cell is further characterized by at least the functional absence of at least one of the other gene products derived from the *Lan*-gene cluster, such as LanB, LanC, (or a functional part from LanM) or LanP. In a preferred embodiment, said host cell comprises a Gram-negative prokaryote or an eukaryote.

Such a host cell as provided herein finds a specific use in a method of producing a (poly)peptide for harvest or modification, as provided herein above. For the purpose of harvest it is especially preferred that LanT is present but that LanB and/or Lan C (or LanM), but preferably both, are absent, at least functionally absent in that they are hampered in binding to or interfering with the polypeptide to be harvested. For the purpose of modification, it is especially preferred that LanT and an essentially extra-cellular protein allowing extra-cellular modification, such as LanB, Lan C or LanM, or instead of LanB, a (preferably N-terminal) LanM fragment having LanB function or instead of LanC, a (preferably C-terminal) LanM fragment having LanC function is present, whereas a further extended or even complete lantibiotic gene-product cluster is preferably not, at least not functionally present.

Another embodiment of the invention is a host cell which is provided with genes coding for LanB, or the equivalent N-terminal part of LanM, with or without a gene coding for LanT, which is capable of exporting dehydrated lantibiotic prepeptides, which have mutations such that chemically or proteolytically fragments can be liberated that are provided with dehydro alanine and/or dehydrobutyrine. Such fragments can inhibit an enzyme, specifically a protease, such as cysteine protease or aspartyl protease.

Furthermore, the invention provides a recombinant nucleic acid comprising a first nucleic acid fragment encoding a leader peptide and a second nucleic acid fragment encoding a desired (poly)peptide, whereby said first and second fragment are within the same open reading frame of said first nucleic acid and said leader peptide is at least functionally equivalent to a N-terminal leader peptide found with the prepeptide of a lantibiotic, and wherein said desired (poly)peptide is of essentially eukaryotic or viral descent. Furthermore, the invention provides an proteinaceous substance comprising a polypeptide encoded by a nucleic acid according to the invention. Such a proteinaceous substance can be harvested, or modified according to a method as provided herein. Use of a host cell or nucleic acid or proteinaceous substance according to the invention for the production of a desired (poly)peptide, and its use in producing a pharmaceutical composition is herein also provided. In particular, the invention provides a (poly)peptide of Gram-negative prokaryotic, viral or eukaryotic descent (examples can be found in Table 1) wherein a serine or threonine has been dehydrated or which has been provided with a thioether bridge. The advantage of such a polypeptide for example lays in the creation of variants of known peptide or protein based drugs, where for example the dose or frequency of administration can be reduced, thus lowering treatment cost, treatment time, and patient inconvenience; the creation of variants of new protein or peptide based drugs where the drug may not have been effective or admitted for use in an unstabilized form; and the creation of new therapeutic entities *per se.*

The invention is further explained in the detailed description.

### Figure legends

Fig. 1 Peptide structure of mature nisin and subtilin
Fig. 2 Genomic organisation of genes involved in subtilin and nisin biosyntheses
Fig. 3 Model for nisin biosynthesis wherein modification occurs intra-cellularly.
Fig. 4 Model for nisin biosynthesis wherein modification occurs extra-cellularly.
   Nisin prepeptide is exported by NisT ("T"), dehydrated by extracellular NisB (B) and subjected to thioether ring closure by extracellular NisC (C).
   Extracellular leader peptidase, NisP (P) cleaves of the leader peptide. Nisin interacts with a membrane bound histidine kinase NisK (K) which phosphorylates a response regulator NisR (R), which in its turn switches on transcription of the *nis*-genes (+, +). The producer cells are protected against nisin by the concerted action of the lipopeptide NisI and the transport system NisEFG.
Fig. 5
   Detection of lantibiotic leader peptide directly from the culture medium by MALDI-TOFMS.
   By using the combination of growth in minimal medium and ziptipping the supernatant of this culture, samples of sufficient purity were obtained for high resolution MALDI-TOFMS. This allowed most significant measurement of nisin leader peptide. This has to our knowledge never been reported. The detection of lantibiotic leader peptide therefore can be used to ascertain the export of (poly)peptide coupled to this lantibiotic leader, i.e. in those cases where the leader peptidase acts extracellularly. Generally this method allows a high detection level for measuring (lantibiotic) (poly)peptides directly from the culture supernatant.
Fig 6.
   Transport of unmodified nisin prepeptide via the nisin transporter NisT. (Example 1)
Fig. 7.
   Transport via NisT of an angiotensin1-7 variant fused to the C-terminus of the nisin leader. (Example 2)
Fig. 8.
   Transport via NisT of a vasopressin variant fused to the C-terminus of the nisin leader. (Example 3)
Fig. 9.
   Transport and dehydration by NisBT of nisin prepeptide. (Example 4)
Fig. 10AB
   Transport, dehydration and ring formation in nisin prepeptide by Lactococcus lactis cells having plasmid pNGnisABTC.

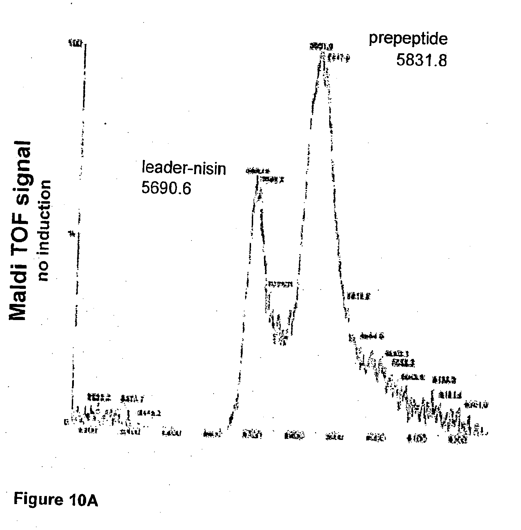

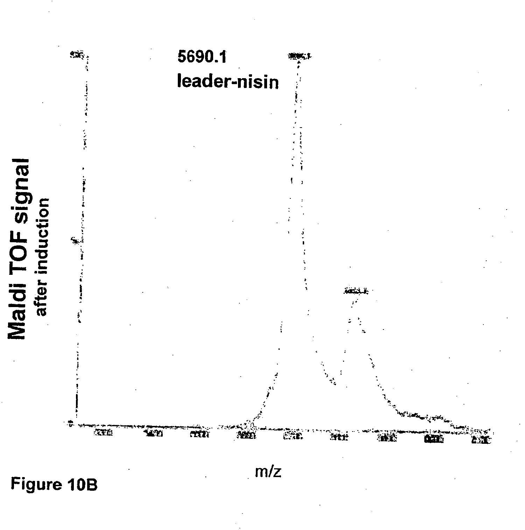

Fig. 10A: no induction, Fig. 10B: induction (Example 6)
Fig. 11
   Transport via NisT of unmodifed nisin prepeptide, C-terminally extended with an enkephalin variant. (Example 13).

Overnight cultures of nisin producing *Lactococcus lactis* NZ9700 grown in M17 broth supplemented with 0.5% glucose were diluted 1/100. At optical density at 660 nm equal to 0.4, cells were centrifuged and the medium was replaced by minimal medium (Jensen and Hammer, 1993. Appl. Environ. Microbiol. 59: 4363-4366) containing 1/1000 of 0.4 mm pore filtered overnight *Lactococcus lactis* NZ9700 supernatant. After overnight incubation the medium was ziptipped using C18 ziptips (Millipore). As matrix for MALDI-TOFMS analysis a cyano cinnaminic acid was used.

The picture shows a peak at 2349.6 corresponding to the nisin leader peptide (theoretical value of 2351.2). Two subpeaks, of 2372.5 and of 2388, correspond to sodium and potassium adducts respectively. One peak of 2482.2 corresponds to the nisin leader peptide with the first methionine still attached (theoretical value is identical: 2482.2). At higher mass the peaks 3352.5, 3372.6 and 3390.1 correspond to nisin, a sodium adduct and a potassium adduct respectively.

### Detailed description.

Lantibiotic enzymes are special. There is no strong homology if any at all with other enzymes. DNA and amino acid sequences of many lantibiotic enzymes are known, but no structures have been determined. The genes involved in the biosynthesis, processing and export of lantibiotics are present in a *lanA B C* / *M (D) P R K T F E G I* cluster. There is no uniform order or orientation of the genes in the different clusters indicating that rearrangements have occurred in the evolution. Lanthionines are the most typical post-translationally formed residues in antibiotics. They are formed via two steps. First propeptide serines and threonines are dehydrated giving rise to dehydro-alanines and dehydrobutyrines respectively. LanB has been proposed to play a role in dehydration, since it has a weak homology to IIvA, a threonine dehydratase from *E.coli.* Moreover it has been shown that overexpression of NisB increases the occurence of dehydration of serine 33 in nisin A, from 10% in the normal situation up to 50% in the case of overexpressed NisB. The LanB protein consists of about 1000 residues. LanB are membrane associated proteins. LanC is thought to be responsible for the subsequent addition of cysteine SH groups to the dehydro amino acids, which results in the thioether rings. In the case of PepC experimental data.support this idea. The presently known LanC proteins are composed of about 400 residues. In type A lantibiotics the N-terminal part of lanthionine and methyllanthionine residues are formed by the dehydroalanine or dehydrobutyrine residues, whereas the C-terminal half is formed by the cysteine residues.

Dehydroalanines and dehydrobutyrines are essential in various (poly)peptides for the activity of the specific (poly)peptide. Dehydroresidues are for instance essential for the nisin-mediated inhibition of the outgrowth of bacterial spores (Liu and Hansen 1996. Appl Environ. Microbiol. 59:648-651), for a neurokinin receptor antagonist (Lombardi et al 1998. Bioorganic and Medicinal Chemistry Letters 8 : 1153-1156), for phenylalanine ammonia lyase (Schuster and Rétey 1995 PNAS 92:8433-8437) for an inhibitor of tripeptidyl peptidase II (Tomkinson et al., 1994. Archives of Biochemistry and Biophysics 314: 276-279), for a peptide inhibitor of HIV-1 protease (Siddiqui et al. 2001. Indian Journal of Biochemistry and Biophysics. 38: 90-95), for activity of peptides against Gram-negative bacteria (Ferrari et al 1996 2:4- Journal of Antibiotics) and for activity of antifungal peptides (Kulanthaivel et al WO 2000063240). (Poly)peptides containing dehydroresidues can be produced by cells having LanT and LanB or

LanT and the N-terminal part of lanM which is equivalent to LanB. The above mentioned activities can be of significant economic importance. For instance inhibition of tripeptidyl peptidase II is of relevance in the battle against obesity. This is evident from the fact that tripeptidyl peptidase II degrades octapeptide cholcystokinin-8, an endogenous satiety agent.

Lanthionine formation between dehydrobutyrine and cysteine is energetically possible at room temperature and can also occur spontaneously.

Lantibiotic maturation and secretion is thought to occur at membrane-associated multimeric lanthionine synthetase complex consisting of proteins LanB, LanC and the ABC transporter molecules LanT. At least two molecules of LanC and two molecules of LanT are part of the modification and transport complex. Some lantibiotics do not have the *lanB* gene, but have a much larger *lanM* gene, whose product has C-terminally some homology with the *lanC* gene product. Since no *lanB* homologue is present in LanM producing clusters the N-terminal part of the LanM protein might fulfil the dehydration reaction typically performed by LanB. The chemical synthesis of lantibiotics is possible but extremely costly and time consuming. Several mutant lantibiotics that contain amino acid substitutions have been obtained by genetic engineering. However, despite many studies, until now only in the lantibiotic Pep5 one lanthionine ring in a new position has been obtained.

As said, the lantibiotic export systems, LanT, (whose sequences are already known) are in general thought to be dedicated for the transport of the fully modified lantibiotic. Indeed the two enzymes involved in the lanthionine formation in nisin (NisB and NisC) have been reported to be located intracellularly in a NisBCT membrane associated complex (Siegers et al., 1996). Such intracellular localization suggests that the prepeptides are dehydrated by LanB whereafter rings are formed by LanC followed by export by LanT. Furthermore if the thioether ring forming enzymes, NisB (responsible for dehydration, which is the first step in ring formation) or NisC (responsible for ring formation between dehydro residues and cysteines) are inactivated by in frame deletion of 61 aa or by plasmid insertion respectively, no peptide is exported any more. The latter suggests that absence of (methyl)lanthionines prevent export.

However, it has now surprisingly been measured that prepeptide, such as nisin prepeptide can be transported through the nisin transporter. This result was obtained using a strain with two plasmids, one coding for the nisin prepeptide and one coding for the nisin transporter. No prepeptide production was observed in a control experiment in which a strain with only the plasmid coding for the prepeptide was used. Some lantibiotics contain dehydrated serines/threonines that do not participate in thioether ring formation. From the latter, in combination with the observation that unmodified peptide is exported, it may be theorized that also translocation or prepeptide without thioether rings but with dehydro residues is possible. It is known that the second step in lantionine ring formation is less difficult to achieve since it can also occur spontaneously at room temperature. Therefore after production of prepeptides with dehydro residues lanthionine rings can be formed extracellularly.

In order to avoid cellular incompatibilities with newly formed thioether and or dehydroresidue containing (poly)peptides, *in vitro* synthesis of thioether (poly)peptides can be performed. Inside out membrane vesicles with LanB or LanBC or LanBT or LanBCT or LanMT, obtained by french pressing cells, can be mixed with a cell extract, obtained by sonicating a cell pellet and centrifugation, with ATP and an ATP generating system, with protease inhibitors and with a leader-(poly)peptide fusion with serines/threonines and cysteines in adequate positions. In the case of LanC or LanCT containing vesicles peptides with dehydro residues can be closed by LanC to form stereospecifically thioether rings. After vortexing with one volume of chloroform, and centrifugation the supernatant contains the fusion peptide with thioether rings and or dehydroresidues as shown by Maldi TOF analysis of the ziptipped supernatant. The formation of rings follows the observed mass after peroxydation since peroxydation gives an addition of three oxygens to free cysteines occurs, whereas only 1 or 2 oxygen atoms to thioether bridges.

For the in *vitro* activities of LanB, LanC and lanM, instead of using membrane vesicles obtained by french pressing also isolated lantibiotic enzymes produced by bacterial or eukaryote organisms can be reconstituted in membrane vesicles or liposomes.

LanC (or C-terminal LanM) (-containing vesicles or proteoliposomes) can also be used *in in vitro* assays for generating stereospecific lanthionines in chemical lanthionine forming procedures, which in the absence of lanC yield diastereomers (Galande and Spatola 2002. Letters in Peptide Science 8:247-251).

The present finding provides the possibility to make new lantibiotics and thus to stabilize peptides / proteins by thioether rings, D-alanines or other residues formed by lantibiotic enzymes. Before (methyl) lanthionine formation, typically the distance of dehydro residues to cysteines is 2-5 residues but also much larger distances are possible. (Methyl)lanthionines can be formed from dehydro residues either to more N-terminally located or to more C-terminally located cysteines. In addition the lantibiotic transport system can be used for the export of other proteins by inserting the sequence coding for the leader peptide in front of the protein DNA sequence.

Short (poly)peptides with dehydroresidues and/or thioether rings can also be obtained by embedding them in a DNA lantibiotic sequence. For instance into a specific eukaryotic peptide of 10 amino acids a thioether ring can be engineered as follows. Based on a lantibiotic of 20 amino acids with a thioether ring between position 13 and 16 a DNA sequence can be designed coding for the first 10 amino acids of the lantibiotic followed by the 10 amino acids of the eukaryotic peptide with a serine in position 13 and a cysteine in position 16. By genetically introducing a (chemical) cleavage site the resulting hybrid peptide, exported in the medium, can be cleaved and the eukaryotic peptide with thioether ring is liberated.

It has been described that a (poly)peptide can be genetically appended behind a lantibiotic and exported (Hansen, US2002019518: Construction of a strain of bacillus subtilis 168 that displays the sublancin lantibiotic on the surface of the cell. However it is also possible to append polypeptides genetically behind a lantibiotic sequence and have the resulting fusion (poly)peptide without modification exported via LanT, or exported via LanBT with just dehydration of serines and threonines in the lantibiotic and in the appended (poly)peptide without ring formation or exported via LanBTC/LanMT with dehydration and ring formation in both the lantibiotic and in the appended (poly)peptide.

It is also possible to generate a lanthionine and/or dehydroresidue containing (poly)peptide by omitting a C-termnal fragment of a lantibiotic sequence and adding a longer fragment to the remaining lantibiotic sequence. For instance the sequence of the 34 amino acid lantibiotic nisin can be replaced by the fist 30 N-terminal amino acids, and C-terminally extended by 6 amino acids: KYSGFC. After dehydration and thioether ring formation by cells with NisBTC, and extracellular trypsin treatment, a lanthionine variant of enkephalin is produced. In this case the nisin Ser33 residue is taking part of the newly formed enkephalin molecule after dehydration and lanthionine formation. Trypsin liberates the enkephalin by cleaving behind the lysine residue that replaces the hisitidine in nisin. Active lanthionine variants of enkephalin are known (Svenssen et al 2003. Journal of Pharmacology and Experimental Therapeutics 304: 827-832).

Several uses are already foreseen. For instance peptide / protein drugs that are rapidly degraded in the blood plasma can be protected against proteolysis by thioether rings. Also, new lantibiotics can be used as antibiotics especially against Gram-positive bacteria. This is useful since there is a growing and spreading resistance against classical antibiotics. Also, new lantibiotics can be used as (food) additives to prevent bacterial growth and increase the shelf life of (food) products. Mastering the enzymatic synthesis of thioether rings further furnishes the possibility of synthesizing an broad variety of new antimicrobial peptides, which gives many possibilities to circumvent resistance. Lantibiotics have a variety of antimicrobial activities: membrane permeabilization, inhibition of cell wall synthesis, modulation of enzyme activities, inhibition of outgrowth of spores. New lantibiotic-type peptides or proteins are more stable (i.e. less prone to proteolytic cleavage) and can have modulated activity or a different spectrum of activity. A selection of such peptides or proteins is herein provided in the examples given below. Dehydro-peptides can be engineered which can be used to block enzymatic activity, especially protease activity.

### EXAMPLE 1

### The NisT transporter can transport unmodified nisin prepeptide

This example involves a *Lactococcus lactis* strain that lacks the entire chromosomal nisin gene cluster, but produces simultaneously plasmid encoded NisT and the NisA prepeptide. Unmodified NisA can be found in the culture supernatant, which demonstrates that NisT is sufficient for the transport of unmodified prepeptides to the exterior of the cell.

### Materials and Methods:

Use for the nisin inducible expression of *nisT* in *Lactococcus lactis* a pNZ8048 (Kuipers et al. 1997. Tibtech. 15: 135-140) derived plasmid. Amplify the *nisT gene* using primers NisT.fw (5'-CGG TCT CCC *ATG* GAT GAA GTG AAA GAA TTC ACA TCA AAA C) and NisT.rev (5'-CGG TCT CTC TAG ATT ATT CAT CAT TAT CCT CAT ATT GCT CTG) with chromosomal DNA of NZ9700 (a nisin producing *L. lactis* strain; Kuipers et al. 1997. Tibtech. 15: 135-140) as template.

Use as PCR conditions: 5 min 94 °C, 30 times [30s 94 °C, 30s 50°C, 3 min 72°C], 10 min 72°C. Purify the PCR product with the Roche PCR-isolation kit. Digest the expression vector with *N*coI/*Xba*I and the PCR fragment with *Eco*31I (underlined in the primers, the sticky ends it generates are indicated in italics and are compatible with *Nco*I and *Xba*I) and ligate subsequently the fragments using T4 ligase (Roche). Designate the resulting plasmid *pNG-nisT.* This plasmid contains a chloramphenicol (Cm) resistance gene as selection marker.

Use for the nisin inducible production of the NisA prepeptide in *L. lactis* a variant of pNZ8048 that contains an erythromycin (Em) resistance selection marker instead of a Cm marker. Amplify the nisA gene using primers NisA.fw (5'-CGG TCT CTC *ATG* AGT ACA AAA GAT TTT AAC TTG GAT TTG G) and NisA.rev (5'-TAT ATG *GAT C*CT TTG CTT ACG TGA ATA CTA CAA TGA CAA G) and chromosomal DNA of strain NZ9700 as template under the same conditions as described above. Purify the PCR product with the Roche PCR-isolation kit. Digest the expression vector with *Nco*I/BamHI and the PCR fragment with *Eco*31I and *Bam*HI (underlined in the primers, the sticky ends it generates are indicated in italics and are compatible with *Nco*I and *Bam*HI) and ligate subsequently the fragments using T4 ligase (Roche). Designate the resulting plasmid pNG-*nis*A.

Grow *L. lactis* strains NZ9000 or PA1001 (a NZ9000 derivative lacking AcmA activity to abolish cell lysis (Buist et al. 1995. J. Bacteriol. 177: 1554-1563) and lacking HtrA to diminish extracellular proteolytic activity (Poquet et al. 2000. Mol. Microbiol. 35: 1042-1051) with both pNG-*nisT* (Cm) and pNG-*nisA* (Em) in M17-based medium (Terzaghi and Sandine. 1975. Appl. Microbiol. 29: 807-813) to an OD₆₀₀ of 0.4. Collect the cells by centrifugation and resuspend in the same volume of Minimal Medium (Jensen and Hammer, 1993. Appl. Environ. Microbiol. 59: 4363-4366) and induce for expression of NisT and NisA prepeptide by addition of nisin as described before (Kuipers et al. 1997. Tibtech. 15: 135-140). After overnight induction and subsequent centrifugation, pipet the culture supernatants up and down in C18 ziptips (Millipore): two times 10 µl 50% acetonitril followed by two times 10 µl demineralized water followed by eight times 10 µl supernatant, followed by two times washing with 10 µl demineralized water, followed by elution by using 2 times 10 ul 50% acetonitril containing 0.1% TFA. Vacuum dry the final eluent and store at -20 °C until analysis by mass spectrometry. Prior to analysis resuspend the dry material in 2.5 µl of 50% acetonitril containing 0.1% TFA and apply 1 µl to the target. After drying, apply 1 µl of matrix (10 mg/ml alpha-cyano-4-hydroxycinnamic acid completely dissolved (by mildly heating and vortexing) in 50% acetonitril containing 0.1% TFA) to the target. Use the following MALDI-TOFMS (linear mode) laser settings: 100% coarse energy, 50% fine, source 20KV, extra 19800, force 15000, suppression 500, pulse time 40, pulse voltage 2200, sampling rate 500 MHz, sensitivity 50mV, shots 15.

### Results

Analyse culture-supernatants of the following induced cultures and analyse by MALDI-TOFMS:
NZ9000 (or PA1001)
NZ9000[pNG-*nisA*] (or PA1001[pNG-*nisA*])
NZ9000[pNG-*nis*A + pNG-*nisT*] or (PA1001 [pNG-*nisA* + pNG-*nisT*])

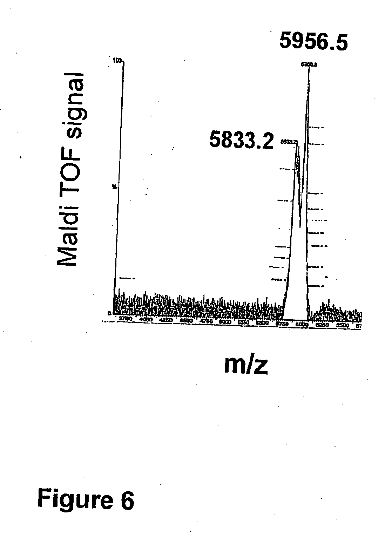

Observe no peaks in samples derived from cultures A and B. Measure in sample C two main peaks: Figure 6. The first one close to or identical to 5832.8 Da corresponding to the unmodified nisin prepeptide: (5831.8 plus 1 proton). The second with about 130 Da higher mass than the first one, which might correspond to the nisin prepeptide with two zinc atoms (Bierbaum, G. 1999. Ed. J. W. Kelly. Amino acids, Peptides, Porphyrins, Alkaloids 4, 275-301. Elsevier. Comprehensive Natural Products Chemistry. Eds. D. Barton, K. Nakanishi & O. Meth-Cohn) or -more likely- to the nisin prepeptide with the methionine in position 1 still present. This result is consistent with unmodified nisin prepeptide being transported by the nisin transporter NisT. This result demonstrates unequivocally that NisT is sufficient for the transport of the nisin prepeptide and that modification prior to transport is not required.

### EXAMPLE 2

### Transport via NisT of a fusion peptide of the nisin leader and an angiotensin variant

This example describes_the transport out of *Lactococcus lactis* via the nisin transporter NisT of a variant of angiotensin¹⁻⁷ which is preceded by the nisin leader. It is shown once more that the nisin transporter is not specific for nisin, but that many (poly)peptides can be transported provided that they are fused to the nisin leader.

### Materials and Methods.

As in example 3, precise genetic fusion of a eukaryote peptide, in this example angiotensin 1-7, behind the nisin leader is obtained.

Obtain the gene of the angiotensin1-7 variant by annealing two phosphorylated oligo's: ang1:5' ACGCAATCGTTCTTATATTTGTCCTTAAG 3' and ang2: 5' GATCCTTAAGGACAAATATAAGAACGATT 3'

The annealed fragment includes a stopcodon and has at its 5'-end the ACGC overhang and at the 3'-end a *Bam*HI compatible sticky end. Ligate the annealed gene fragment into *Eco*31I and *Bam*HI digested pLP1 and designate the resulting plasmid pLP1ang.

Induce strain PA1001 carrying pNGnisT (example 1) and pLP1ang for expression as described in example 1. Perform purification of the secreted peptide and analyses by MALDI-TOFMS (linear mode) essentially as described in example 1.

### Result

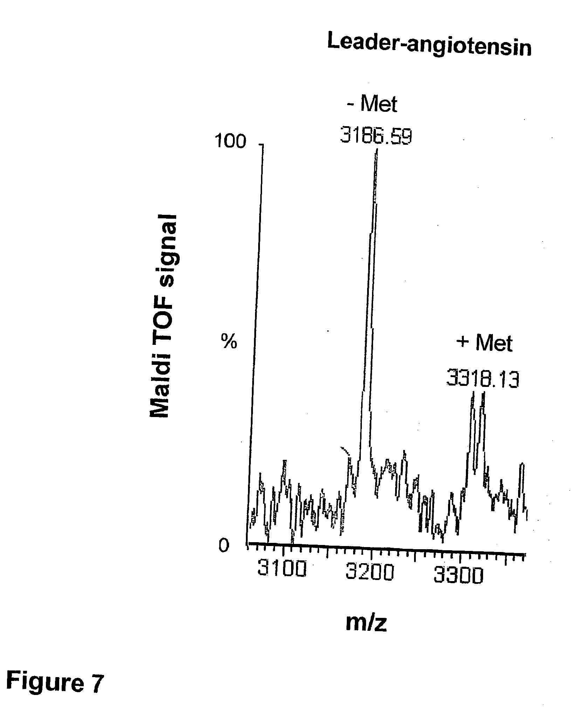

Figure 7: Samples derived from the ziptipped supernant of PA1001 + pNGnisT + pLP1ang show a Maldi TOF peak coresponding to the nisin leader fused to the angiotensin 1-7 variant. This peak is absent in samples derived from cells with just pNGnisT or with just pLPang1. These data prove that the leader-angiotensin fusion peptide can be transported out of the cell via NisT.

### EXAMPLE 3

### Transport via NisT of a fusion peptide of the nisin leader and a vasopressin variant.

This examples shows export out of *Lactococcus lactis* PA1001 via NisT of a fusion of the nisin leader C-terminally extended with a vasopressin variant. Vasopressin is a 9 amino acid (aa) peptide antidiuretic hormone. It has cysteines in position 1 and 6: CYFQNCPRG that form an internal disulfide bond. This example involves a C1S vasopressin variant. This example involves precise fusion of serine altered vasopressin (SerVaso) to the NisA leaderpeptide again by genetic modification.

### Materials and methods

To obtain a precise and in frame fusion of SerVaso with the NisA leaderpeptide, convert first a pNZ8048 expression vector derived plasmid pNG-*nisl-SC3*, that contains SC3 behind the nisin leader into a general NisA leaderpeptide secretion vector. Introduce suitable restriction sites and remove the c-myc-SC3 sequences by PCR amplification of the entire plasmid using primers: pLP.1 (5'-CGG TCT CA*G CGT GGT* GAT GCA CCT GAA TC) and pLP.2 (5'-CCA *CGC* TGA GAC CGC AGC TGG GAT CCG GCT TGA AAC GTT CAA TTG AAA TGG). Cut the PCR product with Eco31I (underlined sequences in the primers) resulting in sticky ends (in italics in the primer sequences) that are compatible for self-ligation of the plasmid. After self-ligation the resulting plasmid, pLP1, can be used for precise fusion of peptides and proteins after the ...GASPR aa sequence of the NisA leaderpeptide by making use of the *Eco*31I restriction site. DNA fragments to be inserted at this position should then contain a 5'-ACGC sticky end to allow ligation. At the 3'-end the DNA fragment should contain a sticky end that is compatible with *Bam*HI (site introduced by primer pLP.2, indicated in bold).

Obtain the SerVaso gene by annealing two oligo's: VP. 1: 5'-ACG CTC ATA TTT TCA AAA TTG TCC TCG TGG TTA AG and VP.2:5'-GAT CCT TAA CCA CGA GGA CAA TTT TGA AAA TAT GA. The annealed fragment includes a stopcodon and has at its 5'-end the ACGC overhang and at the 3'-end a *Bam*HI compatible sticky end. Ligate the SerVaso gene fragment into *Eco*31I and *Bam*HI digested pLP1 and designate the resulting plasmid pLP1vp.

Induce strain PA1001 carrying pNGnisT (example 1) and pLPvp for expression as described in example 1. Perform purification of the secreted peptide and analyses by MALDI-TOFMS (linear mode) essentially as described in example 1.

### Result

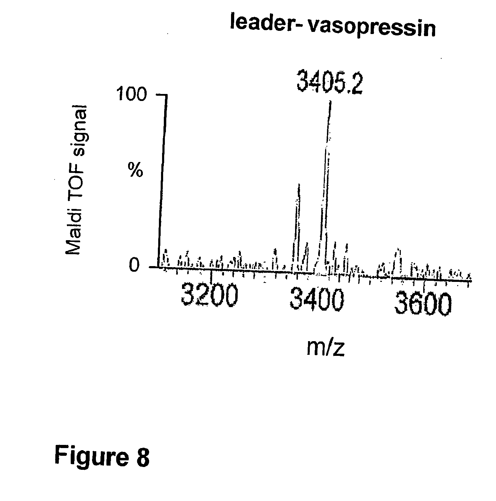

Fig. 8. A MALDI-TOFMS mass consistent with export of the fusion peptide of nisin leader C-terminally extended by C1S vasopressin. This result demonstrates that a eukaryote peptide can be fused to a lantibiotic leader peptide and exported via a lantibiotic transporter as such.

### EXAMPLE 4

### Export of nisin prepeptide via the nisin transporter NisT and modification by the nisin dehydrating enzyme NisB without subsequent enzymatic thioether bridge formation.

### Materials and methods

Clone *NisBT* as in example 1 using the primers nisB fw, (5'-CGG TCT CGC ATG ATA AAA AGT TCA TTT AAA GCT CAA CCG TTT TTA GTA AG) and nisT rev (5'-CGG TCT CTC *TAG* ATT ATT CAT CAT TAT CCT CAT ATT GCT CTG). Transform NZ9000 + pNG*-nisBT* (Cm) with pNG-*nisA* (Em) (constructed as in example 1). Grow in minimal medium NZ9000 + *pNG-nisBT* + pNG-nisA cells and induce as in example 1. Ziptip the supernatant and analyse by MALDI-TOFMS as in example 1.

### Results

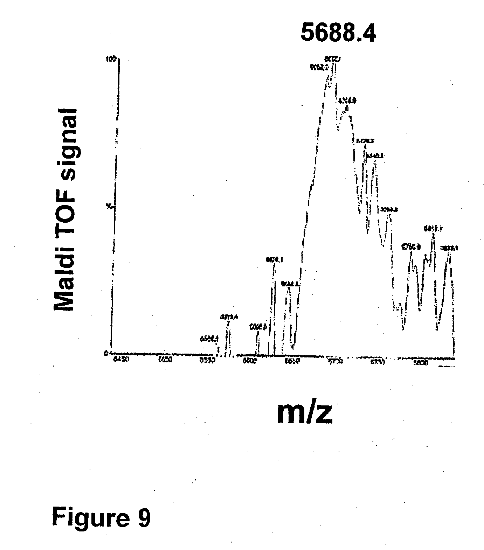

Fig. 9. A MALDI-TOFMS a peak around 5690 Da in the sample derived from the supernatant from NZ9000 + pNG*-nisBT* + pNG-*nisA* cells. Absence of this peak in samples derived from the supernatant of NZ9000 + pNG-*nisT* + + pNG-*nisA* (example 1). Consistence with dehydration of most serines (probably Ser33 remains untouched as in nisine itself) and all threonines.

### EXAMPLE 5

### Export of nisin prepeptide via the nisin transporter NisT and modification by the nisin dehydrating enzyme NisB without subsequent enzymatic thioether bridge formation.

### Materials and Methods

Construct the plasmid pNG-*nisABT* similar to the organisation of these genes in the wild type nisin producer NZ9700, which is with an inverted repeat between the *nisA* and the *nisBT* genes. In short: PCR on Chromosomal DNA with primers nisAfw: 5' CGG TCT CTC ATG AGT ACA AAA GAT TTT AAC TTG GAT TTG G 3' and nisTrev: 5' CGG TCT CTC TAG ATT ATT CAT CAT TAT CCT CAT ATT GCT CTG 3'. Clone the PCR product into pGEM-T (A-T ligation). Digest pGEM-TnisABT with BsaI. Ligate nisABT (BSAI) with pNG8048E(ncoI/XbaI). Transform this plasmid to NZ9000, grow and induce. In this case after induction *nisBT* are transcribed only in low quantity by limited readthrough. Subject the supernatant to ziptipping and MALDI-TOFMS as in example 1.

### Results

A MALDI-TOFMS peak around 5690 consistent with export of the nisin prepeptide and dehydration of propeptide serines (most) and threonines (all).

### EXAMPLE 6

### Export of nisin prepeptide via the nisin transporter NisT and modification by the nisin dehydrating enzyme NisB followed by NisC-mediated thioether bridge formation, involving a pNG-nisABTC plasmid.

### Materials and Methods

Construct the plasmid pNG-*nisABTC* similar to the organisation of these genes in the wild type nisin producer NZ9700, which is with an inverted repeat between the *nisA* and the *nisBTC* genes. This construction can be performed analogous to the construction of pNGnisABT described in example 5, using - instead of nisT rev- nisC rev: 5' CGG TCT CTC TAG ATC ATT TCC TCT TCC CTC CTT TCA AAA AAT C 3'.

Alternatively, and this is the construction of the *nisABTC*-containing plasmid with which the presented data were obtained, clone the *nisABTC* genes on a gateway plasmid. Restrict pNG8048E with HindIII. Remove the Em-r containing fragment by isolating the vector fragment (3kb) from gel. Self-ligate the vector fragment and designate the resulting vector pBMDL1. PCR pBMDL1 with to loose the Cm-r and introduce a PstI site and a XbaI site. Isolate this fragment isolated out of gel and restrict with PstI and XbaI. Cut pNG8048E also with PstI and XbaI to obtain the Em-r fragment. Isolate this 1 kb fragment out of gel and ligate with the former PCR-product. Term the new plasmid pBMDL2. Restrict pBMDL2 with SmaI to linearize it and to obtain blunt ends. Insert a Gateway Vector Conversion Cassette (RfA) (1.7 kb) by blunt-end ligation. Thus obtain a vector to be termed pBMDL3. To prepare a vector that contains the *nisABTC* genes, introduce Gateway's attB-sites by means of PCR. The 6.4 kb PCR-fragment was cleaned over a Zymoclean DNA Clean & Concentrator Kit column. A BP-reaction was performed with this PCR-product and pDONR201 (Invitrogen) during ON incubation at 25C. Obtain the entry vector pBMDL4 in *E.coli* DH5alpha cells via chemical transformation. Perform with this entry vector pBMDL4 and the already created pBMDL3 a LR-reaction. Obtain the vector pBMDL5 (with *nisABTC*) in *E.coli* EC1000 (contains RepA on chromosome) via electroporation. Isolate pBMDL5 (containing *nisABTC*) and transform into PA1001 (electroporation).

Grow NZ9000 + pBMDL5 (or pNGnisABTC) and compare induced and uninduced samples. Analyse the supernatant as in example 1. Next to this, subject trypsin treated supernatant to a growth inhibition assay of nisin sensitive, erythromycin resistant *Lactococus lactis.* In addition test the trypsinated supernatant for its capacity to induce the nisin promotor with the Gus assay (Kuipers et al. 1995. J. Biol. Chem. 270:27299-27304)

### Results

In the uninduced sample a small peak is visible of modified prepeptide (dehydration and lanthionine formation) and a larger peak of unmodifed prepeptide (Fig 10A). In the induced sample a large peak is visible of modified prepeptide (dehydration and lanthionine ring formation) and a small peak which might correspond to modified prepeptide with methionine 1 (Fig 10B). Trypsinated samples of both induced and uninduced supernatants are able to induce the nisin promoter as measured by the gus assay. Induced and trypsinated supernatants have growth inhibitory capacity comparable to the supernatant of the wild type nisin producer NZ9700. Apparently the nisin promoter is leaky. This result on the uninduced sample confirms again the result of example 1 that unmodified prepeptide can be exported.

These results of the induced samples are consistent with export of the nisin prepeptide which has undergone all lanthionine bridge formations. Trypsin cleaves of the leader liberating active nisin with antimicrobial activity and inducing capacity. NisBTC are therefore sufficient for lanthionine formation.

### EXAMPLE 7

### EpilancinBC-mediated synthesis by Staphyloccus epidermis (prokaryote, Gram-positive) of epilancin leader (table 2) coupled to glucagon (table 1) with thioether rings.

The [C5, S24, C29]-sequence of glucagon is HSQGCFTSDYSKYLDSRRAQDFVSWLMNC (table 1). This sequence allows the epilancin K7 enzymes to form thioether rings between S2-C5 and S24-C29.

### Materials and methods

Clone a construct leader-epilancin K7 followed in an open reading frame by mutant glucagon, followed by epilancin BTC. Transform the above plasmid to *Staphylococcus epidermis.* Induce transcription. Continue overnight cell growth in minimal medium, centrifuge, perform ziptipping of the supernatant and MALDI-TOFMS analysis (linear mode).

### Result

A MALDI-TOFMS peak consistent with production of glucagon with dehydrated serines and threonines and thioether rings as indicated in table 1. Maldi TOF analysis of peroxydated samples indicate thioether bridge formation, since to a cysteines 3 oxygens can be added whereas to thioether bridges one or two oxygens are be added.

### EXAMPLE 8

### Production by Streptococcus salivarius (prokaryote Gram positive) of [S3, S12] tachyplesin I (table 1) following export via SalT.

### Materials and methods

Clone a construct sahvaricin-leader (table 2) followed in an open reading frame by mutant tachyplesin (table 1). Clone *salivaricinT* on a second plasmid with different antibiotic marker. Transform both plasmids to a *Streptococcus salivarius* strain devoid of salivaricin genes. Induce transcription of both plasmids, during 2-4 hours of continued growth. Add every 30 min 0.2 mM pmsf (protease inhibitor). Perform ziptipping as in example 1 and analyse by MALDI-TOFMS (linear mode).

### Result

A mass spectrometry peak corresponding to tachyplesin.

### EXAMPLE 9

### Production by Streptococcus salivarius (prokaryote Gram positive) of [S3, S12] tachyplesin I (table1) with salivaricinB-dehydrated serine-3 and serine-12 without subsequent enzymatic thioether ring formation.

Tachyplesin has the following [S3,S12]-sequence: KWSFRVCYRGISYRRCR

### Materials and methods

Clone a construct salivaricin leader (table 2) followed in an open reading frame by mutant tachyplesin (table 1). Clone *salivaricinBT* on a second plasmid with different antibiotic marker. Transform this plasmid to a *Streptococcus salivarius* strain devoid of salivaricin genes. Induce transcription of both plasmids, during 2-4 hours of continued growth. Perform ziptipping as in example 1 and analyse by MALDI-TOFMS (linear mode).

### Result

A mass spectrometry peak corresponding to tachyplesin with dehydrated serines.

### EXAMPLE 10

### Production by Streptococcus salivarius (prokaryote Gram positive) [S3, S12] tachyplesin I (table1) with salivaricinB-dehydrated S3 and S12 without subsequent enzymatic thioether ring formation.

Tachyplesin has the following [S3, S12]-sequence: KWSFRVCYRGISYRRCR

### Materials and methods

Clone a construct salivaricin-leader (table 2) followed in an open reading frame by mutant tachyplesin (table 1) and thereafter *salivaricinBT.* Transform this plasmid to a *Streptococcus salivarius* strain devoid of salivaricin genes. Induce transcription, during 2-4 hours of continued growth. Perform ziptipping as in example 1 and analyse by MALDI-TOFMS (linear mode).

### Result

A mass spectrometry peak corresponding to tachyplesin with dehydrated serines.

### EXAMPLE 11

### Production by Lactococcus lactis (prokaryote, Gram-positive) via lacticinT of vasonatrin (table 1) without modifications.

Lacticin 481-T has leader peptidase activity and therefore in this particular example in the supernatant of the cell culture vasonatrin is found without leader. Vasonatrin is amongst others involved in vaso relaxation. Its sequence is: GLSKGCFGLKLDRIGSMSGLGCNSFRY.

### Materials and methods

Clone a construct lacticin 481-leader (table 2) followed in an open reading frame by vasonatrin (table 1). Clone *lacticin 481-T* on a second plasmid with different antibiotic marker. Transform both plasmids to a *L. Lactis* strain devoid of lacticin 481 genes. Induce transcription of both plasmids during overnight growth in minimal medium. Perform ziptipping as in example 1 and analyse by MALDI-TOFMS (linear mode).

### Result

A mass spectrometry peak corresponding to vasonatrin.

### EXAMPLE 12

### Production by Lactococcus lactis (prokaryote, Gram-positive) via lacticinT of vasonatrin (table 1), with lacticin M mediated thioether rings

Vasonatrin is amongst others involved in vaso relaxation. It has an amino acid sequence, that without mutations permits the formation of two lanthionine rings. Its sequence is: GLSKGCFGLKLDRIGSMSGLGCNSFRY.

Lanthionine rings can be formed from S3-C6 and from S16-C22.

### Materials and methods

Clone a construct lacticin 481-leader (table 2) followed by vasonatrin (table 1). and lacticinM coding equences. Transform the plasmid to *Lactococcus lactis* PA1001. Induce transcription of the plasmid, during overnight growth in minimal medium. Perform ziptipping as in example 1 and analyse by MALDI-TOFMS (reflectron mode). Analyse peroxydized (in the case of a thioether bridge one or two oxygens add; in the case of cysteines three oxygens are added) and non-peroxydized samples.

### Result

Mass spectrometry peaks consistent with lacticinM leader coupled to vasonatrin with two thioether rings and two more dehydrated serines.

### EXAMPLE 13

### Transport via NisT of nisin prepeptide C-terminally fused to an enkephalin variant.

This example shows that unmodifed nisin prepeptide with a C-terminal extension can be exported via NisT. It has been described by others that mature lantibiotics can be exported via LanBTC / TM with a C-terminal extension, but it is not yet known that also unmodified prepeptide with a C-terminal extension can be exported via only the transporter LanT. This example involves a *Lactococcus lactis* strain that lacks the entire chromosomal nisin gene cluster, but produces simultaneously plasmid encoded NisT and a fusion of NisA prepeptide and an enkephalin variant, YTGFC, (the enkephalin genetically fused to the C-terminus of nisin prepeptide). Unmodified fusion prepeptide can be found in the culture supernatant, which demonstrates that NisT is sufficient for the transport of prepeptide with C-terminal extension to the exterior of the cell.

### Materials amd methods.

*Lactococcus lactis,* PA1001 (see example 1), was transformed with pNGnisT, which was constructed as described in example 1. pNGnisA-enkT and pNGnisA-enkS were constructed as follows. pNGnisA was PCRed with the primer couple 5'-GCACGTGTTGCTTTGATTGATAGC-3' and 5'-CTGGATCCTTAACAAAAACCTGTGTATTTGCTTACGTGAATACTAC-'3 (*Bam*H1 site underlined), which leads to C-terminal fusion of the enkephalin variant YTGFC to nisin A (enkT). The PCR product was ligated and transformed to *Lactococcus lactic* PA1001 + pNGnisT. De resulting strain, PA1001 + pNGnisT + pNZenkT was grown in MG17 medium to OD600 = 0.4, pelleted and resuspended in minimal medium supplemented with 1/1000 filtered supernatant of the wild type nisin producer *Lactococcus lactis* NZ9700. After overnight incubation cells were pelleted and the supernatant was ziptipped and subjected to maldi TOF analysis.

### Result:

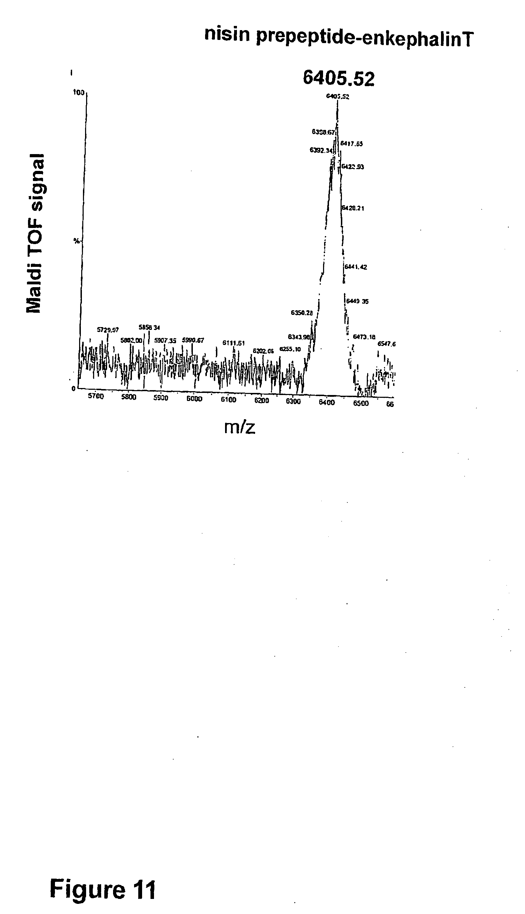

Figure 11. Peaks close to 6400.05 (nisine-prepeptide without methionine1 C-terminally extended with YTGFC) were observed by maldi TOF. Hence the nisin prepeptide genetically fused to an enkephalin variant can be exported via the nisin transporter. Therefore it can be concluded that lantibiotic transporters can also be used for the transport of unmodifed lantibiotic prepeptides that are C-terminally extended by a fused peptide.

### Table 1, selected (poly)peptides

Table 1: (poly)peptides of which the coding DNA is preceded by lantibiotic leader coding DNA in an open reading frame.

Mutation possibilities allowing posttranslational thioether ring(s) formation given for example for vasopressin applies also to other sequences, including those which have already one ring, within other (poly)peptides in Table 1, taking into account the description of thioether ring formation mentioned in the text.

Table 1: (poly)peptides of which the coding DNA is preceded by lantibiotic leader coding DNA in an open reading frame. Mutation possibilities allowing posttranslational thioether ring(s) formation given, for example, for vasopressin applies also to other sequences, including those which have already one ring, within other (poly)peptides in Table 1, taking into account the description of thioether ring formation mentioned in the text.

**Table 1A:**

| Vasopressin: Function : as an antidiuretic hormone: |
|---|
| (A1, S2, R8)-sequence: ASFQNCPRG; lanthionine ring S2-C6 |
| (A1, S2, C3, R8)-sequence: ASCQNCPRG; lanthionine ring S2-C3 |
| (A1, S2, C4, R8)-sequence: ASFCNCPRG ; lanthionine ring S2-C4 |
| (A1, S2, C5, R8)-sequence: ASFQCCPRG (SEQ ID NO:); lanthionine ring S2-C5 |
| (A1, S2, A6, C7, R8)-sequence: ASFQNACRG; lanthionine ring S2-C7 |
| (A1, S2, A6, C8)-sequence: ASFQNAPCG; lanthionine ring S2-C8 |
| (A1, S2, A6, R8, C9)-sequence: ASFQNAPRC; lanthionine ring S2-C9 |
| (A1, S3, R8)-sequence: AYSQNCPRG; lanthionine ring S3-C6 |
| (A1, S3, C4, R8)-sequence: AYSCNCPRG ; lanthionine ring S3-C4 |
| (A1, S3, C5, R8)-sequence: AYSQCCPRG; lanthionine ring S3-C5 |
| (A1, S3, A6, C7, R8)-sequence: AYSQNACRG; lanthionine ring S3-C7 |
| (A1, S3, A6, C8)-sequence: AYSQNAPCG; lanthionine ring S3-C8 |
| (A1, S3, A6, R8, C9)-sequence: AYSQNAPRC; lanthionine ring S3-C9 |
| (A1, S4, R8)-sequence: AYFSNCPRG ; lanthionine ring S4-C6 |
| (A1, S4, C5, R8)-sequence: AYFSCCPRG ; lanthionine ring S4-C5 |
| (A1, S4, A6, C7, R8)-sequence: AYFSNACRG ; lanthionine ring S4-C7 |
| (A1, S4, A6, C8)-sequence: AYFSNAPCG ; lanthionine ring S4-C8 |
| (A1, S4, A6, R8, C9)-sequence: AYFSNAPRC ; lanthionine ring S4-C9 |
| (A1, S5, R8)-sequence: AYFQSCPRG ; lanthionine ring S5-C6 |
| (A1, S5, A6, C7, R8)-sequence: AYFQSACRG ; lanthionine ring S5-C7 |
| (A1, S5, A6, C8)-sequence: AYFQSAPCG ; lanthionine ring S5-C8 |
| (A1, S5, A6, R8, C9)-sequence: AYFQSAPRC ; lanthionine ring S5-C9 |
| (A1, S6, C7, R8)-sequence: AYFQNSCRG ; lanthionine ring S6-C7 |
| (A1, S6, C8)-sequence: AYFQNSPCG ; lanthionine ring S6-C8 |
| (A1, S6, R8, C9)-sequence: AYFQNSPCG ; lanthionine ring S6-C9 |
| (A1, S7, C8)-sequence: AYFQNCSCG ; lanthionine ring S7-C8 |
| (A1, S7, R8, C9)-sequence: AYFQNCSRC ; lanthionine ring S7-C9 |

| Vasopressin: Function : as an antidiuretic hormone |
|---|
| (A1, S7, C9)-sequence: AYFQNCPSC ; lanthionine ring S8-C9. |

### Terlipressin (antidiuretic hormone):

S4-Sequence: GGGSYFQNCPKG
Posttranslational lanthionine: S4-C9.

### Cispressin (antidiuretic hormone):

S4-Sequence: GGGSYFNCPKG
Posttranslational lanthionine ring: S4-C8.

### Adrenomedullin Hypotensive peptide, may function as a hormone in circulation control

A13,S16-Sequence:
   YRQSMNNFQGLRAFGSRFGTCTVQKLAHQIYQFTDKDKDN VAPRSKISPQGY
Posttranslationallanthionine: S16-C21.

### Allatostatin I (neuropeptide inhibitor of juvenile hormone synthesis)

C6-Sequence: APSGACRLYGFGL
Posttranslational lanthionine: S3-C6.

### Angiotensin I

S7,C10-Sequence: DRVYIHSFHC
Posttranslational lanthionine S7-C10
Function: In response to lowered pressure, the enzyme renin cleaves angiotensin I, from angiotensinogen, then removes a dipeptide to yield the physiologically active angiotensin II, the most potent pressor substance known, which helps regulate volume and mineral balance of body fluids.

### Anthopleurin-A (neuropeptide)

A4-Sequence:
   GVSALCDSDGPSVRGNTLSGTLTLYPSGCPSGWHNCKAHGPTI G WCCKQ
Posttranslational lanthionne rings: S3-C6, S27-C31, S33-C38, T44-C48.

### Anti-inflammatory peptide 1 (anti-inflammation)

S1,C6-Sequence: SQMKKCLDS
Posttranslational lanthionine: S1-C6

### Demnaseptin (antimicrobial peptide)

C10-Sequence:
   ALWKTMLKKCGTMALHAGKAALGAAADTISQGTQ
Posttranslational lanthionine: T5-C10.

### Bombinin-like peptide (antimicrobial peptide)

C8-Sequence: GIGASILCAGKSALKGLAKGLAEHFAN
Posttranslationallanthionine: S5-C8.

### Histatin-5 (antimicrobial salivary peptide)

S4,C7-Sequence: DSHSKRCHGYKRKFHDKHHSHRGY
Posttranslationallanthionine: S4-C7.

### Indolicidin (antimicrobial peptide)

S2,C5-Sequence: ISPWCWPWWPWRR
Posttranslationallanthionine: S2-C5.

### Magainin-1 (antimicrobial peptide)

C13-Sequence: GIGKFLHSAGKFGKAFVGEIMKS
Posttranslationallanthionine: S8-C13.

Atrial Natriuretic Factor (potent vasoactive substance with a key role in cardiovascular homeostasis and cGMP-stimulating activity).
Sequence: SLRRSSCFGGRMDRIGAQSGLGCNSFRY
Posttranslational lanthionines: S1-C7, S19-C23.

### Bradykinin (important role in renal physiology and behavior).

C9-Sequence: RPPGFSPFC
Posttranslationallanthionine: S6-C9.

Brain Natriuretic Peptide (acts as a cardiac hormone involved in natriuresis, diuresis, vasorelaxation, inhibition of renin and aldosteron secretion, improves heart function)
S16,C19-Sequence: SPKMVQGSGCFGRKMSRICSSSGLGCKVLRRH
Posttranslational lanthionine: S8-C10, S16-C19

### C-type Natriuretic peptide (exhibits natriuretic and vasodepressor activity)

Sequence:
   DLRVDTKSRAAWARLLQEHPNARKYKGANKKGLSKGCFGLK LDRIG SMSGLGC
Posttranslational lanthionine ring: S34-C37, S47-C53.

### Vasonatrin peptide (vasorelaxation)

Sequence: GLSKGCFGLKLDRIGSMSGLGCNSFRY
Posttranslational lanthionine ring: S3-C6,S17-C22.

### Delta sleep inducing peptide (delta sleep induction)

S2,C6-Sequence: WSGGNCSGE
Posttranslational lanthionine ring: S2-C6.

### Alpha-dendrotoxin

S11,S26-Sequence:
   PRRKLCILHRSPGRCYDKIPAFYYNSKKKQCERFDWSGC GGNSNRFKTIEECRRTCIG
Posttranslational lanthionine: S11-C15, S26-C31
Function: affects potassium channels.

### Eledoisin

C4-Sequence: PSKCAFIGLM Posttranslational lanthionine ring: S2-C4
Function: neuron excitation, causing behavioral responses, vasodilators, secretagogues, causing contraction of smooth muscles.

### Echistatin

Sequence:
   ECESGPCCRNCKFLKEGTICKRARGDDMDDYCNGKTCDCPRNPHK GPAT
Posttranslational lanthionine rings: S4-C7, T18-C20, T36-C37
Function: Inhibitor of fibrinogen-dependent platelet aggregation.

### alpha-endorphin

S2,C6-Sequence: YSGFMCSEKSQTPLVT
Posttranslational lanthionine ring: S2-C6
Function: opioid.

### beta-endorphin

S21 ,C26-Sequence: YGGFMTSEKSQTPLVTLFKNSILKNCYKKGE
Posttranslational lanthionine ring: S21-C26
Function: opioid.

### Defensin I

S2,S 12-Sequence: ASYCRIPACIAGSRRYGTCTYQGRLWAFCC
Posttranslational lanthionine rings: S2-C4, S13-C19
Function: antimicrobial peptide.

### Secretin

S23,C26-Sequence: HSDGTFTSELSRLREFARLQRLSQGCV
Posttranslational lanthionine ring:S23-C26
Function: pH regulation in the stomach.

### Urocortin

C19-Sequence: DNPSLSIDLTFHLLRTLLCLARTQSQRERAEQNRIIFDSV
Posttranslational lanthionine ring: T16-C19
Function: stimulates ACTH secretion.

### Urotensin II

S5-Sequence: AGTASCFWKYCV
Posttranslational lanthionine rings: T3-C6, S5-C11
Function: osmoregulation and corticotropinrelease factor.

### Small Cardioactive Peptide A

S4,C7-Sequence: ARPSYLCFPRM
Posttranslational lanthionine:S4-C7
Function: inhibits acetylcholine release

### Small Cardioactive peptide B

S4,C7-Sequence: MNYSAFCRM
Posttranslational lanthionine: S4-C7
Function: stimulates contraction in the gut, increases amplitude of the heart beat.

### Ceratotoxin A

C9-Sequence: SIGSALKKCLPVAKKIGKIALPIAKAALP Posttranslational lanthionine: S4-C9
Function: antimicrobial, hemolytic peptide with activity against Gram-positive and Gram-negative bacteria, stable at 100 degrees Celsius.

### Cerebellin

C7-Sequence: SGSAKVCFSAIRSTNH
Posttranslational lanthionine: S3-C7
Function: neuromudulation, stimulation of norepinephrine release, enhances indirectly adrenocortical secretion.

### Charybdotoxin

S33-Sequence: FTNVSCTTSKECWSVCQRLHNTSRGKCMNKKSRCYS
Posttranslational (methyl)lanthionine: T3-C7, T8-C13, S15-C17, T23-C28, S33-C35
Function: inhibitor calcium - and voltage activated potassium channels.

### Cholecystokinin

C8-Sequence: KAPSGRMCIVKNQQLDPSHRISDRYMGWMDF
Posttranslational lanthionine: S4-C8
Function: Gall bladder contraction and release of pancreatic enzymes in the gut.

### Conopressin G

S1-Sequence: SFIRNCPKG
Posttranslationallanthionine: S1-C6
Function: behavioral control.

### alpha-Conotoxin EI

S2,S5-Sequence: RSHCSYHPTCNMSNPQIC
Posttranslational lanthionine: S2-C4, S5-C10, S13-C18
Function: blocking nicotinic acetylcholine receptors.

### Corazonin

C9-Sequence: TFQYSRGWCN
Posttranslational lanthionine: S5-C9
Function: Regulation heart beat.

### Leu-enkephalin

S2,C3-Sequence: YSCFL
Posttranslational lanthionine ring: S2-C3
Function: opioid

### Met-enkephalin

S2,C3-Sequence: YSCFM Posttranslational lanthionine ring: S2-C3
Function: opioid.

### Oxytocin

(S1)-Sequence: SYIQNCPLG Posttranslational lanthionine ring S1-C6
Function: Oxytocin stimulates uterine contraction and lactation; increases Na⁺ secretion; stimulates myometrial GTPase and phospholipase C.

### Exendin-3

C35-Sequence:
   HSDGTFTSDLSKQMEEEAVRLFIEWLKNGGPSSGCPPPS
Posttranslational lanthionne ring: S32-C35
Function: secretin-like.

### Experimental Allergic Encephalitogenic peptide

C5-Sequence: FSWGCEGQR Posttranslational lanthionine ring: S2-C5
Function: myelin membrane stabilization.

### Experimental Autoimmune Encephalomyelitis Complementary peptide

S4,C7-Sequence: VFISGPCRLLG Posttranslational lanthionine ring: S4-C7
Effect: having a role in autoimmune encephalomyelitis.

### GonadoliberinII

(C9)-sequence: QHWSHGWYCG Posttranslational lanthionine ring: S4-C9
Function: stimulates the secretion of gonadotropins; it stimulates the secretion of both luteinizing and follicle stimulating hormones.

### Tocinoic acid / pressinoic acid

(S1,I3)-Sequence: SYIQNC posttranslational lanthionine ring S1-C6
Function: Tocinoic acid is an oxytocin inhibitor, induces maternal behavior.

### Leuprolide

Sequences:
   XHWSYGCRPX
Posttranslational thioether ring: S4-C7
XHWSYXCRX
Posttranslational thioether ring: S4-C7
Function: LHRH agonist.

### Calcitonin

Accession number: P01258
(S1)-Sequence: SGNLSTCMLGTYTQDFNKFHTFPQTAIGVGAP
Posttranslational thioether ring S1-C7
Function: CaPi incorporation in bones.

### ACTH, Adrenocorticotropic hormone

(Q5,C6)-Sequence:
   SYSMQCFRWGKPVGKKRRPVKVYPNGAEDESAEAFPLEF
posttranslational lanthionine ring S1-C6

### ACTH-fragment-sequence:

SYSMECFRWG
Posttranslational ring: S2-C6
Function: ACTH stimulates synthesis and secretion of glucocorticoids by adrenal cortex.

### Hepatitis B surface antigen fragment

C6-Sequence: MGTNLCVPNPLGFFPDHQLDP
Posttranslational modification: T3-C6 lanthionine ring
Function: surface antigen

### Conicotropin inhibiting peptide

S4,C8-Sequence: FRWSKPVCKKRRPVKVYPNGAEDSAEAFPLE
Posttranslational lanthionine: S4-C8
Function: inhibition ACTH.

### Corticotropin-Release Factor

S30,C33-Seq:
   SEEPPISLDLTFHLLREVLEMARAEQLAQSAHCNRKLMEII
Posttranslational lanthionine: S30-C33
Function: release of corticotrophin.

### Somatostatin

(S3)-Sequence:AGSKNFFWKTFTSC
posttranslational lanthionne ring S3-C14
Function:somatotropin release inhibition factor, growth hormone release inhibiting factor.

### Human pancreatic polypeptide

(S18, C21)-Sequence: APLEPVYPGDNATPEQMSQYCADLRRUINMLTRPRY,
Posttranslational lanthionine ring S18-C21
Function: Agonist at Y4 neuropeptide receptors.

### Peptide YY

(S22,C25,T29,C32)-Sequence:
   YPIKPEAPGEDASPEELNRYYASLRHYLNLVTRQRY
Posttranslational (methyl)lanthionine rings S22-C25, T29-C32
Function: Gut hormone that inhibits both secretin- and cholecystokinin-stimulated pancreatic secretion.

### Glucagon

(C5,S24,C29)-Sequence: HSQGCFTSDYSKYLDSRRAQDFVSWLMNC
Posttranslational lanthionine rings S1-C5, S24-C29
Function: restoring blood glucose level when too low.

### alpha-neurokinin

(C9)-sequence: HKTDSFVGCM
posttranslational lanthionine ring S5-C9
function: tachykinin antagonist.

### LHRH1, Luteinizing Hormone Releasing Hormone

Function: regulates secretion of gonadotropins, luteinizing hormone and sex steroids.
(Q1, C7)-Sequence: QHWSYGCRPG
Posttranslational lanthionine ring S4-C7

| | | |
|---|---|---|
| (S1, C4)-Sequence: | SHWCYGLRPG | posttr. ring: S1-C4 |
| (S1, A4, C5)-Sequence: | SHWACGLRPG | posttr. ring: S1-C5 |
| (S1, A4, C6)-Sequence: | SHWAYCLRPG | posttr. ring: S1-C6 |
| (Q1, S2, A4, C5)-Sequence: | QSWACGLRPG | posttr. ring: S2-C5 |
| (Q1, S2, A4, C6)-sequence: | QSWAYCLRPG | posttr. ring: S2-C6 |
| (Q1, S2, A4, C7)-sequence: | QSWAYGCRPG | posttr. ring: S2-C7 |
| (Q1, S3, A4, C6)-sequence: | QHSAYCLRPG | posttr. ring: S3-C6 |
| (Q1, S3, A4, C7)-sequence: | QHSAYGCRPG | posttr. ring: S3-C7 |
| (Q1, S3, A4, C8)-sequence: | QHSAYGLCPG | posttr. ring: S3-C8 |
| (Q1, C8)-sequence: | QHWSYGLCPG | posttr. ring: S4-C8 |
| (Q1, C9)-sequence: | QHWSYGLRCG | posttr. ring: S4-C9 |
| (Q1, A4, S5, C8)-sequence: | QHWASGLCPG | posttr. ring: S5-C8 |
| (Q1, A4, S5, C9)-sequence: | QHWASGLRCG | posttr. ring: S5-C9 |
| (Q1, A4, S5, C10)-sequence: | QHWASGLRPC | posttr. ring: S5-C10 |
| (Q1, A4, S6, C9)-sequence: | QHWAYSLRCG | posttr. ring: S6-C9 |
| (Q1, A4, S6, C10)-sequence: | QHWAYSLRPC | posttr. ring: S6-C10 |
| (Q1, A4, S7, C10)-sequence: | QHWAYGSRPC | posttr. ring: S7-C10. |

### LHRH2, Luteinizing Hormone Releasing Hormone fragment

Function: regulates secretion of gonadotropins, luteinizing hormone and sex steroids.
(Q1, C7)-Sequence: QHWSHGCYPG
Posttranslational lanthionne ring S4-C7

| | | |
|---|---|---|
| (S1, C4)-Sequence: | SHWCHGWYPG | posttr. ring: S1-C4 |
| (S1, A4, C5)-Sequence: | SHWACGWYPG | posttr. ring: S1-C5 |
| (S1, A4, C6)-Sequence: | SHWAHCWYPG | posttr. ring: S1-C6 |
| (Q1, S2, A4, C5)-Sequence: | QSWACGWYPG | posttr. ring: S2-C5 |
| (Q1, S2, A4, C6)-sequence: | QSWAHCWYPG | posttr. ring: S2-C6 |
| (Q1, S2, A4, C7)-sequence: | QSWAHGCYPG | posttr. ring: S2-C7 |
| (Q1, S3, A4, C6)-sequence: | QHSAHCWYPG | posttr. ring: S3-C6 |
| (Q1, S3, A4, C7)-sequence: | QHSAHGCYPG | posttr. ring: S3-C7 |
| (Q1, S3, A4, C8)-sequence: | QHSAHGWCPG | posttr. ring: S3-C8 |
| (Q1, C8)-sequence: | QHWSHGWCPG | posttr. ring: S4-C8 |
| (Q1, C9)-sequence: | QHWSHGWYCG | posttr. ring: S4-C9 |
| (Q1, A4, S5, C8)-sequence: | QHWASGWCPG | posttr. ring: S5-C8 |
| (Q1, A4, S5, C9)-sequence: | QHWASGWYCG | posttr. ring: S5-C9 |
| (Q1, A4, S5, C10)-sequence: | QHWASGWYPC | posttr. ring: S5-C10 |
| (Q1, A4, S6, C9)-sequence: | QHWAHSWYCG | posttr. ring: S6-C9 |
| (Q1, A4, S6, C10)-sequence: | QHWAHSWYPC | posttr. ring: S6-C10 |
| (Q1, A4, S7, C10)-sequence: | QHWAHGSYPC | posttr. ring: S7-C10. |

### Brain derived acidic fibroblast growth factor (102-111)

(S103,C109)-Sequence: HSQKHWFCGL
Posttranslational lanthionine ring S103-C109
Function:growth factor.

### Brain derived basic fibroblast growth factor (1-24)

Sequence:PALPEDGGSGAFPPCHFKDPKRLY
Posttranslational lanthionine ring S11-C17
Function: growth factor.

### Insulin

Sequences:
   alpha-chain: GIVEQCCASVCSLYQLENYCN (SEQ ID NO:)
(S9-C14, T27-C30)-beta chain: FVNQHLCGSHLVECLYLVCGERGFFYTPKC (SEQ ID NO:)
Posttranslational (methyl)lanthionine rings S9-C14, T27-C30
disulfide bonds: alpha 6 - 11 alpha 7 - beta 7, alpha 20 - beta 19
function: diabetes treatment.

### Parathormone:

(S36-C39, T79-C82)-Sequence:
   SVSEIELMHNLGKHLNSMERVEWLRKKLQDVHNFVSLGCPLAPRDAG SERPRKKEDNVLVESHEKSLGEADKADVNVLTKACSE (SEQ ID NO:)
Posttranslational (methyl)lanthionine rings S36-C39, T79-C82
Function: modulation of serum calcium content affecting the mineral and bone physiology.

### Fibrinogen Binding Inhibitor peptide

S6,C9-Sequence: HHLGGSKQCGDV
Posttranslational lanthionine: S6-C9.

### Fibroblast growth factor inhibitory peptide

S1,C3-Sequence: SPCGHYKG
Posttranslational lanthionine ring: S1-C3
Effect: inhibition fibroblast growth factor.

### Galanin

C10-Sequence: GWTLNSAGYCLGPHAVGNHRSFSDKNGLTS
Posttranslational lanthionine ring: S6-C10
Function: contracts smooth muscle of the gastrointestinal and genitourinary tract, regulates growth hormone release, modulates insulin release.

### Gastric Inhibitory Polypeptide

S28,C31-Sequence: YAEGTFISDYSIAMDKIHQQDFVNWLLSQKCKKNDWKHNITQ
Function: potent stimulation of insulin secretion and relatively poor inhibitor of gastric acid secretion.

### Big Gastrin-I

S8,C11-Sequence: LGPQGPPSLVCDPSKKQGPWLEEEEEAYGWMDF
Posttranslational lanthionine ring: S8-C11
Function: stimulates gastric HCl secretion, pancreatic enzyme secretion, smooth muscle contraction and increases blood circulation and water secretion in the stomach and intestine.

### Pentagastrin

S1,C4-Sequence: SWMCF
Posttranslational lanthionine ring: S1-C4
Gastrin Releasing Peptide
S9,C12-Sequence: VPLPAGGGSVLCKMYPRGNHWAVGHLM
Posttranslational lanthionine ring: S9-C12
Function: gastrin release.

### Transforming growth factor alpha

Sequence:
   VVSHFNDCPDSHTQFCFHGTCRFLVQEDKPACVCHSGYVGAR CEHA DLLA
Posttranslational (methyl)lanthionine ring: S3-C8, T21-C22, S37-C44
Function: TGF alpha is a mitogenic polypeptide that is able to bind to the egf receptor and act synergistically with TGF beta to promote anchorage-independent cell proliferation in soft agar.

### Human growth hormone

C7-Sequence:
   FPTIPLCRLFDNAMLRAHRLHQLAFDTYQEFEEAYIPKEQKYS
Posttranslational methyllanthionine ring: T3-C7
Function: growth hormone, stimulates amongst others protein synthesis and amino acid uptake.

### Growth hormone release factor

C22-Sequence:
   YADAIFTNSYRKVLGQLSARKCLQDIMSRQQGESNQERGARAR L
Posttranslational lanthionine ring: S18-C22
Function: release of growth hormone.

### Guanylin

Sequence: PGTCEICAYAACTGC
Posttranslational lanthionine ring: T3-C4, T13-C15
Function: activator of guanylate cyclase.

### Helospectin I

S15,C18-Sequence:
   HSDATFTAEYSKLLSKLCLQKYLESILGSSTSPRPPSS
Posttranslational lanthionine ring: S15-C18
Hepatitis B surface antigen fragment
C6-Sequence: MGTNLCVPNPLGFFPDHQLDP
Posttranslational methyllanthionine ring: T3-C6
Function: exendin-1: secretin-like.

### Intercellular adhesion molecule

Sequence: NAQTSVSPSKVILPRGGSVLVTC
Posttranslational lanthionine ring: S 18-C23
Function: anti-hiv.

### Tachyplesin I

(S3,S12)-Sequence: KWSFRVCYRGISYRRCR
Posttranslational lanthionine rings S3-C7, S12-C16
Function Hiv cell fusion inhibitor, anti tumor peptide, antimicrobial peptide.

### HIV (gp 120) antigenic peptide fragment

(S10,C14)-Sequence: CGKIEPLGVSPTKCKRRVVQREKR
Posttranslational lanthionine ring S10-C14.

### HIV (gp 41) antigenic peptide I fragment

(S2)-Sequence: GSSGKLICTTAVPWNAS
Posttranslational lanthionine S2-C8.

### HIV (gp41) antigenic peptide 5

(S20)-Sequence:RVTAIEKYLQDQARLNSWGSAFRQVCHTTVPWVNDS
Posttranslational lanthionine ring S20-C26.

### HIV protease inhibitors

Sequence: TVSFCF
Posttranslational lanthionine ring T1-C5
Function: inhibitor HIV protease.

### Insulin-like growth factor-I analog

S1,C4-Sequence: SYACPLKPAKSC
Posttranslational lanthionine rings: S1-C4, S11-C12.

### IGF II 69-84:

(C7)-Sequence: DVSTPPCVLPDNFPRY (SEQ ID NO:)
Posttranslational lanthionine ring S3-C7.

### Interleukin-8 fragment:

(S6, C10)-Sequence: AVLPRSAKEC (SEQ ID NO:)
Posttranslational lanthionine ring S6-C10
Function: attraction neutrophils, basophils and T-cells, but not monocytes. It is involved in neutrophil activation and is released from several cell-types in response to inflammation.

### Interleukin-2 fragment(60-70) (T-cell growth factor)

Sequence: LTFKFYMSKKC (SEQ ID NO:)
Posttranslational lanthionine ring S67-C70.

### Leucokinin I (neuroactive peptide)

C8-Sequence: DPAFNSWC (SEQ ID NO:)
Posttranslational lanthionine ring: S6-C8.

### Leukopyrokinin

C4-Sequence: TSFCPRL (SEQ ID NO:)
Posttranslational lanthionine ring:T1-C4
Function: mediates visceral muscle contractile activity.

### Mastoparan

S5,C8-Sequence: INLKSLACLAKKIL (SEQ ID NO:)
Posttranslational lanthionine ring: S5-C8
Function: Wasp venom membrane-active toxin.

### Melanin concentrating hormone

S11-Sequence: DFDMLRCMLGSVYRPCWQV (SEQ ID NO:)
Posttranslational lanthionine ring: S11-C16
Function: possible neurotransmitter, involved in the regulation of goal directed behavior.

### Melittin

C14-Sequence: GIGAVLKVLTGLPCLISWIKRKRQQ (SEQ ID NO:)
Posttranslational lanthionine ring: T10-C14
Function: Bee venom membrane-active peptide.

### Motilin

C9-Sequence: FVPIFTYGCLQRMQEKERNKGQ (SEQ ID NO:)
Posttranslational lanthionne ring: T6-C9
Function: regulation of interdigestive gastrointestinal motility.

### Neuropeptide Y

C26-Sequence: YPSKPDNPGEDAPAEDMARYYSALRCYINLITRNRY (SEQ ID NO:)
Posttranslational lanthionine ring S22-C26
Function:control of feeding and secretion of gonadotropin-release hormone.

### Osteocalcin

S4,C8-Sequence: YLYSWLGCPVPYPDPDELADHIGFQEAYRRFYGPV (SEQ ID NO:)
Posttranslational lanthionine ring: S4-C8
Function: constitutes 1-2% of the total bone protein, it binds strongly to apatite and calcium.

### (N-acetyl-)beta-endorphin 1-27

(C21)-Sequence: YGGFMTSEKSQTPLVTLFKNCIIKNAY (SEQ ID NO:)
Posttranslational methyllanthionine T16-C21
Functions: analgesia, behavioral changes, growth hormone release.

### Ras oncogene related peptide

HU-ras^{ha}
(S2, C5)-Sequence: GSGGCGKS (SEQ ID NO:)
Posttranslational lanthionne ring S2-C5.

### Ras oncogene related peptide

Hu-ras^{T24}
(S2, C5)-Sequence: GSVGCGKS (SEQ ID NO:)
Posttranslational lanthionne ring S2-C5.

### Ras oncogene related peptide

Hu-(Hu-ras^{t24})-Lys
(S3, C6)-Sequence: YGSVGCGKSK (SEQ ID NO:)
Posttranslational lanthionine ring S3-C6.

### Table 1 B:

Albumin
Accession number: P02768

Sequence: DTHKSE IAHRFKDLGE EHFKGLVLIA FSQYLQQCPF DEHVKLVNEL TEFAKTCVAD ESHAGCEKSL
HTLFGDELCK VASLRETYGD MADCCEKQEP ERNECFLSHK DDSPDLPKLK PDPNTLCDEF KADEKKFWGK
YLYEIARRHP YFYAPELLYY ANKYNGVFQE CCQAEDKGAC LLPKIETMRE KVLASSARQR LRCASIQKFG ER-
ALKAWSVA RLSQKFPKAE FVEVTKLVTD LTKVHKECCH GDLLECADDR ADLAKYICDN QDTISSKLKE CCDK-
PLLEKS HCIAEVEKDA IPENLPPLTA DFAEDKDVCK NYQEAKDAFL GSFLYEYSRR HPEYAVSVLL RLAKE-
YEATL EECCAKDDPH ACYSTVFDKL KHLVDEPQNL IKQNCDQFEK LGEYGFQNAL IVRYTRKVPQ
VSTPTLVEVS RSLGKVGTRC CTKPESERMP CTEDYLSLIL NRLCVLHEKT PVSEKVTKCC TESLVNRRPC
FSALTPDETY VPKAFDEKLF TFHADICTLP DTEKQIKKQT ALVELLKHKP KATEEQLKTV MENFVAFVDK
CCAADDKEAC FAVEGPKLVV STQTALA

Disulfide bonds: 77-86;99-115;114-125;148-193;192-201;224-270;269-277;289-303;302-313;340-385;384-393;416-462;461-472;485-501;500-511;538-583;582-591 (numbers correspond to the precursor protein which contains 24 amino acids more, N-terminally)
Function: regulation colloidal osmotic pressure of the blood plasma, binding blood plasma molecules.
Alglucerase
Accession number: P04062

Sequence: -
-
-


-


Function: glucosylceramidase.

Alpha -galactosidase
Accession number: P06280

Sequence: LDNGLARTP TMGWLHWERF MCNLDCQEEP DSCISEKLFM EMAELMVSEG WKDAGYEYLC IDD-
CWMAPQR DSEGRLQADP QRFPHGIRQL ANYVHSKGLK LGIYADVGNK TCAGFPGSFG YYDIDAQTFA
DWGVDLLKFD GCYCDSLENL ADGYKHMSLA LNRTGRSIVY SCEWPLYMWP FQKPNYTEIR QYCNHWRNFA
DIDDSWKSIK SILDWTSFNQ ERIVDVAGPG GWNDPDMLVI GNFGLSWNQQ VTQMALWAIM AAPLFMSNDL
RHISPQAKAL LQDKDVIAIN QDPLGKQGYQ LRQGDNFEVW ERPLSGLAWA VAMINRQEIG GPRSYTIAVA SLG-
KGVACNP ACFITQLLPV KRKLGFYEWT SRLRSHINPT GTVLLQLENT MQMSLKDLL

Function: galactosidase.
Alteplase
Accession number: P00750

Sequence: SYQVI CRDEKTQMIY QQHQSWLRPV LRSNRVEYCW CNSGRAQCHS VPVKSCSEPR CFNGGTC-
QQA LYFSDFVCQC PEGFAGKCCE IDTRATCYED QGISYRGTWS TAESGAECTN WNSSALAQKP YSGRRP-
DAIR LGLGNHNYCR NPDRDSKPWC YVFKAGKYSS EFCSTPACSE GNSDCYFGNG SAYRGTHSLT ESGAS-
CLPWN SMILIGKVYT AQNPSAQALG LGKHNYCRNP DGDAKPWCHV LKNRRLTWEY CDVPSCSTCG LR-
QYSQPQFR IKGGLFADIA SHPWQAAIFA KHRRSPGERF LCGGILISSC WILSAAHCFQ ERFPPHHLTV IL-
GRTYRVVP GEEEQKFEVE KYIVHKEFDD DTYDNDIALL QLKSDSSRCA QESSVVRTVC LPPADLQLPD WTE-
CELSGYG KHEALSPFYS ERLKEAHVRL YPSSRCTSQH LLNRTVTDNM LCAGDTRSGG PQANLHDACQ GDS-
GGPLVCL NDGRMTLVGI ISWGLGCGQK DVPGVYTKVT NYLDWIRDNM RP

Disulfide: 41-71; 69-78; 86- 97; 91-108;110-119; 127-208; 148 -190;. 179-203; 215-296;236-278; 267-291; 299-430; 342-358; 350-419; 444-519; 476-492;509-537 (counted with 35 additional N-terminal aa)
Function: cleaves plasminogen to form plasmin

Antithrombin III
Accession number: P01008

Sequence: HGSPVDIC TAKPRDIPMN PMCIYRSPEK KATEDEGSEQ KIPEATNRRV WELSKANSRF ATTFYQH-
LAD SKNDNDNIFL SPLSISTWA MTKLGACNDT LQQLMEVFKF DTISEKTSDQ IHFFFAKLNC RLYRKANKSS
KLVSANRLFG DKSLTFNETY QDISELVYGA KLQPLDFKEN AEQSRAAINK WVSNKTEGRI TDVIPSEAIN ELTV-
LVLVNT IYFKGLWKSK FSPENTRKEL FYKADGESCS ASMMYQEGKF RYRRVAEGTQ VLELPFKGDD ITMV-
LILPKP EKSLAKVEKE LTPEVLQEWL DELEEMMLVV HMPRFRIEDG FSLKEQLQDM GLVDLFSPEK SKLPGI-
VAEG RDDLYVSDAF HKAFLEVNEE GSEAAASTAV VIAGRSLNPN RVTFKANRPF LVFIREVPLN TIIFMGRVAN
PCVK

Disulfide: 40-160;53-127; 279-462 (counted with 32 aa signal sequence)
Function: inhibition coagulation.
Aprotinin
Accession number: P00974
Sequence: RPDFC LEPPYTGPCK ARIIRYFYNA KAGLCQTFVY GGCRAKRNNF KSAEDCMRTC GGA
Disulfide: 40-90; 49-73; 65-86 (counting with 35 aa N-terminal)
Function: Inhibits trypsin, kallikrein, chymotrypsin and plasmin.
Asparaginase
Accession number: P20933
Sequence:

alpha-chain: SPLPLV VNTWPFKNAT EAAWRALASG GSALDAVESG CAMCEREQCD GSVGFGGSPD ELGET-
TLDAM IMDGTTMDVG AVGDLRRIKN AIGVARKVLE HTTHTLLVGE SATTFAQSMG FINEDLSTSA SQALHSD-
WLA RNCQPNYWRN VIPDPSKYCG PYKPPGILKQ DIPIHKETED DRGHD
beta-chain: TIGMV VIHKTGHIAA GTSTNGIKFK IHGRVGDSPI PGAGAYADDT AGAAAATGNG DILMRFLPSY
QAVEYMRRGE DPTIACQKVI SRIQKHFPEF FGAVICANVT GSYGAACNKL STFTQFSFMV YNSEKNQPTE
EKVDCI

Disulfide: 64-69; 163-179; 286-306; 317-345 (counted with 23 extra N-terminal aa)
Function: Cleaving glycoproteins.
Becaplermin
Accession number: P01127

Sequence: SLGSLTIAE PAMIAECKTR TEVFEISRRL IDRTNANFLV WPPCVEVQRC SGCCNNRNVQ CRP-
TQVQLRP VQVRKIEIVR KKPIFKKATV TLEDHLACKC ETVAAARPVT RSPGGSQEQR AKTPQTRVTI RTVRVR-
RPPK GKHRKFKHTH DKTALKETLG

Disulfide: 97-141; 130-178; 134-180;124-124 INTERCHAIN; 133-133 INTERCHAIN. (counting with 81 aa N-terminal)
Function: growth factor from platelet.
Bone morphogenic protein 7
Accession number: P34819

Sequence: GKHNSAPMFM LDLYNAMAVE EGGGPAGQGF SYPYKAVFST QGPPLASLQD SHFLTDADMV MS-
FVNLVEHD KEFFHPRYHH REFRFDLSKI PEGEAVTAAE FRIYKDYIRE RFDNETFRIS VYQVLQEHLG RES-
DLFLLDS RTLWASEEGW LVFDITATSN HWVVNPRHNL GLQLCVETLD GQSINPK

Function: induces bone formation, involved in Ca regulation.
Catalase
Accession number: P04040

Sequence: ADSRDPASDQ MQHWKEQRAA QKADVLTTGA GNPVGDKLNV ITVGPRGPLL VQDVVFTDEM AH-
FDRERIPE RVVHAKGAGA FGYFEVTHDI TKYSKAKVFE HIGKKTPIAV RFSTVAGESG SADTVRDPRG FAVK-
FYTEDG NWDLVGNNTP IFFIRDPILF PSFIHSQKRN PQTHLKDPDM VWDFWSLRPE SLHQVSFLFS DRGIP-
DGHRH MNGYGSHTFK LVNANGEAVY CKFHYKTDQG IKNLSVEDAA RLSQEDPDYG IRDLFNAIAT GKYPSWT-
FYI QVMTFNQAET FPFNPFDLTK VWPHKDYPLI PVGKLVLNRN PVNYFAEVEQ IAFDPSNMPP GIEASPDKML
QGRLFAYPDT HRHRLGPNYL HIPVNCPYRA RVANYQRDGP MCMQDNQGGA PNYYPNSFGA PEQQPSALEH
SIQYSGEVRR FNTANDDNVT QVRAFYVNVL NEEQRKRLCE NL4GHLKDAQ IFIQKKAVKN FTEVHPDYGS
HIQALLDKYN AEKPKNAIHT FVQSGSHLAA REKANL

Function: protection against H₂O₂.
Cecropin B
Accession number: P01508
Sequence: KWKV FKKIEKMGRN IRNGIVKAGP AIAVLGEAKA LG
Function: Antibacterial.
Cellulase
Accession number: P23548

Sequence: MKKKGLKKTF FVIASLVMGF TLYGYTPVSA DAASVKGYYH TQGNKIVDES GKEAAFNGLN WF-
GLETPNYT LHGLWSRSMD DMLDQVKKEG YNLIRLPYSN QLFDSSSRPD SIDYHKNPDL VGLNPIQIMD KLIEK-
AGQRG IQIILDRHRP GSGGQSELWY TSQYPESRWI SDWKMLADRY KNNPTVIGAD LHNEPHGQAS WGT-

GNASTDW RLAAQRAGNA ILSVNPNWLI LVEGVDHNVQ GNNSQYWWGG NLTGVANYPV VLDVPNRVVY SPH-
DYGPGVS SQPWFNDPAF PSNLPAIWDQ TWGYISKQNI APVLVGEFGG RNVDLSCPEG KWQNALVHYI
GANNLYFTYW SLNPNSGDTG GLLLDDWTTW NRPKQDMLGR IMKPVVSVAQ QAEAAAE

Function: hydrolysis cellulose.
Choriogonadotropin alpha
Accession number: P01215

Sequence: APDVQD CPECTLQENP FFSQPGAPIL QCMGCCFSRA YPTPLRSKKT MLVQKNVTSE STCCVAK-
SYN RVTVMGGFKV ENHTACHCST CYYHKS

Function: A heterodimer of a common alpha chain and a unique beta chain confers biological specificity to thyrotropin, lutropin, follitropin and gonadotropin.
Choriogonadotropin beta
Accession number: P01233

Sequence: SKEPLRPRCR PINATLAVEK EGCPVCITVN TTICAGYCPT MTRVLQGVLP ALPQVVCNYR DVRFE-
SIRLP GCPRGVNPVV SYAVALSCQC ALCRRSTTDC GGPKDHPLTC DDPRFQDSSS SKAPPPSLPS
PSRLPGPSDT PILPQ

Disulfide: 29-77; 43-92; 46-130; 54-108; 58-110; 113-120
Function: stimulates steroid production.
Chymopapain
Accession number: P14080

PQSID WRAKGAVTPV KNQGACGSCW AFSTIATVEG INKIVTGNLL ELSEQELVDC DKHSYGCKGG YQTT-
SLQYVA NNGVHTSKVY PYQAKQYKCR ATDKPGPKVK ITGYKRVPSN CETSFLGALA NQPLSVLVEA GGKPFQ-
LYKS GVFDGPCGTK LDHAVTAVGY GTSDGKNYII IKNSWGPNWG EKGYMRLKRQ SGNSQGTCGV YKSSYYP-
FKG FA

Disulfide: 156-197; 190-229; 287-338 (counting with 134 aa N-terminal)
Function: Thiol protease.
Chymotrypsin
Accession number: P54414

MTTSAARKGL RTRGSACPRA TRSASSISSR AQVIVAGPIT DKLAQRTVAH LLALAEDSDE PINMLISSPG GH-
VESGDMIH DVIKFIRPTV RTIGLAWVAS AGALIFVGAD KENRYCLPNT RFLIHQPSVG IGGTSTDMMI
QAEQVRLMRD RLNQIFAEAT GQPVERIEKD TQRDFWLNTQ EALDYGLLGK VIRSVDELK

Function: serine protease.
Big Endothelin
Sequence: CSCSSLMDKECVYFCHLDIIWVNTPEHVVPYGLGSPRS
Function: endothelins are endothelium derived vasoconstrictor peptides.
Clostridium botulinum toxin type A
Accession number: Q45894

Sequence A-light chain: PFVNKQFNYK DPVNGVDIAY IKIPNAGQMQ PVKAFKIHNK IWVIPERDTF TNPEEGDL-
NP PPEAKQVPVS YYDSTYLSTD NEKDNYLKGV TKLFERIYST DLGRMLLTSI VRGIPFWGGS TIDTELKVID TNC-
INVIQPD GSYRSEELNL VIIGPSADII QFECKSFGHD VLNLTRNGYG STQYIRFSPD FTFGFEESLE VDTNPLL-
GAG KFATDPAVTL AHELIHAEHR LYGIAINPNR VFKVNTNAYY EMSGLEVSFE ELRTFGGHDA KFIDSLQENE
FRLYYYNKFK DVASTLNKAK SIIGTTASLQ YMKNVFKEKY LLSEDTSGKF SVDKLKFDKL YKMLTEI YTE DN-
FVNFFKVI NRKTYLNFDK AVFRINIVPD ENYTIKDGFN LKGANLSTNF NGQNTEINSR NFTRLKNFTG LFEFYKLL-
CV RGIIPFKTKS LDEGYNK

Sequence A-heavy chain: ALN DLCIKVNNWD LFFSPSEDNF TNDLDKVEEI TADTNIEAAE ENISLDLIQQ YYLT-
FDFDNE PENISIENLS SDIIGQLEPM PNIERFPNGK KYELDKYTMF HYLRAQEFEH GDSRIILTNS AEEALLKPNV
AYTFFSSKYV KKINKAVEAF MFLNWAEELV YDFTDETNEV TTMDKIADIT IIVPYIGPAL NIGNMLSKGE FVEAI-
IFTGV VAMLEFIPEY ALPVFGTFAI VSYIANKVLT VQTINNALSK RNEKWDEVYK YTVTNWLAKV NTQIDLIREK
MKKALENQAE ATKAIINYQY NQYTEEEKNN INFNIDDLSS KLNESINSAM ININKFLDQC SVSYLMNSMI
PYAVKRLKDF DASVRDVLLK YIYDNRGTLV LQVDRLKDEV NNTLSADIPF QLSKYVDNKK LLSTFTEYIK NIVNT-
SILSI VYKKDDLLDL SRYGAKINIG DRVYYDSIDK NQIKLINLES STIEVILKNA IVYNSMYENF STSFWIKIPK YF-
SKINLNNE YTIINCIENN SGWKVSLNYG EIIWTLQDNK QNIQRVVFKY SQMVNISDYI NRWIFVTITN NRLTKSKIYI
NGRLIDQKPI SNLGNIHASN KIMFKLDGCR DPRRYIMIKY FNLFDKELNE KEIKDLYDSQ SNSGILKDFW
GNYLQYDKPY YMLNLFDPNK YVDVNNIGIR GYMYLKGPRG SVVTTNIYLN STLYEGTKFI IKKYASGNED
NIVRNNDRVY INVVVKNKEY RLATNASQAG VEKILSALEI PDVGNLSQVV VMKSKDDQGI RNKCKMNLQD NNG-
NDIGFIG FHLYDNIAKL VASNWYNRQV GKASRTFGCS WEFIPVDDGW GESSL

Disulfide: 429-453 INTERCHAIN (BY SIMILARITY); 1234-1279
Function: blocking neurotransmitter release by hydrolysis of snap25.
Clostridium botulinum toxin type B
Accession number: P10844

Sequence: PVTINNFNYN DPIDNNNIIM MEPPFARGTG RYYKAFKITD RIWIIPERYT FGYKPEDFNK SSGIFNRD-
VC EYYDPDYLNT NDKKNIFLQT MIKLFNRIKS KPLGEKLLEM IINGIPYLGD RRVPLEEFNT NIASVTVNKL ISNP-
GEVERK KGIFANLIIF GPGPVLNENE TEDIGIQNHF ASREGFGGIM QMKFCPEYVS VFNNVQENKG ASIF-
NRRGYF SDPALILMHE LIHVLHGLYG IKVDDLPIVP NEKKFFMQST DAIQAEELYT FGGQDPSIIT PSTDKSIYDK
VLQNFRGIVD RLNKVLVCIS DPNININIYK NKFKDKYKFV EDSEGKYSID VESFDKLYKS LMFGFTETNI AENYKIK-
TRA SYFSDSLPPV KIKNLLDNEI YTIEEGFNIS DKDMEKEYRG QNKAINKQAY EEISKEHLAV YKIQMCKSVK
APGICIDVDN EDLFFIADKN SFSDDLSKNE RIEYNTQSNY IENDFPINEL ILDTDLISKI ELPSENTESL TDFN-
VDVPVY EKQPAIKKIF TDENTIFQYL YSQTFPLDIR DISLTSSFDD ALLFSNKVYS FFSMDYIKTA NKVVEAGLFA
GWVKQIVNDF VIEANKSNTM DKIADISLIV PYIGLALNVG NETAKGNFEN AFEIAGASIL LEFIPELLIP VVGAFLLE-
SY IDNKNKIIKT IDNALTKRNE KWSDMYGLIV AQWLSTVNTQ FYTIKEGMYK ALNYQAQALE EIIKYRYNIY
SEKEKSNINI DFNDINSKLN EGINQAIDNI NNFINGCSVS YLMKKMIPLA VEKLLDFDNT LKKNLLNYID ENKLYLIG-
SA EYEKSKVNKY LKTIMPFDLS IYTNDTILIE MFNKYNSEIL NNIILNLRYK DNNLIDLSGY GAKVEVYDGV ELND-
KNQFKL TSSANSKIRV TQNQNIIFNS VFLDFSVSFW IRIPKYKNDG IQNYIHNEYT IINCMKNNSG WKISIRGNRI
IWTLIDINGK TKSVFFEYNI REDISEYINR WFFVTITNNL NNAKIYINGK LESNTDIKDI REVIANGEII FKLDGDIDRT
QFIWMKYFSI FNTELSQSNI EERYKIQSYS EYLKDFWGNP LMYNKEYYMF NAGNKNSYIK LKKDSPVGEI
LTRSKYNQNS KYINYRDLYI GEKFIIRRKS NSQSINDDIV RKEDYIYLDF FNLNQEWRVY TYKYFKKEEE KLFLA-
PISDS DEFYNTIQIK EYDEQPTYSC QLLFKKDEES TDEIGLIGIH RFYESGIVFE EYKDYFCISK WYLKEVKRKP
YNLKLGCNWQ FIPKDEGWTE

Disulfide: 436-445 INTERCHAIN (PROBABLE).
Function: endopeptidase that cleaves synaptobrevin-2 and thus blocks neurotransmission.
Collagen
Accession number: P30754

Sequence: YRAGPRYIQA QVGPIGPRGP PGPPGSPGQQ GYQGLRGEPG DSGPMGPIGK RGPPGPAGIA GKS-
GDDGRDG EPGPRGGIGP MGPRGAGGMP GMPGPXGHRG FRGLSGSXGE QGKSGNQGPD GGPGPAGPSG
PIGPRGQTGE RGRDGKSGLP GLRGVDGLAG PPGPPGPIGS TGSPGFPGTP GSKGDRGQSG LXGAQGLQGP
VGLSGQPGVA GENGHPGMPG MDGANGEPGA SGESGLPGPS GFPGPRGMPG TAGSPGQAGA XGDGGPT-
GEQ GRPGAPGVXG SSGPPGDVGA PGHAGEAGKR GSPGSPGPAG SPGPQGDRGL PGSRGLPGMT GAS-
GAMGIPG EKGPSGEPGA KGPTGDTGRQ GNQGTPGIAG LPGNPGSDGR PGKDGRPGIR GKDGKQGEQG
PQGPQGLAGL QGRAGPPGAR GEPGKNGAPG EPGAHGEQGD AGKDGETGAA GPPGAAGPTG ARGPPG-
PRGQ QGFQGLAGAQ GTPGEAGKTG ERGAVGATGP SGPAGPGGER GAPGDRGNVG PRGMPGERGA
TGPAGPTGSP GVAGAKGQGG PPGPAGLVGL PGERGPKGVG GSXGSRGDIG PRGKAGERGK DGERGER-
GEN GLPGPSGLAA SXGERGDMGS PGERGSPGPA GERGPAGSQG IQGQPGPPGD AGPAGTXGDI GF-
PGERGTRG ATGKQGARGP RGLAGKRGLR GAGGSRGETG AQGEIGLPGS PGQPGLPGPS GQPGPSGPAG
TAGKQGVXGA RGSPGLVGKQ GDRGSDGEPG RDGTXGERGE DGPPGVSGPT GAPGQQGERG MPGM-
VGLRGE TGPMGGQGMX GDGGPPGPSG DRGERGNAGP QGPTGPSGQA GAPGQEGAPG KDGLPGLAGR
PGERGEPGVA GRAGSQGLAG LMGQRGLPGA AGPPGDRGER GEPGGQGVQG PVGAPGSQGP AGIMGMX-
GEA GGKGAXGDKG WTGLPGLQGL QGTPGHSGES GPPGAPGPRG ARGEAGGRGS QGPPGKDGQP GPS-
GRVGPRG PSGDDGRSGP PGPPGPPGPP GNSDYGA

Function: fibril formation
Collagenase
Accession number: P08897

Sequence: IINGYEAYTG LFPYQAGLDI TLQDQRRVWC GGSLIDNKWI LTAAHCVHDA VSVVVYLGSA
VQYEGEAVVN SERIISHSMF NPDTYLNDVA LIKIPHVEYT DNIQPIRLPS GEELNNKFEN IWATVSGWGQ SNT-
DTVILQY TYNLVIDNDR CAQEYPPGII VESTICGDTC DGKSPCFGDS GGPFVLSDKN LLIGVVSFVS GAGCES-
GKPV GFSRVTSYMD WIQQNTGIIF

Disulfide: 60-76; 181-196; 206-234
Function: Serine protease.
Corticotropin, ACTH
Accession number: P01189

Sequence: WCLE SSQCQDLTTE SNLLECIRAC KPDLSAETPM FPGNGDEQPL TENPRKYVMG HFRWDRFGRR
NSSSSGSSGA GQKREDVSAG EDCGPLPEGG PEPRSDGAKP GPREGKRSYS MEHFRWGKPV GKKRRPVKVY
PNGAEDESAE AFPLEFKREL TGQRLREGDG PDGPADDGAG AQADLEHSLL VAAEKKDEGP YRMEHFRWGS
PPKDKRYGGF MTSEKSQTPL VTLFKNAIIK NAYKKGE

Disulfide: 28-50 (counting with 26 aa signal)
Function: melanocyte stimulation.
Domase alfa
Accession number: P24855

LKIAAFNI QTFGETKMSN ATLVSYIVQI LSRYDIALVQ EVRDSHLTAV GKLLDNLNQD APDTYHYVVS EPLGRN-
SYKE RYLFVYRPDQ VSAVDSYYYD DGCEPCGNDT FNREPAIVRF FSRFTEVREF AIVPLHAAPG DAVAEIDALY
DVYLDVQEKW GLEDVMLMGD FNAGCSYVRP SQWSSIRLWT SPTFQWLIPD SADTTATPTH CAYDRIVVAG
MLLRGAVVPD SALPFNFQAA YGLSDQLAQA ISDHYPVEVM LK

Disulfide: 123-126; 195-231 (counted with 21 aa extra N-terminal)
Function: endonucleolytic, binds G-actin.
Eptacog alpha (factor VII)
Accession number: P08709

Sequence: NAFLEELRP GSLERECKEE QCSFEEAREI FKDAERTKLF WISYSDGDQC ASSPCQNGGS CK-
DQLQSYIC FCLPAFEGRN CETHKDDQLI CVNENGGCEQ YCSDHTGTKR SCRCHEGYSL LADGVSCTPT
VEYPCGKIPI LEKRNASKPQ GRIVGGKVCP KGECPWQVLL LVNGAQLCGG TLINTIWVVS AAHCFDKIKN WRN-
LIAVLGE HDLSEHDGDE QSRRVAQVII PSTYVPGTTN HDIALLRLHQ PVVLTDHVVP LCLPERTFSE RTLAFVRF-
SL VSGWGQLLDR GATALELMVL NVPRLMTQDC LQQSRKVGDS PNITEYMFCA GYSDGSKDSC KGDSGG-
PHAT HYRGTWYLTG IVSWGQGCAT VGHFGVYTRV SQYIEWLQKL MRSEPRPGVL LRAPFP

Disulfide: 77-82; 110-121; 115-130; 132-141; 151-162; 158-172; 174-187; 195-322; 219-224; 238-254; 370-389; 400-428 (counted with 61 aa N-terminally)
Function: coagulation.
Etanercept
Accession number: P20333

Sequence: LPAQVAFT PYAPEPGSTC RLREYYDQTA QMCCSKCSPG QHAKVFCTKT SDTVCDSCED STYTQL-
WNWV PECLSCGSRC SSDQVETQAC TREQNRICTC RPGWYCALSK QEGCRLCAPL RKCRPGFGVA RPGTET-
SDVV CKPCAPGTFS NTTSSTDICR PHQICNVVAI PGNASRDAVC TSTSPTRSMA PGAVHLPQPV STR-
SQHTQPT PEPSTAPSTS FLLPMGPSPP AEGSTGDFAL PVGLIVGVTA LGLLIIGVVN CVIMTQVKKK
PLCLQREAKV PHLPADKARG TQGPEQQHLL ITAPSSSSSS LESSASALDR RAPTRNQPQA PGVEASGAGE AR-
ASTGSSDS SPGGHGTQVN VTCIVNVCSS SDHSSQCSSQ ASSTMGDTDS SPSESPKDEQ VPFSKEECAF
RSQLETPETL LGSTEEKPLP LGVPDAGMKP S

Disulfide: 40-53; 54-67; 57-75; 78-93; 96-110; 100-118; 120-126; 134-143; 137-161; 164 179 (counted with 22 aa extra N-terminally)
Function: receptor TNF-alpha.
Erythropoietin
Accession number: P01588

Sequence: APP RLICDSRVLE RYLLEAKEAE NITTGCAEHC SLNENITVPD TKVNFYAWKR MEVGQQAVEV
WQGLALLSEA VLRGQALLVN SSQPWEPLQL HVDKAVSGLR SLTTLLRALG AQKEAISPPD AASAAPLRTI TADT-
FRKLFR VYSNFLRGKL KLYTGEACRT GDR

Disulfide: 34-188; disulfide: 56-60 (counted with 27 aa N-terminally) Erythropoietin fragment:
Sequence:
YASHFGPLGWVCK
Posttranslational lanthionine ring: S3-C12
Erythropoietin fragment2:
Sequence:
YASHFGPLTWVCK
Posttranslational lanthionine ring: S3-C12
Function: erythropoiese.
Exendin-4
Accession number: P26349
Sequence: MPVESGL SSEDSASSES FASKIKRHGE GTFTSDLSKQ MEEEAVRLFI EWLKNGGPSS GAPPPSG
86 AMIDATION (G-87 PROVIDE AMIDE GROUP). (counted with 23 aa signal N-terminally)
Function: secretin-lilce.
Factor VIII
Accession number: P00451

Sequence: A TRRYYLGAVE LSWDYMQSDL GELPVDARFP PRVPKSFPFN TSVVYKKTLF VEFTDHLFNI AK-
PRPPWMGL LGPTIQAEVY DTVVITLKNM ASHPVSLHAV GVSYWKASEG AEYDDQTSQR EKEDDKVFPG GSH-
TYVWQVL KENGPMASDP LCLTYSYLSH VDLVKDLNSG LIGALLVCRE GSLAKEKTQT LHKFILLFAV FDEG-
KSWHSE TKNSLMQDRD AASARAWPKM HTVNGYVNRS LPGLIGCHRK SVYWHVIGMG TTPEVHSIFL EGHT-
FLVRNH RQASLEISPI TFLTAQTLLM DLGQFLLFCH ISSHQHDGME AYVKVDSCPE EPQLRMKNNE EAEDYD-
DDLT DSEMDVVRFD DDNSPSFIQI RSVAKKHPKT WVHYIAAEEE DWDYAPLVLA PDDRSYKSQY LNNGPQRI-
GR KYKKVRFMAY TDETFKTREA IQHESGILGP LLYGEVGDTL LIIFKNQASR PYNIYPHGIT DVRPLYSRRL
PKGVKHLKDF PILPGEIFKY KWTVTVEDGP TKSDPRCLTR YYSSFVNMER DLASGLIGPL LICYKESVDQ RGN-

QIMSDKR NVILFSVFDE NRSWYLTENI QRFLPNPAGV QLEDPEFQAS NIMHSINGYV FDSLQLSVCL HEVAY-
WYILS IGAQTDFLSV FFSGYTFKHK MVYEDTLTLF PFSGETVFMS MENPGLWILG CHNSDFRNRG MTAL-
LKVSSC DKNTGDYYED SYEDISAYLL SKNNAIEPRS FSQNSRHPST RQKQFNATTI PENDIEKTDP
WFAHRTPMPK IQNVSSSDLL MLLRQSPTPH GLSLSDLQEA KYETFSDDPS PGAIDSNNSL SEMTHFRPQL
HHSGDMVFTP ESGLQLRLNE KLGTTAATEL KKLDFKVSST SNNLISTIPS DNLAAGTDNT SSLGPPSMPV HYD-
SQLDTTL FGKKSSPLTE SGGPLSLSEE NNDSKLLESG LMNSQESSWG KNVSSTESGR LFKGKRAHGP ALL-
TKDNALF KVSISLLKTN KTSNNSATNR KTHIDGPSLL IENSPSVWQN ILESDTEFKK VTPLIHDRML MDKNATAL-
RL NHMSNKTTSS KNMEMVQQKK EGPIPPDAQN PDMSFFKMLF LPESARWIQR THGKNSLNSG QGPSP-
KQLVS LGPEKSVEGQ NFLSEKNKVV VGKGEFTKDV GLKEMVFPSS RNLFLTNLDN LHENNTHNQE KKIQEE-
IEKK ETLIQENVVL PQIHTVTGTK NFMKNLFLLS TRQNVEGSYD GAYAPVLQDF RSLNDSTNRT KKHTAHFSKK
GEEENLEGLG NQTKQIVEKY ACTTRISPNT SQQNFVTQRS KRALKQFRLP LEETELEKRI IVDDTSTQWS KNM-
KHLTPST LTQIDYNEKE KGAITQSPLS DCLTRSHSIP QANRSPLPIA KVSSFPSIRP IYLTRVLFQD NSSHLPAASY
RKKDSGVQES SHFLQGAKKN NLSLAILTLE MTGDQREVGS LGTSATNSVT YKKVENTVLP KPDLPKTSGK
VELLPKVHIY QKDLFPTETS NGSPGHLDLV EGSLLQGTEG AIKWNEANRP GKVPFLRVAT ESSAKTPSKL LD-
PLAWDNHY GTQIPKEEWK SQEKSPEKTA FKKKDTILSL NACESNHAIA AINEGQNKPE IEVTWAKQGR TERLC-
SQNPP VLKRHQREIT RTTLQSDQEE IDYDDTISVE MKKEDFDIYD EDENQSPRSF QKKTRHYFIA AVERL-
WDYGM SSSPHVLRNR AQSGSVPQFK KVVFQEFTDG SFTQPLYRGE LNEHLGLLGP YIRAEVEDNI MVTFRN-
QASR PYSFYSSLIS YEEDQRQGAE PRKNFVKPNE TKTYFWKVQH HMAPTKDEFD CKAWAYFSDV DLEKD-
VHSGL IGPLLVCHTN TLNPAHGRQV TVQEFALFFT IFDETKSWYF TENMERNCRA PCNIQMEDPT FKENYRF-
HAI NGYIMDTLPG LVMAQDQRIR WYLLSMGSNE NIHSIHFSGH VFTVRKKEEY KMALYNLYPG VFETVEMLPS
KAGIWRVECL IGEHLHAGMS TLFLVYSNKC QTPLGMASGH IRDFQITASG QYGQWAPKLA RLHYSGSINA
WSTKEPFSWI KVDLLAPMII HGIKTQGARQ KFSSLYISQF IIMYSLDGKK WQTYRGNSTG TLMVFFGNVD SS-
GIKHNIFN PPIIARYIRL HPTHYSIRST LRMELMGCDL NSCSMPLGME SKAISDAQIT ASSYFTNMFA TWSP-
SKARLH  LQGRSNAWRP QVNNPKEWLQ VDFQKTMKVT GVTTQGVKSL LTSMYVKEFL ISSSQDGHQW
TLFFQNGKVK VFQGNQDSFT PVVNSLDPPL LTRYLRIHPQ SWVHQIALRM EVLGCEAQDL Y

Disulfide: 172-198;547-573; 1851-1877; 2040-2188; 2193-2345 (counted with 19 aa extra N-terminally)
Function: coagulation.
Factor IX
Accession number: P00740
Sequence:

Light chain: NSG KLEEFVQGNL ERECMEEKCS FEEAREVFEN TERTTEFWKQ YVDGDQCESN PCLNGGSCKD
DINSYECWCP FGFEGKNCEL DVTCNIKNGR CEQFCKNSAD NKVVCSCTEG YRLAENQKSC EPAVPFPCGR
VSVSQTSKLT R

Heavy chain: AEAVFPDVD YVNSTEAETI LDNITQSTQS FNDFTRVVGG EDAKPGQFPW QVVLNGKVDA
FCGGSIVNEK WIVTAAHCVE TGVKITVVAG EHNIEETEHT EQKRNVIRII PHHNYNAAIN KYNHDIALLE LDEPLV-
LNSY VTPICIADKE YTNIFLKFGS GYVSGWGRVF HKGRSALVLQ YLRVPLVDRA TCLRSTKFTI YNNMFCAGFH
EGGRDSCQGD SGGPHVTEVE GTSFLTGIIS WGEECAMKGK YGIYTKVSRY VNWIKEKTKL T

Disulfide: 64-69; 97-108; 102-117; 119-128; 134-145; 141-155; 157-170 (counted in the precursor)
Function: coagulation.

Factor X
Accession number: P00742
Sequence:

Light chain: ANSFLEEMKK GHLERECMEE TCSYEEAREV FEDSDKTNEF WNKYKDGDQC ETSPCQNQGK CK-
DGLGEYTC TCLEGFEGKN CELFTRKLCS LDNGDCDQFC HEEQNSVVCS CARGYTLADN GKACIPTGPY PCG-
KQTLER

Heavy chain: R KRSVAQATSS SGEAPDSITW KPYDAADLDP TENPFDLLDF NQTQPERGDN NLTRIVGGQE CK-
DGECPWQA LLINEENEGF CGGTILSEFY ILTAAHCLYQ AKRFKVRVGD RNTEQEEGGE AVHEVEVVIK HN-
RFTKETYD FDIAVLRLKT PITFRMNVAP ACLPERDWAE STLMTQKTGI VSGFGRTHEK GRQSTRLKML EVPY-
VDRNSC KLSSSFIITQ NMFCAGYDTK QEDACQGDSG GPHVTRFKDT YFVTGIVSWG EGCARKGKYG
IYTKVTAFLK WIDRSMKTRG LPKAKSHAPE VITSSPLK

Disulfide: 90-101; 95-110; 112-121; 129-140; 136-149; 151-164; 172-342; 241-246; 261 277; 390-404; 415-443
(counted with 40 aa signal sequence)
Function: coagulation, factor Xa (part of factor X-heavy chain) is a vitamin K-dependent glycoprotein that converts prothrombin into thrombin in the presence of amongst others anionic phospholipid.
Factor XIII
Accession number: P00488

Sequence: VN LQEFLNVTSV HLFKERWDTN KVDHHTDKYE NNKLIVRRGQ SFYVQIDFSR PYDPRRDLFR VE-

YVIGRYPQ ENKGTYIPVP IVSELQSGKW GAKIVMREDR SVRLSIQSSP KCIVGKFRMY VAVWTPYGVL RTSRN-
PETDT YILFNPWCED DAVYLDNEKE REEYVLNDIG VIFYGEVNDI KTRSWSYGQF EDGILDTCLY VMDRAQM-
DLS GRGNPIKVSR VGSAMVNAKD DEGVLVGSWD NIYAYGVPPS AWTGSVDILL EYRSSENPVR YGQCWVF-
AGV FNTFLRCLGI PARIVTNYFS AHDNDANLQM DIFLEEDGNV NSKLTKDSVW NYHCWNEAWM TRP-
DLPVGFG GWQAVDSTPQ ENSDGMYRCG PASVQAIKHG HVCFQFDAPF VFAEVNSDLI YITAKKDGTH VVEN-
VDATHI GKLIVTKQIG GDGMMDITDT YKFQEGQEEE RLALETALMY GAKKPLNTEG VMKSRSNVDM DFEVE-
NAVLG KDFKLSITFR NNSHNRYTIT AYLSANITFY TGVPKAEFKK ETFDVTLEPL SFKKEAVLIQ AGEYMGQLLE
QASLHFFVTA RINETRDVLA KQKSTVLTIP EIIIKVRGTQ VVGSDMTVTV QFTNPLKETL RNVWVHLDGP GVTRP-
MKKMF REIRPNSTVQ WEEVCRPWVS GHRKLIASMS SDSLRHVYGE LDVQIQRRPS M

Function: coagulation, indirectly stabilizing fibrin chains.
Fibronectin
Accession number: P02751

Sequence: QAQQMVQPQ SPVAVSQSKP GCYDNGKHYQ INQQWERTYL GNALVCTCYG GSRGFNCESK PE-
AEETCFDK YTGNTYRVGD TYERPKDSMI WDCTCIGAGR GRISCTIANR CHEGGQSYKI GDTWRRPHET GGYM-
LECVCL GNGKGEWTCK PIAEKCFDHA AGTSYVVGET WEKPYQGWMM VDCTCLGEGS GRITCTSRNR
CNDQDTRTSY RIGDTWSKKD NRGNLLQCIC TGNGRGEWKC ERHTSVQTTS SGSGPFTDVR AAVYQPQPHP
QPPPYGHCVT DSGVVYSVGM QWLKTQGNKQ MLCTCLGNGV SCQETAVTQT YGGNSNGEPC VLPFTYNGRT
FYSCTTEGRQ DGHLWCSTTS NYEQDQKYSF CTDHTVLVQT QGGNSNGALC HFPFLYNNHN YTDCTSEGRR
DNMKWCGTTQ NYDADQKFGF CPMAAHEEIC TTNEGVMYRI GDQWDKQHDM GHMMRCTCVG NGRGEWT-
CIA YSQLRDQCIV DDITYNVNDT FHKRHEEGHM LNCTCFGQGR GRWKCDPVDQ CQDSETGTFY QIGD-
SWEKYV HGVRYQCYCY GRGIGEWHCQ PLQTYPSSSG PVEVFITETP SQPNSHPIQW NAPQPSHISK YILR-
WRPKNS VGRWKEATIP GHLNSYTIKG LKPGVVYEGQ LISIQQYGHQ EVTRFDFTTT STSTPVTSNT VTGET-
TPFSP LVATSESVTE ITASSFVVSW VSASDTVSGF RVEYELSEEG DEPQYLDLPS TATSVNIPDL LPGRKYIVNV
YQISEDGEQS LILSTSQTTA PDAPPDPTVD QVDDTSIVVR WSRPQAPITG YRIVYSPSVE GSSTELNLPE TANS-
VTLSDL QPGVQYNITI YAVEENQEST PVVIQQETTG TPRSDTVPSP RDLQFVEVTD VKVTIMWTPP ESAVT-
GYRVD VIPVNLPGEH GQRLPISRNT FAEVTGLSPG VTYYFKVFAV SHGRESKPLT AQQTTKLDAP TN-
LQFVNETD STVLVRWTPP RAQITGYRLT VGLTRRGQPR QYNVGPSVSK YPLRNLQPAS EYTVSLVAIK GN-
QESPKATG VFTTLQPGSS IPPYNTEVTE TTIVITWTPA PRIGFKLGVR PSQGGEAPRE VTSDSGSIVV SGLT-
PGVEYV YTIQVLRDGQ ERDAPIVNKV VTPLSPPTNL HLEANPDTGV LTVSWERSTT PDITGYRITT
TPTNGQQGNS LEEVVHADQS SCTFDNLSPG LEYNVSVYTV KDDKESVPIS DTIIPAVPPP TDLRFTNIGP DTM-
RVTWAPP PSIDLTNFLV RYSPVKNEED VAELSISPSD NAVVLTNLLP GTEYVVSVSS VYEQHESTPL RGRQKT-
GLDS PTGIDFSDIT ANSFTVHWIA PRATITGYRI RHHPEHFSGR PREDRVPHSR NSITLTNLTP GTEYVVSIVA
LNGREESPLL IGQQSTVSDV PRDLEVVAAT PTSLLISWDA PAVTVRYYRI TYGETGGNSP VQEFTVPGSK STA-
TISGLKP GVDYTITVYA VTGRGDSPAS SKPISINYRT EIDKPSQMQV TDVQDNSISV KWLPSSSPVT GYRVTT-
TPKN GPGPTKTKTA GPDQTEMTIE GLQPTVEYVV SVYAQNPSGE SQPLVQTAVT NIDRPKGLAF TDVDVDSIKI
AWESPQGQVS RYRVTYSSPE DGIHELFPAP DGEEDTAELQ GLRPGSEYTV SVVALHDDME SQPLIGTQST AI-
PAPTDLKF TQVTPTSLSA QWTPPNVQLT GYRVRVTPKE KTGPMKEINL APDSSSVVVS GLMVATKYEV SVY-
ALKDTLT SRPAQGVVTT LENVSPPRRA RVTDATETTI TISWRTKTET ITGFQVDAVP ANGQTPIQRT IKPDVRSY-
TI TGLQPGTDYK IYLYTLNDNA RSSPVVIDAS TAIDAPSNLR FLATTPNSLL VSWQPPRARI TGYIIKYEKP GSP-
PREVVPR PRPGVTEATI TGLEPGTEYT IYVIALKNNQ KSEPLIGRKK TDELPQLVTL PHPNLHGPEI LDVP-
STVQKT PFVTHPGYDT GNGIQLPGTS GQQPSVGQQM IFEEHGFRRT TPPTTATPIR HRPRPYPPNV GEEI-
QIGHIP REDVDYHLYP HGPGLNPNAS TGQEALSQTT ISWAPFQDTS EYIISCHPVG TDEEPLQFRV PGTSTS-
ATLT GLTRGATYNI IVEALKDQQR HKVREEVVTV GNSVNEGLNQ PTDDSCFDPY TVSHYAVGDE WERMSES-
GFK LLCQCLGFGS GHFRCDSSRW CHDNGVNYKI GEKWDRQGEN GQMMSCTCLG NGKGEFKCDP HEATCY-
DDGK TYHVGEQWQK EYLGAICSCT CFGGQRGWRC DNCRRPGGEP SPEGTTGQSY NQYSQRYHQR
TNTNVNCPIE CFMPLDVQAD REDSRE

Disulfide: 52-78; 76-87; 97-125; 123-135; 141-169; 167-179; 186-215; 213-225; 231-260; 258-270; 308-335; 333-342; 360-386; 374-401; 420-446; 434-461; 470-498; 496-508; 518 545; 543-555; 561-589; 587-599; 2206-2235; 2233-2245; 2251-2278; 2276-2288; 2295-2319; 2317-2333; 2367-2367; 2371-2371 INTERCHAIN (WITH 2367 OF OTHER CHAIN). (counted with 31 aa extra N-terminally)
Function: wound healing, cell shape.
Fibrinogen
Accession number: P02671

Sequence: GPRVV ERHQSACKDS DWPFCSDEDW NYKCPSGCRM KGLIDEVNQD FTNRINKLKN
SLFEYQKNNK DSHSLTTNIM EILRGDFSSA NNRDNTYNRV SEDLRSRIEV LKRKVIEKVQ HIQLLQKNVR
AQLVDMKRLE VDIDIKIRSC RGSCSRALAR EVDLKDYEDQ QKQLEQVIAK DLLPSRDRQH LPLIKMKPVP
DLVPGNFKSQ LQKVPPEWKA LTDMPQMRME LERPGGNEIT RGGSTSYGTG SETESPRNPS SAGSWNSGSS
GPGSTGNRNP GSSGTGGTAT WKPGSSGPGS TGSWNSGSSG TGSTGNQNPG SPRPGSTGTW NPGSSERG-

SA GHWTSESSVS GSTGQWHSES GSFRPDSPGS GNARPNNPDW GTFEEVSGNV SPGTRREYHT EKLVT-
SKGDK ELRTGKEKVT SGSTTTTRRS CSKTVTKTVI GPDGHKEVTK EVVTSEDGSD CPEAMDLGTL SGIGTLDG-
FR HRHPDEAAFF DTASTGKTFP GFFSPMLGEF VSETESRGSE SGIFTNTKES SSHHPGIAEF PSRGKSSSYS
KQFTSSTSYN RGDSTFESKS YKMADEAGSE ADHEGTHSTK RGHAKSRPVR DCDDVLQTHP SGTQSGIFNI
KLPGSSKIFS VYCDQETSLG GWLLIQQRMD GSLNFNRTWQ DYKRGFGSLN DEGEGEFWLG NDYLHLLTQR
GSVLRVELED WAGNEAYAEY HFRVGSEAEG YALQVSSYEG TAGDALIEGS VEEGAEYTSH NNMQFSTFDR
DADQWEENCA EVYGGGWWYN NCQAANLNGI YYPGGSYDPR NNSPYEIENG VVWVSFRGAD YSLRAVRMKI
RPLVTQ

Disulfide: 47-47 INTERCHAIN (WITH C-47'); 55-55 INTERCHAIN (WITH C-95 IN BETA); 64 -64 INTERCHAIN (WITH C-49 IN GAMMA); 68-68 INTERCHAIN (WITH C-106 IN BETA); 180 -180 INTERCHAIN (WITH C-165 IN GAMMA); 184-184 INTERCHAIN (WITH C-223 IN BETA); 461-491
Function: fibrin formation, platelet aggregation.
Filgrastim
Accession number: P09919

Sequence: TPLGPASSLP QSFLLKCLEQ VRKIQGDGAA LQEKLVSECA TYKLCHPEEL VLLGHSLGIP WAPLSS-
CPSQ ALQLAGCLSQ LHSGLFLYQG LLQALEGISP ELGPTLDTLQ LDVADFATTI WQQMEELGMA PALQPTQ-
GAM PAFASAFQRR AGGVLVASHL QSFLEVSYRV LRHLAQP

Disulfide: 69-75; 97-107 (counted with 30 aa N-terminally)
Function: granulocyte stimulation.
Follitropin alpha
Accession number: P37036

Sequence: FPBGZFTMZG CPZCKLKZBK YFSKLGAPIY ZCMGCCFSRA YPTPARSKKT MLVPKNITSZ ATCC-
VAKAFT KATVMGBARV ZNHTZCHCST CYYHKS

Disulfide: 11-35; 14-64; 32-86; 36-88; 63-91
Function: follicle stimulation.
Follitropin beta
Accession numbers: P01225

Sequence: S CELTNITIAI EKEECRFCIS INTTWCAGYC YTRDLVYKDP ARPKIQKTCT FKELVYETVR VPGCAH-
HADS LYTYPVATQC HCGKCDSDST DCTVRGLGPS YCSFGEMKE

Disulfide: 21-69; 35-84; 38-122; 46-100; 50-102; 105-112 (counted with 18 aa N-terminally)
Function: follicle stimulation.
Growth hormone releasing hormone
Accession number: P48144
Sequence: HADGLLDR ALRDILVQLS ARKYLHSLTA VRVGEEEEDE EDSEPLS
Function: growth hormone release.
Pituitary adenylate cyclase activating polypeptide
Accession number: P48144
Sequence: H SDGIFTDSYS RYRKQMAVKK YLAAVLGRRY RQRFRN (amidation of last residue)
Function: see name.
Hyaluronidase
Accession number: P38567

Sequence: LNFRA PPVIPNVPFL WAWNAPSEFC LGKFDEPLDM SLFSFIGSPR INATGQGVTI FYVDRLGYYP
YIDSITGVTV NGGIPQKISL QDHLDKAKKD ITFYMPVDNL GMAVIDWEEW RPTWARNWKP KDVYKNRSIE
LVQQQNVQLS LTEATEKAKQ EFEKAGKDFL VETIKLGKLL RPNHLWGYYL FPDCYNHHYK KPGYNGSCFN
VEIKRNDDLS WLWNESTALY PSIYLNTQQS PVAATLYVRN RVREAIRVSK IPDAKSPLPV FAYTRIVFTD QVLK-
FLSQDE LVYTFGETVA LGASGIVIWG TLSIMRSMKS CLLLDNYMET ILNPYIINVT LAAKMCSQVL CQEQGVCIRK
NWNSSDYLHL NPDNFAIQLE KGGKFTVRGK PTLEDLEQFS EKFYCSCYST LSCKEKADVK DTDAVDVCIA DGV-
CIDAFLK PPMETEEPQI FYNASPSTLS ATMFIVSILF LIISSVASL

Function: glycosyl hydrolase.
Hirudin II
Accession number: P28504
Sequence: ITYTDCTESG QDLCLCEGSN VCGKGNKCIL GSNGEENQCV TGEGTPKPQS HNDGDFEEIP EEYLQ
Disulfide: 6-14; 16-28; 22-39
Function: thrombin inhibitor.
Imiglucerase
Accession number: P04062

Sequence: -
-

-


-


Function: Glucohydrolase.
Interleukin 2
Accession number: P01585

Sequence: -


Disulfide: 78-125 (counted with 20 aa signal N-terminally)
Function: growth factor.
Interferon alpha-4
Accession numbers: P01562

Sequence: CDLPETH SLDNRRTLML LAQMSRISPS SCLMDRHDFG FPQEEFDGNQ FQKAPAISVL HELIQQIFNL
FTTKDSSAAW DEDLLDKFCT ELYQQLNDLE ACVMQEERVG ETPLMNADSI LAVKKYFRRI TLYLTEKKYS
PCAWEVVRAE IMRSLSLSTN LQERLRRKE

Disulfide: 24-122; 52-162 (counted with 23 aa N-terminally)
Function: Antiviral, interferon stimulates the production of two enzymes, a protein kinase and an oligoadenylate synthetase.
Interferon-beta
Accession numbers: P01575

Sequence: INYKQLQLQ ERTNIRKCQE LLEQLNGKIN LTYRADFKIP MEMTEKMQKS YTAFAIQEML QNVFLV-
FRNN FSSTGWNETI VVRLLDELHQ QTVFLKTVLE EKQEERLTWE MSSTALHLKS YYWRVQRYLK LMKYN-
SYAWM VVRAEIFRNF LIIRRLTRNF QN

Function: antiviral, antibacterial and anticancer.
Intrinsic factor
Accession number: P27352

Sequence: ST QTQSSCSVPS AQEPLVNGIQ VLMENSVTSS AYPNPSILIA MNLAGAYNLK AQKLLTYQLM SSDN-
NDLTIG HLGLTIMALT SSCRDPGDKV SILQRQMENW APSSPNAEAS AFYGPSLAIL ALCQKNSEAT LPIAVR-
FAKT LLANSSPFNV DTGAMATLAL TCMYNKIPVG SEEGYRSLFG QVLKDIVEKI SMKIKDNGII GDIYSTGLAM
QALSVTPEPS KKEWNCKKTT DMILNEIKQG KFHNPMSIAQ ILPSLKGKTY LDVPQVTCSP DHEVQPTLPS NPG-
PGPTSAS NITVIYTINN QLRGVELLFN ETINVSVKSG SVLLVVLEEA QRKNPMFKFE TTMTSWGLVV SSINNI-
AENV NHKTYWQFLS GVTPLNEGVA DYIPFNHEHI TANFTQY

Disulfide: 26-246; 103-288; 143-182 (counted with 18 aa N-terminally extra) Function: cobalamin endocytosis.
Invertase
Accession number: Q60115

Sequence: MFNFNASRWT RAQAMKVNKF DLTTSMPEIG TDFPINIRDDL WLWDTWPLRD INGNPVSFKG WNV-
IFSLVAD RNIPWNDRHS HARIGYFYSK DGKSWVYGGH LLQESANTRT AEWSGGTIMA PGSRNQVETF FT-
STLFDKNG VREAVAAVTK GRIYADSEGV WFKGFDQSTD LFQADGLFYQ NYAENNLWNF RDPHVFINPE DG-
ETYALFEA NVATVRGEDD IGEDEIGPVP ANTVVPKDAN LCSASIGIAR CLSPDRTEWE LLPPLLTAFG
VNDQMERPHV IFQNGLTYLF TISHDSTYAD GLTGSDGLYG FVSENGIFGP YEPLNGSGLV LGGPASQPTE
AYAHYIMNNG LVESFINEII DPKSGKVIAG GSLAPTVRVE LQGHETFATE VFDYGYIPAS YAWPVWPFPD RRK

Function: sucrase.
Lepirudin
Accession number: P01050
Sequence: VVYTDCTESG QNLCLCEGSN VCGQGNKCIL GSDGEKNQCV TGEGTPKPQS HNDGDFEEIP EEYLQ
Disulfides: 6-14; 16-28
Function: thrombin inhibitor.
Lutropin beta
Accession number: P01229

Sequence: SREPLRPWCH PINAILAVEK EGCPVCITVN TTICAGYCPT MMRVLQAVLP PLPQVVCTYR DVRFE-
SIRLP GCPRGVDPVV SFPVALSCRC GPCRRSTSDC GGPKDHPLTC DHPQLSGLLF L

Disulfide: 29-77; 43-92; 46-130; 54-108; 58-110; 113-120
Function: stimulates synthesis of steroids.
Lysozyme
Accession number: P21270

Sequence: MDPRLREEVV RLIIALTSDN GASLSKRLQS RVSALEKTSQ IHSDTILRIT QGLDDANKRI IALEQSRD-

DL VASVSDAQLA ISRLESSIGA LQTVVNGLDS SVTQLGARVG QLETGLADVR VDHDNLVARV DTAERNIGSL
TTELSTLTLR VTSIQADFES RISTLERTAV TSAGAPLSIR NNRITMGLND GLTLSGNNLA IRLPGNTGLN IQNG-
GLQFRF NTDQFQIVNN NLTLKTTVFD SINSRIGATE QSYVASAVTP LRLNSSTKVL DMLIDMSTLE INSSGQLTVR
STSPNLRYPI ADVSGGIGMS PNYRFR

Function: hydrolysis peptidoglycan.
Metalloproteinase inhibitor
Accession number: P16035

Sequence: CSCS PVHPQQAFCN ADVVIRAKAV SEKEVDSGND IYGNPIKRIQ YEIKQIKMFK GPEKDIEFIY
TAPSSAVCGV SLDVGGKKEY LIAGKAEGDG KMHITLCDFI VPWDTLSTTQ KKSLNHRYQM GCECKITRCP MI-
PCYISSPD ECLWMDWVTE KNINGHQAKF FACIKRSDGS CAWYRGAAPP KQEFLDIEDP

Disulfides: 27-98; 29-127; 39-152; 154-201; 159-164; 172-193 (counted with 26 aa N-terminally)
Function: inactivation protease.
Neurophysin
Accession number: P01185

Sequence: AMSDLELRQ CLPCGPGGKG RCFGPSICCA DELGCFVGTA EALRCQEENY LPSPCQSGQK ACGS-
GGRCAA FGVCCNDESC VTEPECREGF HRRA

Disulfide: 41-85; 44-58; 52-75; 59-65; 92-104; 98-116; 105-110
Function: Neurophysin binds vasopressin.

Papain
Accession number: P00784

Sequence: VY MGLSFGDFSI VGYSQNDLTS TERLIQLFES WMLKHNKIYK NIDEKIYRFE IFKDNLKYID
ETNKKNNSYW LGLNVFADMS NDEFKEKYTG SIAGNYTTTE LSYEEVLNDG DVNIPEYVDW RQKGAVTPVK
NQGSCGSCWA FSAVVTIEGI IKIRTGNLNE YSEQELLDCD RRSYGCNGGY PWSALQLVAQ YGIHYRNTYP
YEGVQRYCRS REKGPYAAKT DGVRQVQPYN EGALLYSIAN QPVSVVLEAA GKDFQLYRGG IFVGPCGNKV
DHAVAAVGYG PNYILIKNSW GTGWGENGYI RIKRGTGNSY GVCGLYTSSF YPVKN

Disulfide: 155-196; 189-228; 286-333 (counted with 18 aa N-terminally)
Function: Proteinase.
Pepsin
Accession number: P00790

Sequence: VDEQPLEN YLDMEYFGTI GIGTPAQDFT VVFDTGSSNL WVPSVYCSSL ACTNHNRFNP
EDSSTYQSTS ETVSITYGTG SMTGILGYDT VQVGGISDTN QIFGLSETEP GSFLYYAPFD GILGLAYPSI SSS-
GATPVFD NIWNQGLVSQ DLFSVYLSAD DQSGSVVIFG GIDSSYYTGS LNWVPVTVEG YWQITVDSIT MNGEAI-
ACAE GCQAIVDTGT SLLTGPTSPI ANIQSDIGAS ENSDGDMVVS CSAISSLPDI VFTINGVQYP VPPSAYILQS
EGSCISGFQG MNLPTESGEL WILGDVFIRQ YFTVFDRANN QVGLAPVA

Disulfide: 107-112; 268-272; 311-344 (counted with 62 aa N-terminally)
Function: Peptidase.
Plasminogen
Accession number: P00747

Sequence: E PLDDYVNTQG ASLFSVTKKQ LGAGSIEECA AKCEEDEEFT CRAFQYHSKE QQCVIMAENR KSSI-
IIRMRD VVLFEKKVYL SECKTGNGKN YRGTMSKTKN GITCQKWSST SPHRPRFSPA THPSEGLEEN YCRNP-
DNDPQ GPWCYTTDPE KRYDYCDILE CEEECMHCSG ENYDGKISKT MSGLECQAWD SQSPHAHGYI PSKF-
PNKNLK KNYCRNPDRE LRPWCFTTDP NKRWELCDIP RCTTPPPSSG PTYQCLKGTG ENYRGNVAVT VSGHT-
CQHWS AQTPHTHNRT PENFPCKNLD ENYCRNPDGK RAPWCHTTNS QVRWEYCKIP SCDSSPVSTE
QLAPTAPPEL TPVVQDCYHG DGQSYRGTSS TTTTGKKCQS WSSMTPHRHQ KTPENYPNAG LTMNYCRNPD
ADKGPWCFTT DPSVRWEYCN LKKCSGTEAS VVAPPPVVLL PDVETPSEED CMFGNGKGYR GKRATTVTGT
PCQDWAAQEP HRHSIFTPET NPRAGLEKNY CRNPDGDVGG PWCYTTNPRK LYDYCDVPQC AAPSFDCGKP
QVEPKKCPGR VVGGCVAHPH SWPWQVSLRT RFGMHFCGGT LISPEWVLTA AHCLEKSPRP SSYKVILGAH
QEVNLEPHVQ EIEVSRLFLE PTRKDIALLK LSSPAVITDK VIPACLPSPN YVVADRTECF ITGWGETQGT
FGAGLLKEAQ LPVIENKVCN RYEFLNGRVQ STELCAGHLA GGTDSCQGDS GGPLVCFEKD KYILQGVTSW
GLGCARPNKP GVYVRVSRFV TWIEGVMRNN

Disulfide: 49-73; 53-61; 103-181; 124-164; 152-176; 185-262; 188-316; 206-245; 234-257; 275- 352; 296-335; 324-347; 377-454; 398-437; 426-449; 481-560; 502-543; 531-555; 567-685; 577- 585; 607-623; 699-766; 729-745; 756-784
Function: Protease.
Protamine
Accession number: P04554

Sequence: MVRYRVRSLS ERSHEVYRQQ LHGQEQGHHG QEEQGLSPEH VEVYERTHGQ SHYRRRHCSR

RRLHRIHRRQ HRSCRRRKRR SCRHRRRHRR GCRTRKRTCR RH

Function: histon substitution.
Prothrombin
Accession number: P12259

Sequence: AQ LRQFYVAAQG ISWSYRPEPT NSSLNLSVTS FKKIVYREYE PYFKKEKPQS TISGLLGPTL YAE-
VGDIIKV HFKNKADKPL SIHPQGIRYS KLSEGASYLD HTFPAEKMDD AVAPGREYTY EWSISEDSGP THDDPP-
CLTH IYYSHENLIE DFNSGLIGPL LICKKGTLTE GGTQKTFDKQ IVLLFAVFDE SKSWSQSSSL MYTVNGYVNG
TMPDITVCAH DHISWHLLGM SSGPELFSIH FNGQVLEQNH HKVSAITLVS ATSTTANMTV GPEGKWIISS LTP-
KHLQAGM QAYIDIKNCP KKTRNLKKIT REQRRHMKRW EYFIAAEEVI WDYAPVIPAN MDKKYRSQHL DNFSN-
QIGKH YKKVMYTQYE DESFTKHTVN PNMKEDGILG PIIRAQVRDT LKIVFKNMAS RPYSIYPHGV TFSPYEDEVN
SSFTSGRNNT MIRAVQPGET YTYKWNILEF DEPTENDAQC LTRPYYSDVD IMRDIASGLI GLLLICKSRS LDR-
RGIQRAA DIEQQAVFAV FDENKSWYLE DNINKFCENP DEVKRDDPKF YESNINISTIN GYVPESITTL GFCFD-
DTVQW HFCSVGTQNE ILTIHFTGHS FIYGKRHEDT LTLFPMRGES VTVTMDNVGT WMLTSMNSSP RSKKLR-
LKFR DVKCIPDDDE DSYEIFEPPE STVMATRKMH DRLEPEDEES DADYDYQNRL AAALGIRSFR NS-
SLNQEEEE FNLTALALEN GTEFVSSNTD IIVGSNYSSP SNISKFTVNN LAEPQKAPSH QQATTAGSPL RHLIG-
KNSVL NSSTAEHSSP YSEDPIEDPL QPDVTGIRLL SLGAGEFKSQ EHAKHKGPKV ERDQAAKHRF SWMKL-
LAHKV GRHLSQDTGS PSGMRPWEDL PSQDTGSPSR MRPWKDPPSD LLLLKQSNSS KILVGRWHLA SEKG-
SYEIIQ DTDEDTAVNN WLISPQNASR AWGESTPLAN KPGKQSGHPK FPRVRHKSLQ VRQDGGKSRL KKSQF-
LIKTR KKKKEKHTHH APLSPRTFHP LRSEAYNTFS ERRLKHSLVL HKSNETSLPT DLNQTLPSMD FGWIASLP-
DH NQNSSNDTGQ ASCPPGLYQT VPPEEHYQTF PIQDPDQMHS TSDPSHRSSS PELSEMLEYD RSHKSFPTDI
SQMSPSSEHE VWQTVISPDL SQVTLSPELS QTNLSPDLSH TTLSPELIQR NLSPALGQMP ISPDLSHTTL SP-
DLSHTTLS LDLSQTNLSP ELSQTNLSPA LGQMPLSPDL SHTTLSLDFS QTNLSPELSH MTLSPELSQT NLSPAL-
GQMP ISPDLSHTTL SLDFSQTNLS PELSQTNLSP ALGQMPLSPD PSHTTLSLDL SQTNLSPELS QTNLSPDLSE
MPLFADLSQI PLTPDLDQMT LSPDLGETDL SPNFGQMSLS PDLSQVTLSP DISDTTLLPD LSQISPPPDL DQI-
FYPSESS QSLLLQEFNE SFPYPDLGQM PSPSSPTLND TFLSKEFNPL VIVGLSKDGT DYIEIIPKEE VQSSED-
DYAE IDYVPYDDPY KTDVRTNINS SRDPDNIAAW YLRSNNGNRR NYYIAAEEIS WDYSEFVQRE TDIEDSDDIP
EDTTYKKVVF RKYLDSTFTK RDPRGEYEEH LGILGPIIRA EVDDVIQVRF KNLASRPYSL HAHGLSYEKS SEG-
KTYEDDS PEWFKEDNAV QPNSSYTYVW HATERSGPES PGSACRAWAY YSAVNPEKDI HSGLIGPLLI CQKGIL-
HKDS NMPVDMREFV LLFMTFDEKK SWYYEKKSRS SWRLTSSEMK KSHEFHAING MIYSLPGLKM
YEQEWVRLHL LNIGGSQDIH VVHFHGQTLL ENGNKQHQLG VWPLLPGSFK TLEMKASKPG WWLLNTEVGE
NQRAGMQTPF LIMDRDCRMP MGLSTGIISD SQIKASEFLG YWEPRLARLN NGGSYNAWSV EKLAAEFASK
PWIQVDMQKE VIITGIQTQG AKHYLKSCYT TEFYVAYSSN QINWQIFKGN STRNVMYFNG NSDASTIKEN QFDP-
PIVARY IRISPTRAYN RPTLRLELQG CEVNGCSTPL GMENGKIENK QITASSFKKS WWGDYWEPFR ARLNAQ-
GRVN AWQAKANNNK QWLEIDLLKI KKITAIITQG CKSLSSEMYV KSYTIHYSEQ GVEWKPYRLK SSMVDKIFEG
NTNTKGHVKN FFNPPIISRF IRVIPKTWNQ SITLRLELFG CDIY

Disulfide: 167-193; 500-526; 1725-1751; 1907-2061; 2066-2221 (counted with 28 N-terminal aa)
Function: Coagulation.
Protirelin
Accession number: P20396

Sequence: QPEAAQ QEAVTAAEHP GLDDFLRQVE RLLFLRENIQ RLQGDQGEHS ASQIFQSDWL SKRQHPG-
KRE EEEEEGVEEE EEEEGGAVGP HKRQHPGRRE DEASWSVDVT QHKRQHPGRR SPWLAYAVPK RQHP-
GRRLAD PKAQRSWEEE EEEEEREEDL MPEKRQHPGK RALGGPCGPQ GAYGQAGLLL GLLDDLSRSQ
GAEEKRQHPG RRAAWVREPL EE

Function: thyrotropin release.
SC3
Accession number: P16933
Sequence:

GGHPGT TTPPVTTTVT VTTPPSTTTI AAGGTCTTGS LSCCNQVQSA SSSPVTALLG LLGIVLSDLN VLVGISCS-
PL TVIGVGGSGC SAQTVCCENT QFNGLINIGC TPINIL

Function: hydrophobin.
Sermorelin
Accession number: P01286
Sequence: YADAIFTNS YRKVLGQLSA RKLLQDIMSR QQGESNQERG ARARL
Function: growth hormone release.

Streptodornase
Accession number: P26295

IPPYHH NTVLAKTVSV NQTYGEYKDY YTVIGESNID QSAFPKIYKT TERVYKGQGT SEKRVTVSDV VYN-

PLDGYKR STGAYGVVTK DMIDMSKGYR EKWETNPEPS GWFRFYNRAD NEEISEKEYD SRRTKSYKVT
NNVPVVLTTL KGKKYNSHLF VASHLFADSL GGKSIRKNAI TGTQMQNVGT RKGGMQYIEK KVLSHITKNP DVY-
VFYSAIP EYQGAELLAR SVLVSALSSD GVINETVRVF NTADGFNINY EKGGLLTESP VSEIDNIEDS TTDEIENS-
VD DSEEIVYNDT TTEEEEN

Function: DNAse.
Streptokinase
Accession number: P00779

Sequence: IAGP EWLLDRPSVN NSQLVVSVAG TVEGTNQDIS LKFFEIDLTS RPAHGGKTEQ GLSPKSKPFA
TDSGAMSHKL EKADLLKAIQ EQLIANVHSN DDYFEVIDFA SDATITDRNG KVYFADKDGS VTLPTQPVQE
FLLSGHVRVR PYKEKPIQNQ AKSVDVEYTV QFTPLNPDDD FRPGLKDTKL LKTLAIGDTI TSQELLAQAQ
SILNKNHPGY TIYERDSSIV THDNDIFRTI LPMDQEFTYR VKNREQAYRI NKKSGLNEEI NNTDLISEKY YV-
LKKGEKPY DPFDRSHLKL FTIKYVDVDT NELLKSEQLL TASERNLDFR DLYDPRDKAK LLYNNLDAFG IM-
DYTLTGKV EDNHDDTNRI ITVYMGKRPE GENASYHLAY DKDRYTEEER EVYSYLRYTG TPIPDNPND

Function: activating plasminogen.
Thyroglobulin
Accession number: P01266

Sequence: N IFEYQVDAQP LRPCELQRET AFLKQADYVP QCAEDGSFQT VQCQNDGRSC WCVGANGSEV LG-
SRQPGRPV ACLSFCQLQK QQILLSGYIN STDTSYLPQC QDSGDYAPVQ CDVQQVQCWC VDAEGMEVYG
TRQLGRPKRC PRSCEIRNRR LLHGVGDKSP PQCSAEGEFM PVQCKFVNTT DMMIFDLVHS YNRFPDAFVT
FSSFQRRFPE VSGYCHCADS QGRELAETGL ELLLDEIYDT IFAGLDLPST FTETTLYRIL QRRFLAVQSV IS-
GRFRCPTK CEVERFTATS FGHPYVPSCR RNGDYQAVQC QTEGPCWCVD AQGKEMHGTR QQGEPPSCAE
GQSCASERQQ ALSRLYFGTS GYFSQHDLFS SPEKRWASPR VARFATSCPP TIKELFVDSG LLRPMVEGQS
QQFSVSENLL KEAIRAIFPS RGLARLALQF TTNPKRLQQN LFGGKFLVNV GQFNLSGALG TRGTFNFSQF
FQQLGLASFL NGGRQEDLAK PLSVGLDSNS STGTPEAAKK DGTMNKPTVG SFGFEINLQE NQNALKFLAS
LLELPEFLLF LQHAISVPED VARDLGDVME TVLSSQTCEQ TPERLFVPSC TTEGSYEDVQ CFSGECWCVN
SWGKELPGSR VRGGQPRCPT DCEKQRARMQ SLMGSQPAGS TLFVPACTSE GHFLPVQCFN SECYCVDAEG
QAIPGTRSAI GKPKKCPTPC QLQSEQAFLR TVQALLSNSS MLPTLSDTYI PQCSTDGQWR QVQCNGPPEQ
VFELYQRWEA QNKGQDLTPA KLLVKIMSYR EAASGNFSLF IQSLYEAGQQ DVFPVLSQYP SLQDVPLAAL EG-
KRPQPREN ILLEPYLFWQ ILNGQLSQYP GSYSDFSTPL AHFDLRNCWC VDEAGQELEG MRSEPSKLPT CPG-
SCEEAKL RVLQFIRETE EIVSASNSSR FPLGESFLVA KGIRLRNEDL GLPPLFPPRE AFAEQFLRGS DYAIR-
LAAQS TLSFYQRRRF SPDDSAGASA LLRSGPYMPQ CDAFGSWEPV QCHAGTGHCW CVDEKGGFIP
GSLTARSLQI PQCPTTCEKS RTSGLLSSWK QARSQENPSP KDLFVPACLE TGEYARLQAS GAGTWCVDPA
SGEELRPGSS SSAQCPSLCN VLKSGVLSRR VSPGYVPACR AEDGGFSPVQ CDQAQGSCWC VMDSGEEVPG
TRVTGGQPAC ESPRCPLPFN ASEVVGGTIL CETISGPTGS AMQQCQLLCR QGSWSVFPPG PLICSLESGR
WESQLPQPRA CQRPQLWQTI QTQGHFQLQL PPGKMCSADY AGLLQTFQVF ILDELTARGF CQIQVKTFGT
LVSIPVCNNS SVQVGCLTRE RLGVNVTWKS RLEDIPVASL PDLHDIERAL VGKDLLGRFT DLIQSGSFQL HLD-
SKTFPAE TIRFLQGDHF GTSPRTWFGC SEGFYQVLTS EASQDGLGCV KCPEGSYSQD EECIPCPVGF
YQEQAGSLAC VPCPVGRTTI SAGAFSQTHC VTDCQRNEAG LQCDQNGQYR ASQKDRGSGK AFCVDGEGRR
LPWWETEAPL EDSQCLMMQK FEKVPESKVI FDANAPVAVR SKVPDSEFPV MQCLTDCTED EACSFFTVST TE-
PEISCDFY AWTSDNVACM TSDQKRDALG NSKATSFGSL RCQVKVRSHG QDSPAVYLKK GQGSTTTLQK
RFEPTGFQNM LSGLYNPIVF SASGANLTDA HLFCLLACDR DLCCDGFVLT QVQGGAIICG LLSSPSVLLC NVK-
DWMDPSE AWANATCPGV TYDQESHQVI LRLGDQEFIK SLTPLEGTQD TFTNFQQVYL WKDSDMGSRP ESM-
GCRKDTV PRPASPTEAG LTTELFSPVD LNQVIVNGNQ SLSSQKHWLF KHLFSAQQAN LWCLSRCVQE HSFC-
QLAEIT ESASLYFTCT LYPEAQVCDD IMESNAQGCR LILPQMPKAL FRKKVILEDK VKNFYTRLPF QKLMGISIRN
KVPMSEKSIS NGFFECERRC DADPCCTGFG FLNVSQLKGG EVTCLTLNSL GIQMCSEENG GAWRILDCGS
PDIEVHTYPF GWYQKPIAQN NAPSFCPLVV LPSLTEKVSL DSWQSLALSS VVVDPSIRHF DVAHVSTAAT SNF-
SAVRDLC LSECSQHEAC LITTLQTQPG AVRCMFYADT QSCTHSLQGQ NCRLLLREEA THIYRKPGIS LLS-
YEASVPS VPISTHGRLL GRSQAIQVGT SWKQVDQFLG VPYAAPPLAE RRFQAPEPLN WTGSWDASKP RAS-
CWQPGTR TSTSPGVSED CLYLNVFIPQ NVAPNASVLV FFHNTMDREE SEGWPAIDGS FLAAVGNLIV VTASYR-
VGVF GFLSSGSGEV SGNWGLLDQV AALTWVQTHI RGFGGDPRRV SLAADRGGAD VASIHLLTAR ATNSQL-
FRRA VLMGGSALSP AAVISHERAQ QQAIALAKEV SCPMSSSQEV VSCLRQKPAN VLNDAQTKLL AVSGPFHY-
WG PVIDGHFLRE PPARALKRSL WVEVDLLIGS SQDDGLINRA KAVKQFEESR GRTSSKTAFY QALQNSLGGE
DSDARVEAAA TWYYSLEHST DDYASFSRAL ENATRDYFII CPIIDMASAW AKRARGNVFM YHAPENYGHG
SLELLADVQF ALGLPFYPAY EGQFSLEEKS LSLKIMQYFS HFIRSGNPNY PYEFSRKVPT FATPWPDFVP RAG-
GENYKEF SELLPNRQGL KKADCSFWSK YISSLKTSAD GAKGGQSAES EEEELTAGSG LREDLLSLQE PGSK-
TYSK

Function: precursor thyroid hormone.
Urokinase,
accession: P00749
Sequence: 
MRALLARLLLCVLVVSDSKGSNELHQVPSNCDCLNGGTCVSNKYF FT SNIHWCNCPKKFGGQHCEIDKSKT-
CYEGNGHFYRGKASTDTMGRPCLFWNS ATVLQQTYFTHAHRSDALQLGLGKHNYCRNPDNRRRPWCY-
VQVGLKPLVQ ECMVHDCADGKKPSSPPEEFTLKFQCGQKTLRPRFKIIGGEFTTIENQPWFAAI
YRRHRGGSVTYVCGGSLISPCWVISAFTTHCFIDYPKKEDYIVYLGRSRLNSN
TQGEMKFEVENLILHKDYSADTLAHHNDIALLKIFTRSKEGRCAQPSRTIQTIC
LPSMYNDPQFGTSCEITGFGKENSTDYLYPEQLKMTVVKLIFTSHRECQQPHY YGSEVTTKMLCAADPQWKTD-
SCQGDSGGPLVCSLQGRMTLTGIVSWGRG FT CALKDKPGVYTRVSHFLPWIRSHTKEENGLAL
Function: plasminogen activation.

### Table 2, leader peptides

**Table 2**

| | | | |
|---|---|---|---|
| Epicidin-280 | | MENKKDLFDLEIKKDNMENNNELEAQ | |
| Pep-5 | | MKNNKNLFDLEIKKETSQNTDELEPQ | |
| Epilancin-K7 | | MNNSLFDLNLNKGVETQKSDLSPQ | |
| Nisin-A/Z | | | MSTKDFNLDLVSVSKKDSGASPR |
| Subtilin | | | MSKFDDFDLDVVKVSKQDSKITPQ |
| Epidermin | | | |
| | MEAVKEKNDLFNLDVKVNAKESNDSGAEPR | | |
| Gallidermin | | | |
| | MEAVKEKNELFDLDVKVNAKESNDSGAEPR | | |
| Mutacin-1140/III | | | |
| | | | MSNTQLLEVLGTETFDVQEDLFAFD |
| | | TTDTTIVASNDDPD TR | |
| Lacticin-481 | | MKEQNSFNLLQEVTESELDLILGA | |
| Variacin | | | MTNAFQALDEVTDAELDAILGG |
| Mutacin-II | | MNKLNSNAVVSLNEVSDSELDTILGG | |
| Streptococcin-A-FF22 | | | MEKNNEVINSIQEVSLEELDQIIGA |
| Salivaricin-A | | MNAMKNSKDILNNAIEEVSEKELMEVAGG | |
| Sublancin | | MEKLFKEVKLEELENQKGS | |
| Lactocin-S | | | |
| | MKTEKKVLDELSLHASAKMGARDVESSMNAD | | |
| Ruminococcin A | | | MRNDVLTLTNPMEEKELEQILGG |
| Butyrivibriocin OR79A | | | MNKELNALTNPIDEKELEQILGG |
| Streptococcin A-M49 | | MTKEHEIINSIQEVSLEELDQIIGA | |
| Bacteriocin J46 | | | MKEQNSFNLLQEVTESELDLILGA |
| Salivaricin A1 | | | |
| | MKNSKDILTNATEEVSEKELMEVAGG | | |
| Streptin | | | MNNTIKDFDLDLKTNKKTATPY |
| Plantaricin-W alpha | | MKISKIEAQARKDFFKKIDTNSNLLNVNGA | |
| Lacticin-3147A1 | | | |
| | MNKNEIETQPVTWLEEVSDQNFDEDVFGA | | |
| Staphylococcin-C55 alpha | | | |
| | MKSSFLEKDIEEQVTWFEEVSEQEFDDDIFGA | | |
| Plantaricin-W beta | | | |
| | MTKTSRRKNAIANYLEPVDEKSINESFGAGDPEAR | | |
| Lacticin-3147A2 | | | |
| | MKEKNMKKNDTIELQLGKYLEDDMIELAEGDESHGG | | |
| Staphylococcin-C55 beta | | | |

## Claims

1. A method for producing a thioether bridge containing polypeptide, whose origin is not from a Gram positive bacterium, in a host cell, said method comprising:
a) selecting a host cell having a nucleic acid molecule comprising a first and a second nucleic acid fragment which are within the same open reading frame, wherein
- the first nucleic acid fragment encodes a lantibiotic leader peptide that is functionally equivalent to an N-terminal leader peptide found within a prepeptide of a lantibiotic, and wherein this leader peptide acts as a translocation signal sequence and a recognition signal, such that thioether bridge formation may occur;
- the second nucleic acid fragment encodes a desired polypeptide comprising a sequence to be modified into a thioether bridge, the sequence being selected from the group of sequences consisting of Ser-Xaa*ₙ*-Cys and Thr-Xaa*ₙ*-Cys, wherein Xaa is any amino acid and wherein n is 1-13, and wherein the polypeptide is not of Gram positive bacterium origin;
b) selecting the host cell for the presence of the transporter protein LanT;
c) translating the nucleic acid molecule, thus producing a fusion peptide of the lantibiotic leader peptide and the polypeptide comprising the sequence selected from the group of sequences consisting of Ser-Xaaₙ-Cys and Thr-Xaa ₙ-Cys, wherein Xaa is any amino acid and wherein n is 1-13; and
d) harvesting the polypeptide containing the thioether bridge from a medium of the host cell.

2. A method according to claim 1, further comprising harvesting said desired polypeptide after detecting the presence of said leader peptide in the culture medium of said cell.

3. A method according to claim 1 or 2 wherein said desired polypeptide is of essentially eukaryotic or viral descent.

4. A method according to claim 1 or 2 wherein said polypeptide is selected from the group consisting of polypeptides with SEQ ID NO: 1 to 249 as listed in table 1A.

5. A method according to anyone of claims 1 to 4 wherein said leader peptide is selected from the group consisting of leader peptides with SEQ ID NO: 250 to 280 as listed in table 1B.

6. A method according to anyone of claims 1 to 5 wherein said host cell is a Gram-negative prokaryote or an eukaryote.

7. A method according to anyone of claims 1 to 6 wherein said (poly)peptide has not undergone intra-cellular post-translational modification comprising dehydration of a serine or a threonine and/or thioether bridge formation.

8. A method allowing for modification of a desired polypeptide produced by a recombinant host cell, said method comprising steps a, b, c and d of claim 1 and further comprising selecting said host cell for the presence of an enzyme capable of providing post-translational modification.

9. A method according to claim 8 allowing for extra-cellular modification of said desired (poly)peptide said method further comprising selecting said host cell for the presence of an essentially extra-cellular enzyme capable of providing post-translational modification.

10. A method according to claim 8 or 9 wherein said enzyme is capable of dehydrating a serine or a threonine.

11. A method according to claim 8 or 9 wherein said enzyme is capable of providing for thioether bridge formation.

12. A method according to anyone of claims 8 to 11 wherein said desired (poly)peptide is of essentially eukaryotic or viral descent.

13. A method according to anyone of claims 8 to 11 wherein said polypeptide is selected from the group consisting of polypeptides with SEQ ID NO: 1 to 249.

14. A method according to anyone of claims 8 to 13 wherein said leader peptide is selected from the group consisting of leader peptides with SEQ ID NO: 250 to 280.

15. A method according to anyone of claims 8 to 14 wherein said modification comprises dehydration of a serine or a threonine and/or thioether bridge formation.

16. A method according to anyone of claims 8 to 15 wherein said host cell is a Gram-negative prokaryote or an eukaryote.

17. A method according to anyone of claims 8 to 16 wherein said polypeptide has not undergone intra-cellular post-translational modification comprising dehydration of a serine or a threonine and/or thioether bridge formation.

18. A method for the production of a polypeptide of non-lantibiotic descent comprising dehydro alanines and/or dehydro butyric acid residues comprising:
a) selecting a host cell having a nucleic acid molecule comprising a first and a second nucleic acid fragment which are within the same open reading frame, wherein
- the first nucleic acid fragment encodes a lantibiotic leader peptide that is functionally equivalent to an N-terminal leader peptide found within a prepeptide of a lantibiotic, and wherein this leader peptide acts as a translocation signal sequence and a recognition signal, such that thioether bridge formation may occur;
- the second nucleic acid fragment encodes a desired polypeptide comprising a sequence to be modified into a thioether bridge, the sequence being selected from the group of sequences consisting of Ser-Xaa ₙ -Cys and Thr-Xaa ₙ-Cys, wherein Xaa is any amino acid and wherein n is 1-13, and wherein the polypeptide is of essentially eukaryotic or viral descent;
- and a nucleic acid molecule coding for LanB or the N-terminal part of LanM and a nucleic acid molecule coding for LanT;
b) allowing for the translation of said nucleic acid molecules; and
c) harvesting said desired polypeptide from a medium of the host cell.

19. A host cell comprising a recombinant nucleic acid molecule comprising a first and a second nucleic acid fragment which are within the same open reading frame, wherein
- the first nucleic acid fragment encodes a lantibiotic leader peptide that is functionally equivalent to an N-terminal leader peptide found within a prepeptide of a lantibiotic, and wherein this leader peptide acts as a translocation signal sequence and a *recognition* signal, such that thioether bridge formation may occur;
- the second nucleic acid fragment encodes a desired polypeptide comprising a sequence to be modified into a thioether bridge, the sequence being selected from the group of sequences consisting of Ser-Xaa ₙ -Cys and Thr-Xaa ₙ-Cys, wherein Xaa is any amino acid and wherein n is 1-13, wherein the polypeptide is of essentially eukaryotic or viral descent, and wherein the host cell was selected for the presence of the transporter protein LanT.

20. A host cell according to claim 19 wherein said (poly)peptide is selected from the group consisting of polypeptides with SEQ ID NO: 1 to 249.

21. A host cell according to anyone of claims 19 to 20 wherein said leader peptide is selected from the group consisting of leader peptides with SEQ ID NO: 250 to 280.

22. A host cell according to anyone of claims 19 to 21 said host cell being a Gram-negative prokaryote or an eukaryote.

23. A host cell according to anyone of claims 19 to 22 provided with a LanT protein and not provided with a LanB protein.

24. A host cell according to anyone of claims 19 to 23 provided with a LanT protein and not provided with a LanC protein.

25. A host cell according to claim 24 provided with a LanB.

26. A host cell according to claim 22 provided with a LanT, LanB, LanC, and/or LanM protein.

27. A recombinant nucleic acid molecule comprising a first and a second nucleic acid fragment which are within the same open reading frame, wherein
- the first nucleic acid fragment encodes a lantibiotic leader peptide that is functionally equivalent to an N-terminal leader peptide found within a prepeptide of a lantibiotic, and wherein this leader peptide acts as a translocation signal sequence and a recognition signal, such that thioether bridge formation may occur;
- the second nucleic acid fragment encodes a desired polypeptide comprising a sequence to be modified into a thioether bridge, the sequence being selected from the group of sequences consisting of Ser-Xaa ₙ -Cys and Thr-Xaa ₙ-Cys, wherein Xaa is any amino acid and wherein n is 1-13; and
- wherein said polypeptide is of eukaryotic or viral descent.

28. A nucleic acid according to claim 28 wherein said (poly)peptide is selected from the group consisting of polypeptides with SEQ ID NO: 1 to 249.

29. A nucleic acid according to claim 27 or 28, wherein said leader peptide is selected from the group consisting of leader peptides with SEQ ID NO: 250 to 280.

## Patentansprüche

1. Verfahren zur Herstellung einer Thioetherbrücke enthaltend Polypeptid, dessen Ursprung nicht von einem grampositiven Bakterium stammt, in einer Wirtszelle, wobei das Verfahren Folgendes umfasst:
a) Auswählen einer Wirtszelle mit einem Nukleinsäuremolekül, umfassend ein erstes und ein zweites Nukleinsäurefragment, die innerhalb desselben offenen Leserahmens sind, wobei
- das erste Nukleinsäurefragment ein lantibiotisches Leader-Peptid kodiert, das funktionell gleichwertig ist mit einem N-terminalen Leader-Peptid, gefunden in einem Präpeptid eines Lantibioticums und wobei dieses Leader-Peptid als eine Translokationssignalsequenz und ein Erkennungssignal dient, sodass eine Thioether-Brückenbildung stattfinden kann;
- das zweite Nukleinsäurefragment ein erwünschtes Polypeptid kodiert, umfassend eine Sequenz, zu modifizieren in eine Thioetherbrücke, wobei die Sequenz ausgewählt ist aus der Gruppe von Sequenzen, bestehend aus Ser-Xaaₙ-Cys und Thr-Xaaₙ-Cys, wobei Xaa eine Aminosäure ist und wobei n 1-13 ist und wobei das Polypeptid nicht von einem grampositiven Bakterium stammt;
b) Auswählen der Wirtszelle nach der Anwesenheit des Transporterproteins LanT;
c) Translatieren des Nukleinsäuremoleküls, um so ein Fusionspeptid des lantibiotischen Leader-Peptids und des Polypeptids, umfassend die Sequenz, ausgewählt aus der Gruppe von Sequenzen bestehend aus Ser-Xaaₙ-Cys und Thr-Xaaₙ-Cys, zu produzieren, wobei Xaa eine Aminosäure ist und wobei n 1-13 ist; und
d) Ernten des Polypeptids, enthaltend die Thioetherbrücke von einem Medium der Wirtszelle.

2. Verfahren nach Anspruch 1, das ferner das Ernten des erwünschten Polypeptids nach dem Detektieren der Anwesenheit des Leader-Peptids in dem Kulturmedium der Zelle umfasst.

3. Verfahren nach Anspruch 1 oder 2, wobei das erwünschte Polypeptid von im Wesentlichen eukaryotischer oder viraler Abstammung ist.

4. Verfahren nach Anspruch 1 oder 2, wobei das Polypeptid ausgewählt ist aus der Gruppe bestehend aus Polypeptiden mit SEQ ID NO: 1 bis 249, wie in Tabelle 1A aufgeführt.

5. Verfahren nach einem der Ansprüche 1 bis 4, wobei das Leader-Peptid ausgewählt ist aus der Gruppe bestehend aus Leader-Peptiden mit SEQ ID NO: 250 bis 280, wie in Tabelle 1B aufgeführt.

6. Verfahren nach einem der Ansprüche 1 bis 5, wobei die Wirtszelle ein gramnegativer Prokaryot oder ein Eukaryot ist.

7. Verfahren nach einem der Ansprüche 1 bis 6, wobei das (Poly)peptid keiner intrazellulären posttranslationalen Modifizierung, umfassend die Dehydratation eines Serins oder eines Threonins und/oder Thioether-Brückenbildung, unterzogen wurde.

8. Verfahren, das die Modifizierung eines erwünschten Polypeptids, hergestellt durch eine rekombinante Wirtszelle, erlaubt, wobei das Verfahren die Schritte a, b, c und d von Anspruch 1 umfasst und ferner das Auswählen der Wirtszelle nach der Anwesenheit eines Enzyms, fähig zur Bereitstellung posttranslationaler Modifizierung, umfasst.

9. Verfahren nach Anspruch 8, das extrazelluläre Modifizierung des erwünschten (Poly)peptids erlaubt, wobei das Verfahren ferner das Auswählen der Wirtszelle nach der Anwesenheit eines im Wesentlichen extrazellulären Enzyms, fähig zur Bereitstellung posttranslationaler Modifizierung, umfasst.

10. Verfahren nach Anspruch 8 oder 9, wobei das Enzym zur Dehydratation eines Serins oder eines Threonins fähig ist.

11. Verfahren nach Anspruch 8 oder 9, wobei das Enzym fähig ist, Thioether-Brückenbildung zu bewerkstelligen.

12. Verfahren nach einem der Ansprüche 8 bis 11, wobei das erwünschte (Poly)peptid von im Wesentlichen eukaryotischer oder viraler Abstammung ist.

13. Verfahren nach einem der Ansprüche 8 bis 11, wobei das Polypeptid ausgewählt ist aus der Gruppe bestehend aus Polypeptiden mit SEQ ID NO: 1 bis 249.

14. Verfahren nach einem der Ansprüche 8 bis 13, wobei das Leader-Peptid ausgewählt ist aus der Gruppe bestehend aus Leader-Peptiden mit SEQ ID NO: 250 bis 280.

15. Verfahren nach einem der Ansprüche 8 bis 14, wobei die Modifizierung die Dehydratation eines Serins oder eines Threonins und/oder eine Thioether-Brückenbildung umfasst.

16. Verfahren nach einem der Ansprüche 8 bis 15, wobei die Wirtszelle ein gramnegativer Prokaryot oder ein Eukaryot ist.

17. Verfahren nach einem der Ansprüche 8 bis 16, wobei das Polypeptid keiner intrazellulären posttranslationalen Modifizierung, umfassend Dehydratation eines Serins oder eines Threonins und/oder Thioether-Brückenbildung, unterzogen wurde.

18. Verfahren zur Herstellung eines Polypeptids von nicht lantibiotischer Abstammung, umfassend Dehydro-Alanine und/oder Dehydro-Buttersäurereste, das Folgendes umfasst:
a) Auswählen einer Wirtszelle mit einem Nukleinsäuremolekül, umfassend ein erstes und ein zweites Nukleinsäurefragment, die innerhalb desselben offenen Leserahmens sind, wobei
- das erste Nukleinsäurefragment ein lantibiotisches Leader-Peptid kodiert, das funktionell gleichwertig ist mit einem N-terminalen Leader-Peptid, gefunden in einem Präpeptid eines Lantibioticums und wobei dieses Leader-Peptid als eine Translokationssignalsequenz und ein Erkennungssignal dient, sodass eine Thioether-Brückenbildung stattfinden kann;
- das zweite Nukleinsäurefragment ein erwünschtes Polypeptid kodiert, umfassend eine Sequenz, zu modifizieren in eine Thioetherbrücke, wobei die Sequenz ausgewählt ist aus der Gruppe von Sequenzen bestehend aus Ser-Xaaₙ-Cys und Thr-Xaaₙ-Cys, wobei Xaa eine Aminosäure ist und wobei n 1-13 ist und wobei das Polypeptid von im Wesentlichen eukaryotischer oder viraler Abstammung ist;
- und ein Nukleinsäuremolekül LanB oder den N-terminalen Teil von LanM codiert und ein Nukleinsäuremolekül LanT kodiert;
b) Erlauben der Translation der Nukleinsäuremoleküle; und
c) Ernten des erwünschten Polypeptids aus einem Medium der Wirtszelle.

19. Wirtszelle, umfassend ein rekombinantes Nukleinsäuremolekül, umfassend ein erstes und ein zweites Nukleinsäurefragment, die innerhalb desselben offenen Leserahmens sind, wobei
- das erste Nukleinsäurefragment ein lantibiotisches Leader-Peptid kodiert, das funktionell gleichwertig ist mit einem N-terminalen Leader-Peptid, gefunden in einem Präpeptid eines Lantibioticums, und wobei dieses Leader-Peptid als eine Translokationssignalsequenz und ein Erkennungssignal dient, sodass eine Thioether-Brückenbildung stattfinden kann;
- das zweite Nukleinsäurefragment ein erwünschtes Polypeptid kodiert, umfassend eine Sequenz, zu modifizieren in eine Thioetherbrücke, wobei die Sequenz ausgewählt ist aus der Gruppe von Sequenzen bestehend aus Ser-Xaaₙ-Cys und Thr-Xaaₙ-Cys, wobei Xaa eine Aminosäure ist und wobei n 1-13 ist und wobei das Polypeptid von im Wesentlichen eukaryotischer oder viraler Abstammung ist und wobei die Wirtszelle auf die Anwesenheit des Transporterproteins LanT selektiert wurde.

20. Wirtszelle nach Anspruch 19, wobei das (Poly)peptid ausgewählt ist aus der Gruppe, bestehend aus Polypeptiden mit SEQ ID NO: 1 bis 249.

21. Wirtszelle nach einem der Ansprüche 19 bis 20, wobei das Leader-Peptid ausgewählt ist aus der Gruppe, bestehend aus Leader-Peptiden mit SEQ ID NO: 250 bis 280.

22. Wirtszelle nach einem der Ansprüche 19 bis 21, wobei die Wirtszelle ein gramnegativer Prokaryot oder ein Eukaryot ist.

23. Wirtszelle nach einem der Ansprüche 19 bis 22, versehen mit einem LanT-Protein und nicht versehen mit einem LanB-Protein.

24. Wirtszelle nach einem der Ansprüche 19 bis 23, versehen mit einem LanT-Protein und nicht versehen mit einem LanC-Protein.

25. Wirtszelle nach Anspruch 24, versehen mit einem LanB-Protein.

26. Wirtszelle nach Anspruch 22, versehen mit einem LanT-, LanB-, LanC- und/oder LanM-Protein.

27. Rekombinantes Nukleinsäuremolekül, umfassend ein erstes und ein zweites Nukleinsäurefragment, die innerhalb desselben offenen Leserahmens sind, wobei
- das erste Nukleinsäurefragment ein lantibiotisches Leader-Peptid kodiert, das funktionell gleichwertig ist mit einem N-terminalen Leader-Peptid, gefunden in einem Präpeptid eines Lantibioticums und wobei dieses Leader-Peptid als eine Translokationssignalsequenz und ein Erkennungssignal dient, sodass eine Thioether-Brückenbildung stattfinden kann;
- das zweite Nukleinsäurefragment ein erwünschtes Polypeptid kodiert, umfassend eine Sequenz, zu modifizieren in eine Thioetherbrücke, wobei die Sequenz ausgewählt ist aus der Gruppe von Sequenzen bestehend aus Ser-Xaaₙ-Cys und Thr-Xaaₙ-Cys, wobei Xaa eine Aminosäure ist und wobei n 1-13 ist; und
- wobei das Polypeptid von eukaryotischer oder viraler Abstammung ist.

28. Nukleinsäure nach Anspruch 28, wobei das (Poly)peptid ausgewählt ist aus der Gruppe bestehend aus Polypeptiden mit SEQ ID NO: 1 bis 249.

29. Nukleinsäure nach Anspruch 27 oder 28, wobei das Leader-Peptid ausgewählt ist aus der Gruppe bestehend aus Leader-Peptiden mit SEQ ID NO: 250 bis 280.

## Revendications

1. Procédé de production d'un polypeptide contenant un pont thioéther, dont l'origine n'est pas d'une bactérie Gram positif, dans une cellule hôte, le procédé comprenant ;
a) la sélection d'une cellule hôte comportant une molécule d'acide nucléique comprenant un premier et un second fragment d'acide nucléique qui sont dans le même cadre de lecture ouvert,
- le premier fragment d'acide nucléique codant pour un peptide leader de lantibiotique qui est fonctionnellement équivalent à un peptide leader N-terminal trouvé dans un pré-peptide d'un lantibiotique, et ce peptide leader agissant comme une séquence signal de translocation et un signal de reconnaissance, de telle sorte que la formation d'un pont thioéther peut avoir lieu ;
- le second fragment d'acide nucléique codant pour un polypeptide voulu comprenant une séquence à modifier en un pont thioéther, la séquence étant choisie parmi le groupe de séquences constitué de Ser -Xaaₙ-Cys et Thr-Xaa ₙ-Cys , où Xaa est un acide aminé quelconque et où n est de 1 à 13 , et dans lequel le polypeptide n'a pas pour origine une bactérie Gram positif ;
b) la sélection de la cellule hôte pour la présence de la protéine de transport LanT ;
c) la traduction de la molécule d'acide nucléique, produisant ainsi un peptide de fusion du peptide leader de lantibiotique et du polypeptide comprenant la séquence sélectionnée parmi le groupe de séquences constitué de Ser - Xaaₙ-Cys et Thr -Xaaₙ-Cys , où Xaa est un acide aminé quelconque et dans lequel n est de 1 à 13, et
d) la récolte du polypeptide contenant le pont thioéther à partir d'un milieu de la cellule hôte.

2. Procédé selon la revendication 1, comprenant en outre la récolte du polypeptide voulu après détection de la présence du peptide leader dans le milieu de culture de la cellule.

3. Procédé selon la revendication 1 ou 2, dans lequel le polypeptide voulu est de souche essentiellement eucaryote ou virale.

4. Procédé selon la revendication 1 ou 2, dans lequel le polypeptide est choisi dans le groupe constitué de polypeptides ayant SEQ ID NO : 1 à 249, comme indiqué dans le tableau 1A.

5. Procédé selon l'une quelconque des revendications 1 à 4, dans lequel le peptide leader est choisi dans le groupe constitué de peptides leader ayant SEQ ID NO : 250 à 280, comme indiqué dans le tableau 1B.

6. Procédé selon l'une quelconque des revendications 1 à 5, dans lequel la cellule hôte est une cellule procaryote ou eucaryote Gram négatif.

7. Procédé selon l'une quelconque des revendications 1 à 6, dans lequel le (poly)peptide n'a pas subi de modification intracellulaire post-traductionnelle comprenant la déshydratation d'une sérine ou d'une thréonine et/ou une formation de pont thioéther.

8. Procédé permettant la modification d'un polypeptide voulu produit par une cellule hôte recombinante, le procédé comprenant les étapes a , b , c et d de la revendication 1 et comprenant en outre la sélection de la cellule hôte pour la présence d'une enzyme capable de fournir une modification post-traductionnelle.

9. Procédé selon la revendication 8 permettant une modification extracellulaire du (poly)peptide voulu, le procédé comprenant en outre la sélection de la cellule hôte pour la présence d'une enzyme essentiellement extracellulaire capable de fournir une modification post-traductionnelle.

10. Procédé selon la revendication 8 ou 9, dans lequel l'enzyme est capable de déshydrater une sérine ou une thréonine.

11. Procédé selon la revendication 8 ou 9, dans lequel l'enzyme est capable de fournir une formation de pont thioéther.

12. Procédé selon l'une quelconque des revendications 8 à 11, dans lequel le (poly)peptide voulu est de souche essentiellement eucaryote ou virale.

13. Procédé selon l'une quelconque des revendications 8 à 11, dans lequel le polypeptide est choisi dans le groupe constitué de polypeptides ayant SEQ ID NO : 1 à 249.

14. Procédé selon l'une quelconque des revendications 8 à 13, dans lequel le peptide leader est choisi dans le groupe constitué de peptides leader ayant SEQ ID NO : 250 à 280.

15. Procédé selon l'une quelconque des revendications 8 à 14, dans lequel la modification comprend la déshydratation d'une sérine ou d'une thréonine et/ou la formation d'un pont thioéther t.

16. Procédé selon l'une quelconque des revendications 8 à 15, dans lequel la cellule hôte est une cellule procaryote ou eucaryote Gram négatif.

17. Procédé selon l'une quelconque des revendications 8 à 16, dans lequel le polypeptide n'a pas subi de modification intra-cellulaire post-traductionnelle comprenant la déshydratation d'une sérine ou d'une thréonine et/ou une formation de pont thioéther.

18. Procédé pour la production d'un polypeptide de souche non lantibiotique comprenant des déhydro alanines et/ou des résidus d'acide déhydro butyrique consistant à :
a) sélectionner une cellule hôte ayant une molécule d'acide nucléique comprenant un premier et un second fragment d'acide nucléique qui sont dans le même cadre de lecture ouvert, dans lequel
- le premier fragment d'acide nucléique code pour un peptide leader de lantibiotique qui est fonctionnellement équivalent à un peptide leader N-terminal trouvé dans un pré-peptide d'un lantibiotique , et dans lequel ce peptide leader agit comme une séquence signal de translocation et un signal de reconnaissance, de telle sorte que la formation d'un pont thioéther peut avoir lieu ;
- le second fragment d'acide nucléique code pour un polypeptide voulu comprenant une séquence à modifier en un pont thioéther, la séquence étant choisie parmi le groupe de séquences constitué de Ser -Xaan-Cys et Thr -Xaa ₙ-Cys , où Xaa est un acide aminé quelconque et dans lequel n est de 1 à 13 , et dans lequel le polypeptide n'a pas pour origine une bactérie Gram positif ;
- et une molécule d'acide nucléique codant pour LanB ou la partie N-terminale de LanM et facultativement une molécule d'acide nucléique codant pour LanT ;
b) permettre la traduction des molécules d'acide nucléique et
d) recueillir le polypeptide voulu à partir d'un milieu de la cellule hôte.

19. Cellule hôte comprenant une molécule d'acide nucléique recombinante comprenant un premier et un second fragment d'acide nucléique qui sont à l'intérieur du même cadre de lecture ouvert, dans laquelle
- le premier fragment d'acide nucléique code pour un peptide leader de lantibiotique qui est fonctionnellement équivalent à un peptide leader N-terminal trouvé dans un pré-peptide d'un lantibiotique , et dans laquelle ce peptide leader agit comme une séquence signal de translocation et un signal de reconnaissance, de telle sorte que la formation d'un pont thioéther peut avoir lieu ;
- le second fragment d'acide nucléique code pour un polypeptide voulu comprenant une séquence à modifier en un pont thioéther, la séquence étant choisie parmi le groupe de séquences constitué de Ser -Xaaₙ-Cys et Thr -Xaa ₙ-Cys , où Xaa est un acide aminé quelconque et dans lequel n est de 1 à 13 , et dans laquelle le polypeptide est de souche essentiellement eucaryote ou virale et dans laquelle la cellule hôte était sélectionnée pour la présence de la protéine de transport LanT.

20. Cellule hôte selon la revendication 19, dans laquelle le (poly) peptide est choisi dans le groupe constitué de polypeptides ayant SEQ ID NO : 1 à 249.

21. Cellule hôte selon l'une quelconques des revendications 19 à 20, dans laquelle le peptide leader est choisi dans le groupe constitué de peptides leader ayant la SEQ ID NO : 250 à 280.

22. Cellule hôte selon l'une quelconque des revendications 19 à 21, la cellule hôte étant une cellule procaryote ou eucaryote Gram négatif.

23. Cellule hôte selon l'une quelconque des revendications 19 à 22 munie d'une protéine LanT et non munie d'une protéine LanB.

24. Cellule hôte selon l'une quelconque des revendications 19 à 23 munie d'une protéine LanT et non munie d' une protéine LanC.

25. Cellule hôte selon la revendication 24 munie d'une protéine LanB.

26. Cellule hôte selon la revendication 22 munie d'une protéine LanT, LanB, LanC, et/ou LanM.

27. Molécule d'acide nucléique recombinante comprenant un premier et un second fragment d'acide nucléique qui sont dans le même cadre de lecture ouvert, dans laquelle
- le premier fragment d'acide nucléique code pour un peptide leader de lantibiotique qui est fonctionnellement équivalent à un peptide leader N-terminal trouvé dans un pré-peptide d'un lantibiotique , et dans laquelle ce peptide leader agit comme une séquence signal de translocation et un signal de reconnaissance, de telle sorte que la formation d'un pont thioéther peut avoir lieu ;
- le second fragment d'acide nucléique code pour un polypeptide voulu comprenant une séquence à modifier en un pont thioéther, la séquence étant choisie parmi le groupe de séquences constitué de Ser -Xaa ₙ- Cys et Thr -Xaaₙ-Cys , où Xaa est un acide aminé quelconque et dans lequel n est de 1 à 13 , et
- dans laquelle le polypeptide est de souche eucaryote ou virale.

28. Acide nucléique selon la revendication 28, dans lequel le (poly)peptide est sélectionné dans le groupe constitué de polypeptides ayant SEQ ID NO : 1 à 249.

29. Acide nucléique selon la revendication 27 ou 28, dans lequel le peptide leader est choisi dans le groupe constitué de peptides leader ayant la SEQ ID NO : 250 à 280.
